# EUROPÄISCHE PATENTSCHRIFT

(11) **EP 3 352 896 B1**
(45) Veröffentlichungstag und Bekanntmachung des Hinweises auf die Patenterteilung: **23.10.2019**
(21) Anmeldenummer: 16770915.3
(22) Anmeldetag: 20.09.2016
(51) Int. Cl.: C07C 263/10, C07C 265/14, B01J 19/00

(54) **VERFAHREN ZUR HERSTELLUNG VON ISOCYANATEN**
METHOD FOR MAKING ISOCYANATES
PROCÉDÉ DE FABRICATION D'ISOCYANATES

(30) Priorität: 24.09.2015 EP 15186734
(43) Veröffentlichungstag der Anmeldung: 01.08.2018
(73) Patentinhaber: Covestro Deutschland AG, 51373 Leverkusen (DE)
(72) Erfinder: KNAUF, Thomas, 41542 Dormagen (DE); MANZEL, Dirk, 47447 Moers (DE); PLATHEN, Peter, 47829 Krefeld (DE); SPRIEWALD, Jürgen, 25337 Kölln-Reisiek (DE)
(74) Vertreter: Levpat
(86) Internationale Anmeldenummer: PCT/EP2016/072327
(87) Internationale Veröffentlichungsnummer: WO 2017/050776

(56) Entgegenhaltungen:
- WO-A1-2009/037179
- WO-A1-2013/029918
- DE-A1- 19 521 800

## Beschreibung

Die Erfindung betrifft ein Verfahren zur in wirtschaftlicher und technischer Hinsicht verbesserten Ausgestaltung einer Produktionsunterbrechung in einem Herstellungsverfahren für Isocyanate durch Phosgenierung der korrespondierenden Amine, bei dem während der Außerbetriebnahme eines Anlagenteils oder mehrerer Anlagenteile nicht die ganze Produktionsanlage stillgelegt wird, sondern in der Produktionsanlage vorhandene Einsatzstoffe und/oder Reaktionsprodukte durch mindestens einen Teil der nicht außer Betrieb genommenen Anlagenteile in Kreislauffahrweise geführt werden. Des Weiteren betrifft die vorliegende Erfindung eine Anlage zur Herstellung von Isocyanaten und ein Verfahren zum Betrieb einer Anlage zur Herstellung von Isocyanaten.

Die großtechnische Herstellung von Di- und Polyisocyanaten durch Umsetzung der korrespondierenden Amine mit Phosgen ist seit längerem aus dem Stand der Technik bekannt, wobei die Reaktion in der Gas- oder Flüssigphase sowie diskontinuierlich oder kontinuierlich durchgeführt werden kann (W. Siefken, Liebigs Ann. 562, 75 - 106 (1949)). Verfahren zur Herstellung von organischen Isocyanaten aus primären Aminen und Phosgen sind bereits mehrfach beschrieben worden, siehe beispielsweise Ullmanns Encyklopädie der technischen Chemie, 4. Aufl. (1977), Band 13, S. 351 bis 353 sowie G. Wegener et. al. Applied Catalysis A: General 221 (2001), p. 303 - 335, Elsevier Science B.V. Es kommen dabei sowohl aromatische Isocyanate wie beispielsweise Methylendiphenyldiisocyanat (MMDI - "monomeres MDI"), Polymethylen-Polyphenylen-Polyisocyanate (das sind die höheren Homologen des MMDI, auch PMDI, "polymeres MDI" genannt; diese fallen technisch stets im Gemisch mit MMDI-Anteilen an) oder Toluylendiisocyanat (TDI) als auch aliphatische Isocyanate wie z. B. Hexamethylendiisocyanat (HDI) oder Isophorondiisocyanat (IPDI) weltweit zum Einsatz.

Die großtechnische Herstellung von Phosgen, das bei der Phosgenierung der entsprechenden Amine zum Einsatz kommt, aus CO und Chlor an Aktivkohle-Katalysatoren in einem Rohrbündelreaktor ist ebenfalls aus dem Stand der Technik bekannt (z.B. Ullmann's Encyclopedia of Industrial Chemistry, 5th ed. Vol. A 19p 413f., VCH Verlagsgesellschaft mbH, Weinheim, 1991). Dabei wird Kohlenmonoxid im stöchiometrischen Überschuss mit Chlor vereinigt und über einen Festbettkatalysator geleitet. Als Katalysator wird für industrielle Zwecke Aktivkohle eingesetzt, wobei die Auswahl einer geeigneten Aktivkohle bis heute empirisch erfolgt (Mitchell et al.: Selection of carbon catalysts for the industrial manufacture of phosgene; Catal. Sei. Technol., 2012, 2, 2109-2115).

WO 2009/037179 A1 befasst sich mit einem Verfahren zur Herstellung von Isocyanaten mit möglichst geringer Bevorratung von Phosgen und möglichst geringer Menge an Phosgen, die sich jeweils in den Verfahrensstufen befinden. Die Anmeldung schlägt hierzu ein Verfahren vor, in dem das in den Verfahrensstufen befindliche Phosgen im Wesentlichen gasförmig vorliegt.

WO 2013/029918 A1 beschreibt ein Verfahren zur Herstellung von Isocyanaten durch Umsetzung der korrespondierenden Amine mit Phosgen, das auch bei unterschiedlichen Auslastungen der Anlage ohne Probleme durchgeführt werden kann, insbesondere soll auch bei Betreiben der Anlage im Teillastbereich das Mischen und/oder die Reaktion in dem jeweils optimierten Verweilzeitfenster erfolgen, indem das Verhältnis von Phosgen zu Amin erhöht wird oder ein oder mehrere inerte Stoffe dem Phosgen- und/oder Aminstrom zugesetzt werden. Das erfindungsgemäße Verfahren soll es ermöglichen, eine bestehende Anlage bei unterschiedlichen Auslastungen mit gleichbleibender Produkt- und Verfahrensqualität zu betreiben. Dies soll die Anschaffung mehrerer Anlagen mit unterschiedlichen Nennkapazitäten einsparen.

Die Anmeldung lehrt, dass wesentliche Parameter einer Phosgenierung, wie insbesondere die Verweilzeiten der Reaktionspartner in den einzelnen Apparaten, für den Betrieb der Produktionsanlage bei Nennkapazität optimiert sind, was zu Problemen hinsichtlich Ausbeute und Produktreinheit führen kann, wenn die Anlage bei geringerer als Nennkapazität betrieben wird (vgl. S. 2, Z. 20 bis 36). Um die optimierten - engen - Verweilzeitfenster auch bei Teilauslastung (d. h. gegenüber Betrieb bei Nennkapazität mit reduziertem Aminstrom) erreichen zu können, wird vorgeschlagen, entweder den Phosgenstrom und/oder den Inertenanteil zu erhöhen (vgl. S. 3, Z. 5 bis 19), und zwar bevorzugt so, dass der Gesamtmengenstrom aller Komponenten im Wesentlichen dem bei Nennkapazität entspricht (vgl. S. 6, Z. 4 bis 8). Die Anmeldung erwähnt zwar An- und Abfahrprozesse in der Beschreibung des Hintergrunds der beanspruchten Erfindung auf S. 2, offenbart jedoch keinerlei technische Lehre zum konkreten Handeln, wie denn eine nicht in Betrieb befindliche Produktionsanlage (d. h. der Aminstrom und damit der Strom an produziertem Isocyanat sind gleich *null*) am vorteilhaftesten auf den angestrebten Betriebszustand der Nennkapazität gebracht wird, oder wie denn eine in Betrieb befindliche Produktionsanlage am vorteilhaftesten außer Betrieb genommen wird (d. h. der Aminstrom wird auf null reduziert, sodass kein weiteres Isocyanat mehr produziert werden kann). Die in der Anmeldung offenbarten technischen Maßnahmen (d. h. die Erhöhung des Phosgenstroms und/oder des Inertenanteils) sind ausschließlich im Zusammenhang mit der Problematik des *Betriebs* (d. h. der Aminstrom ist signifikant *größer* als null) einer Produktionsanlage bei geringerer als Nennkapazität bzw. mit der Problematik, wie eine bei Nennkapazität *betriebene* Anlage vorteilhafterweise auf den Betrieb bei geringerer als Nennkapazität umgestellt werden kann (siehe die Beispiele), zu sehen.

Der Reaktionsaustrag aus der Phosgenierstraße kann, wie in EP 1 546 091 B1 beschrieben, aufgearbeitet werden. Die Aufarbeitung des Reaktionsproduktes erfolgt in einem Schichtverdampfer, bevorzugt einem Fallfilmverdampfer, in dem Phosgen und HCl schonend verdampft werden.

US 5 136 087 (B) beschreibt ebenso die Entfernung von Phosgen aus der Reaktionsmischung der Phosgenierung mittels eines inerten Lösungsmitteldampfes, der aus der Lösungsmittelrückgewinnung der Phosgenieranlage stammen kann.

Eine mögliche Ausführungsform der Lösungsmittelabtrennung und -rückgewinnung ist in EP 1 854 783 B1 beschrieben. Di- und Polyisocyanate der Diphenylmethanreihe (MDI), die durch Umsetzung von in einem Lösungsmittel gelösten entsprechenden Aminen mit Phosgen gewonnen wurden, werden zuerst von Chlorwasserstoff und überschüssigem Phosgen befreit, und anschließend wird eine destillative Auftrennung dieser Rohlösung in Isocyanate und Lösungsmittel durchgeführt. Das Lösungsmittel wird in den Prozess zurückgeführt zur Herstellung von Lösungen der Einsatzstoffe der Polyisocyanatherstellung.

Die Güte eines Verfahrens zur Herstellung von Di- und Polyisocyanaten ist einerseits definiert durch den Gehalt des Roh-Produktes an unerwünschten Nebenkomponenten und Verunreinigungen, die durch unsachgemäße Reaktionsführung entstehen. Andererseits ist die Güte eines Verfahrens dadurch definiert, dass der gesamte Prozess ohne technischen Produktionsausfall oder Problemen, die zu einem Eingriff in den Prozess nötigen, betrieben werden kann und dass Verluste an Einsatzstoffen vermieden oder zumindest minimal gehalten werden.

Solche Probleme können beispielsweise beim "Anfahren" (Inbetriebnehmen der Produktionsanlage) bzw. "Abfahren" (Stilllegung der Produktionsanlage) der Phosgenierung der entsprechenden Amine auftreten. Derartige Probleme können beispielsweise sein, dass es zur Entstehung von Feststoffen kommt, die zu Anbackungen und Verblockungen an der Ausrüstung (Mischer, Düse, Reaktorwände, Leitungen, etc.) führen. Nachteilig ist des Weiteren, dass bei notwendigen Revisions-, Wartungs-, Reparatur- und Reinigungsarbeiten an oder in einem Reaktor oder einem sonstigen Anlagenteil regelmäßig immer alle Anlagenteile abgeschaltet werden müssen, da die Verfahrensschritte aufeinander aufbauen und somit immer sukzessive erfolgen. Dadurch muss die gesamte Anlage geleert werden, was zu einem erheblichen Materialausschuss führt und sehr zeitaufwändig ist. Des Weiteren muss Energie aufgewendet werden, um Reaktoren und Anlagenteile wieder auf die jeweiligen Betriebstemperaturen zu bringen. Solche Produktionsstillstände für Anlagenrevisionen, Reparatur- und Reinigungsmaßnahmen oder auftretende Rohstoff- oder Hilfsstoffmängel, geplant oder ungeplant, sind immer wiederkehrende Anlagenzustände, die einen erheblichen Einfluss auf das wirtschaftliche Betreiben einer kontinuierlich arbeitenden Anlage bzw. eines kontinuierlich arbeitenden Verfahrens haben.

Den beschriebenen Verfahren des Standes der Technik gelingt es zwar, mit hoher Ausbeute Di- und Polyisocyanate herzustellen, ohne dass es bei den Endprodukten zu einer Qualitätseinbuße kommt, jedoch werden nur Verfahren beschrieben, die sich im Normalbetrieb befinden. Produktionsstillstände für Anlagenrevisionen, Reparatur- und Reinigungsmaßnahmen oder z. B. Rohstoff- oder Hilfsstoffmangel werden nicht berücksichtigt. Dabei sind Produktionsstillstände, geplant oder ungeplant, immer wiederkehrende Anlagenzustände, die einen erheblichen Einfluss auf das wirtschaftliche Betreiben einer kontinuierlich arbeitenden Anlage haben.

Bei einem solchen Produktionsstillstand kann es sich um einen Revisionsstillstand halten, der im Voraus geplant wird, wobei zu diesem Zweck die Anlage abgefahren, die Energiezufuhren abgestellt und üblicherweise alle zu revidierenden Anlagenteile geöffnet und zum Zwecke der Prüfung gereinigt werden. Eine solche Revision kann eine oder mehrere Wochen dauern. Nach Beendigung der Revision wird die Produktionsanlage geschlossen, gegebenenfalls inertisiert, mit Hilfsstoffen versehen, und, nachdem die entsprechenden Energien und Rohstoffe zur Verfügung stehen, wieder angefahren. Ein Produktionsstillstand ist allerdings nicht zwangsläufig mit einem Öffnen oder einem anderen mechanischen Eingriff in einen Reaktor oder einen anderen Apparat der Anlage verbunden, sondern kann auch mit dem Abstellen und erneuten Starten der Produktionsanlage aus diversen anderen Gründen in Verbindung stehen, wie z. B. bei einem Ausfall der Rohstoffversorgung. In einem solchen Fall wird die Anlage üblicherweise im Teillastbetrieb gefahren und muss im schlimmsten Fall, wenn die logistische Versorgungskette unterbrochen ist, abgestellt werden. Des Weiteren können Produktionsstillstände durch Wartungs-, Reinigungs- oder Reparaturbedarf an der Produktionsanlage erzwungen sein. Hier spricht man üblicherweise von Kurzstillständen im Herstellungsprozess von Di- und Polyisocyanaten, wenn die Produktion bis zu einem Tag unterbrochen ist. Alle diese Produktionsstillstände zeichnen sich in der Praxis dadurch aus, dass es zu Produktionsverlusten kommt und dass beim Wiederanfahren der Anlage, wenn z. B. inertisiert werden muss, Stickstoff verbraucht wird oder beim Aufheizen der Anlage oder der Einsatzstoffe Energien wie Dampf und Strom benötigt werden.

Dem Fachmann ist bekannt, dass ein semikontinuierlich oder kontinuierlich betriebener industrieller Prozess ausgehend von einer in Betrieb befindlichen Produktionsanlage nicht augenblicklich in einen Produktionsstillstand überführt werden kann, sondern vorher kontrolliert abgefahren werden muss. Dies gilt auch für einen Anlagenausfall im Falle einer Havarie. Um nach dem Produktionsstillstand wieder produzieren zu können, muss die Anlage wieder auf die Prozessparameter vor dem Produktionsstillstand hochgefahren werden. Edukte und Apparaturen müssen aufgeheizt werden, Apparate müssen gegebenenfalls inertisiert werden, die Belastung der Apparate mit den Edukten wird allmählich auf den angestrebten Soll-Wert gesteigert. Während dieser Anfahrphase geht also weiterhin Produktionsmenge verloren, und es muss überproportional viel Energie aufgewendet werden, um die ausgekühlte Anlage zum Anfahren vorzubereiten und sie dann auch unter Beachtung aller betriebsrelevanten Parameter auf den gewünschten Sollwert hochzufahren.

Wünschenswert wäre also ein Verfahren, bei dem es durch einfache Maßnahmen ermöglicht wird, Produktionsstillstände beim Betreiben des Herstellungsprozesses von Di- und Polyisocyanaten hinsichtlich Zeitaufwand, Energieverbrauch, Hilfsstoff- und Rohstoffverbrauch und/oder Verringerung von Abfällen zu optimieren. Dieses würde in nicht unerheblichem Ausmaß zu einer Verbesserung der Produktivität bzw. Wirtschaftlichkeit eines kontinuierlich betriebenen Verfahrens bzw. einer entsprechenden Produktionsanlage führen.

Überraschend wurde gefunden, dass diese Aufgabe für einen Herstellprozess von Di- und Polyisocyanaten gelöst werden kann, wenn (vereinfacht ausgedrückt und ohne hierauf beschränkt zu sein) während eines Kurzstillstandes so viele Anlagenteile wie möglich in "Kreislauffahrweise" gestellt werden, um nach der Maßnahme die Gesamtanlage schnellstmöglich wieder anfahren zu können. Überraschend wurde auch gefunden, dass der Energieverbrauch bei in für 30 Minuten bis zu 1 Tag in Kreislauf gestellter Anlage manchmal geringer ist als im Fall einer Komplettabschaltung der Anlage für einen Tag mit erneutem Wiederanfahren. Durch eine gezielte Kreislauffahrweise der nicht von dem Kurzstillstand betroffenen Anlagenteile werden vielfältige Vorteile realisiert, wie weiter unten noch näher erläutert wird.

### Die vorliegende Erfindung stellt daher Folgendes bereit:

Ein **Verfahren zur Herstellung von Isocyanaten** (1), umfassend die Schritte:
I) Umsetzung eines Amins (2) mit Phosgen (3) in der Flüssigphase in einer **Reaktionsstrecke** (**1000**) umfassend
   I.1) eine Einrichtung (1020) zur Bereitstellung eines Amins (2), bevorzugt in Form einer Aminlösung (20) in einem Lösungsmittel (4),
   I.2) eine Einrichtung (1030) zur Bereitstellung von Phosgen (3), bevorzugt in Form einer Phosgenlösung (30) in einem Lösungsmittel (4),
   I.3) eine Einrichtung (1040) zur Bereitstellung eines Lösungsmittels (4),
   I.4) eine Mischeinrichtung (1100) zur Vermischung von Amin (2) oder Aminlösung (20) mit Phosgen (3) oder Phosgenlösung (30) und optional weiterem Lösungsmittel (4) und
   I.5) einen strömungstechnisch nach der Mischeinrichtung angeordneten Reaktionsraum (1200), dem optional eine Abscheidereinrichtung (1210) nachgeschaltet ist,
   wobei
   das Amin (2), bevorzugt in Form einer Aminlösung (20), mit einem Massenstrom m₂ aus der Einrichtung 1020 und
   das Phosgen (3), bevorzugt in Form einer Phosgenlösung (30), mit einem Massenstrom m₃ aus der Einrichtung 1030 sowie
   optional Lösungsmittel (4) aus der Einrichtung 1040 mit einem Massenstrom m₄
   in die Mischeinrichtung 1100 geführt und dort vermischt werden,
   die erhaltene Mischung im nachgeschalteten Reaktionsraum umgesetzt wird und
   in einen flüssigen, das Roh-Isocyanat und Lösungsmittel (sowie Spuren an Phosgen und Chlorwasserstoff) enthaltenden Strom (60) und einen gasförmigen, Phosgen und Chlorwasserstoff (sowie Spuren an Lösungsmittel) enthaltenden Strom (70) aufgetrennt wird;
II) Trennung des flüssigen Stroms (60) aus Schritt I) in einen flüssigen, Lösungsmittel und Roh-Isocyanat (sowie Spuren an Phosgen) enthaltenden Strom (80) und einen gasförmigen, Phosgen und Chlorwasserstoff (sowie Spuren an Lösungsmittel) enthaltenden Strom (90) in einer **Destillationsvorrichtung (2100** - **"Entphosgenierkolonne")**;
III) Trennung des flüssigen Stroms (80) in einen gasförmigen, Lösungsmittel (sowie Spuren an Phosgen) enthaltenden Strom (110) und eine flüssigen, Roh-Isocyanat (sowie Spuren an Lösungsmittel) enthaltenden Strom (100) in einer **Destillationsvorrichtung (2200** - **"Lösungsmittelkolonne")**;
IV) Trennung des gasförmigen Stroms (110), bevorzugt nach dessen Verflüssigung in einem Kondensator (2310), in einen flüssigen, Lösungsmittel enthaltenden Strom (120) und einen gasförmigen, Phosgen enthaltenden Strom (130) in einer **Destillationsvorrichtung (2300** - **"Lösungsmittelstripper")**;
V) Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, in einer **Destillationsvorrichtung (2400),** optional umfassend die Abtrennung von polymeren Isocyanatfraktionen in einer vorgeschalteten Einrichtung zur **Polymerabtrennung (2410 - "Polymerabtrennung", PMA)** als Strom (141);
VI) Absorption der gasförmigen Ströme (70), (90) und (130) in Lösungsmittel (4) unter Erhalt eines flüssigen, Lösungsmittel und Phosgen enthaltenden Stroms (160) und eines gasförmigen, Chlorwasserstoff enthaltenden Stroms (170) in einer **Absorptionsvorrichtung (2500 - "Phosgenabsorber")**;
VII) optional und bevorzugt, Absorption des gasförmigen Stroms (170) in Wasser oder verdünnter Salzsäure in einer weiteren **Absorptionsvorrichtung (2600 - "HCl-Absorptionskolonne")**;
VIII) optional und bevorzugt, Reinigung von Abgasströmen mindestens aus VII), bevorzugt Reinigung von Abgasströmen aus allen vorhandenen Anlagenteilen, in einer **Vorrichtung zur Abgasreinigung (3000);**
IX) optional und bevorzugt, Herstellung von Phosgen (3) aus Kohlenmonoxid und Chlor in einer **Phosgenerzeugungs-Vorrichtung (4000),** welche mit der Einrichtung (1030) zur Bereitstellung von Phosgen verbunden ist;
wobei
bei einer Außerbetriebnahme eines Anlagenteils oder mehrerer Anlagenteile aus den Schritten (I) bis (IX), insofern diese durchgeführt werden, der Massenstrom m₂ auf null reduziert wird und in mindestens einem der nicht außer Betrieb genommenen Anlagenteile der Ausgangsstrom dieses mindestens einen nicht außer Betrieb genommenen Anlagenteils
(i) in den jeweiligen Anlagenteil oder
(ii) in einen strömungstechnisch davor oder dahinter liegenden Anlagenteil geführt und von dort, optional über weitere nicht außer Betrieb genommene Anlagenteile, in den ausgehenden Anlagenteil
zurückgeführt wird (sog. "Kreislauffahrweise"). Auf diese Weise wird bewirkt, dass durch den mindestens einen nicht außer Betrieb genommenen Anlagenteil kontinuierlich ein Stoffstrom fließt. Bevorzugt wird in allen nicht außer Betrieb genommenen Anlagenteilen so verfahren, d. h. bevorzugt werden alle nicht außer Betrieb genommenen Anlagenteile gemäß (i) oder (ii) in Kreislauffahrweise betrieben bzw. in eine Kreislauffahrweise eingebunden.

Ein weiterer Gegenstand der vorliegenden Erfindung ist eine **Anlage zur Herstellung von Isocyanaten in der Flüssigphase,** wie sie weiter unten noch im Detail beschrieben wird und die sich für die Durchführung des erfindungsgemäßen Verfahrens eignet.

Schließlich ist ein Gegenstand der vorliegenden Erfindung ein **Verfahren zum Betrieb einer Anlage zur Herstellung von Isocyanaten in der Flüssigphase,** was weiter unten noch im Detail beschrieben wird.

Das erfindungsgemäße Verfahren wird im Regelbetrieb kontinuierlich betrieben. Dies bedeutet, dass die Anlagenteile aus den Schritten (I) bis (IX), insofern diese durchgeführt werden, während der Produktion, also während der Massenstrom m₂ von null verschieden ist (m₂ ≠ 0), kontinuierlich mit den entsprechenden Eingangsströmen (z. B. Aminlösung und Phosgenlösung in die Mischeinrichtung 1100) beschickt werden und ihnen kontinuierlich die jeweiligen Produkte (z. B. der flüssige, das Roh-Isocyanat und Lösungsmittel (sowie Spuren an Phosgen und Chlorwasserstoff) enthaltende Strom (60) und der gasförmige, Phosgen und Chlorwasserstoff (sowie Spuren an Lösungsmittel) enthaltende Strom (70)) entnommen werden.

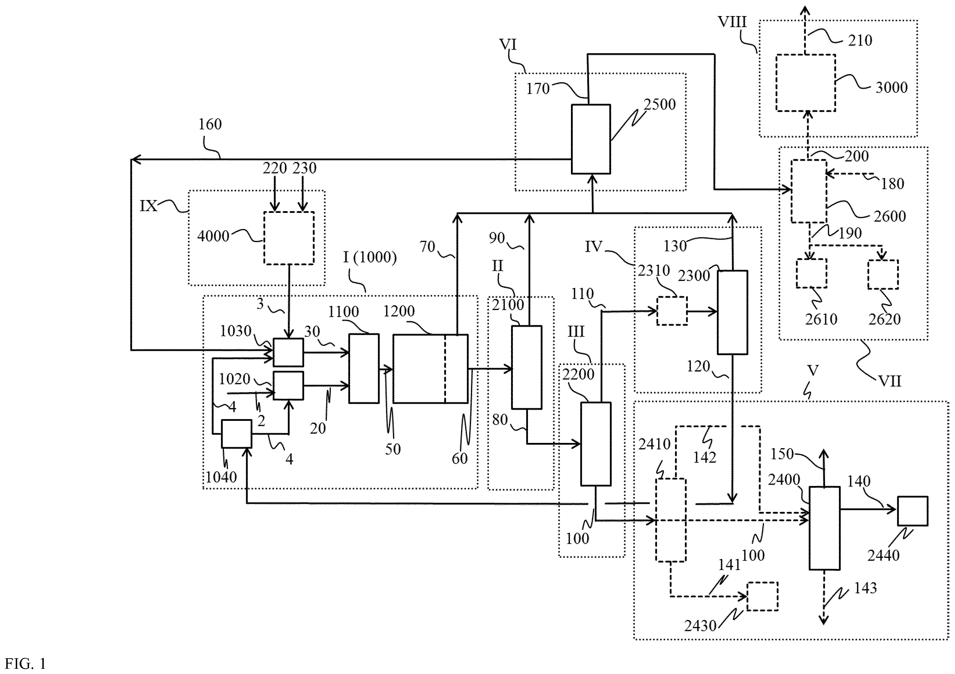

Das erfindungsgemäße Verfahren ist grundsätzlich zur Herstellung beliebiger aromatischer, aliphatischer und araliphatischer *Isocyanate (1)* geeignet. Bevorzugt eingesetzt wird das erfindungsgemäße Verfahren zur Herstellung von Methylendiphenyldiisocynat (aus Methylendiphenyldiamin), Polymethylenpolyphenylenisocyanat (aus Polymethylenpolyphenylenpolyamin), Gemischen aus Methylendiphenyldiisocynat und Polymethylenpolyphenylenisocyanat, Toluylendiisocyanat (aus Toluylendiamin), Xylylendiisocyanat (aus Xylylendiamin), Hexamethylendiisocyanat (aus Hexamethylendiamin), Isophorondiisocyanat (aus Isophorondiamin) und Naphthyldiisocyanat (aus Naphthyldiamin), besonders bevorzugt von Methylendiphenyldiisocyanat, Gemischen aus Methylendiphenyldiisocyanat und Polymethylenpolyphenylenpolyisocyanat sowie Toluylendiisocyanat. Ganz besonders bevorzugt eignet sich das erfindungsgemäße Verfahren zur Herstellung von Methylendiphenyldiisocyanat und Gemischen aus Methylendiphenyldiisocyanat und Polymethylenpolyphenylenpolyisocyanat. Isocyanate (1) fallen im erfindungsgemäßen Verfahren mindestens in Strom (140) an, wobei (140) auch summarisch für verschiedene Isocyanat-Ströme (1) *unterschiedlicher Isomerenzusammensetzung* (140-1, 140-2, ...) stehen kann, sofern die Destillation in 2400 nicht nur eine Reinigung, sondern auch eine Isomerentrennung beinhaltet (siehe die Detailerläuterungen weiter unten). Daneben enthält auch der in besonderen Ausführungsformen anfallende Strom 141 Isocyanate (1), wobei Strom 141 vor allem *polymere Isocyanatfraktionen* (das sind Isocyanate (1), die sich von polymerisierten Aminen ableiten lassen, z. B. Polyisocyanate der Diphenylmethanreihe mit drei oder mehr Benzol-"Kernen") umfasst. Auch der in besonderen Ausführungsformen anfallende "Rückstandsstrom" 143 (vgl. FIG. 1 und die Detailerläuterungen dazu weiter unten) enthält noch Isocyanat, welches aus diesem Strom gewonnen werden kann.

Geeignete erfindungsgemäß einsetzbare inerte *Lösungsmittel (4)* sind dabei unter den Reaktionsbedingungen inerte Lösungsmittel wie z. B. Monochlorbenzol, Dichlorbenzol (insbesondere das *ortho*-Isomer), Dioxan, Toluol, Xylol, Methylenchlorid, Perchlorethylen, Trichlorfluormethan oder Butylacetat. Das inerte Lösungsmittel (4) ist bevorzugt im Wesentlichen frei von Isocyanat (Massenanteil < 100 ppm angestrebt) und im Wesentlichen frei von Phosgen (Massenanteil < 100 ppm angestrebt), worauf bei Einsatz von Recycleströmen zu achten ist. Bevorzugt wird daher nach einem Verfahren, wie in EP 1 854 783 A2 beschrieben, gearbeitet. Die Lösungsmittel können einzeln oder als beliebige Gemische der beispielhaft genannten Lösungsmittel eingesetzt werden. Vorzugsweise werden Monochlorbenzol (MCB) oder *ortho*-Dichlorbenzol (ODB) eingesetzt.

Geeignete *Einrichtungen 1020, 1030 und 1040* sind beispielsweise Tankbehälter, wobei eine Einrichtung 1020, 1030 oder 1040 jeweils auch mehrere Tankbehälter (z. B. in Gestalt einer Tankfarm) umfassen kann.

Geeignete *Mischeinrichtungen 1100* sind aus dem Stand der Technik hinreichend bekannt.

Der *Reaktionsraum 1200* ist eine Verweilzeiteinrichtung, in welcher das in der Mischeinrichtung 1100 erhaltene Gemisch ausreichende Gelegenheit zum Ausreagieren erhält. Geeignete Apparate sind aus dem Stand der Technik hinlänglich bekannt.

Die Trennung des rohen Verfahrensproduktes in den flüssigen Strom 60 und den gasförmigen Strom 70 erfolgt im Reaktionsraum selber oder in einer nachgeschalteten *Abscheidereinrichtung 2010.* Es ist auch möglich, die Mischeinrichtung und den Reaktionsraum oder die Mischeinrichtung, den Reaktionsraum und die Abscheidereinrichtung oder den Reaktionsraum und die Abscheidereinrichtung in einen einzigen Apparat (z. B. in einen entsprechenden Reaktor) zu integrieren. Erfindungsgemäß können auch mehrere Mischeinrichtungen und/oder Reaktionsräume und/oder, sofern vorhanden, Abscheidervorrichtungen, seriell oder parallel geschaltet sein; z. B. in Form einer Kaskade mehrerer in Serie geschalteter Reaktoren. (Mit anderen Worten, in erfindungsgemäß verwendeten Bezeichnungen wie *"eine Mischeinrichtung"*, *"ein Reaktionsraum"* und dgl. ist das Wort *"ein(e)"* als unbestimmter Artikel und nicht als Zahlwort aufzufassen. Gleiches gilt selbstverständlich auch für andere Apparate auch anderer Anlagenteile).

Im Rahmen der vorliegenden Erfindung wird in allen Ausführungsformen die *"Außerbetriebnahme eines Anlagenteils oder mehrerer Anlagenteile aus den Schritten (I) bis (IX), insofern diese durchgeführt werden",* durchgeführt. Die *"Außerbetriebnahme"* eines Anlagenteils meint dabei dessen Stilllegung so, dass in dem Anlagenteil eine Revisions-, Reparatur-, Wartungs- oder Reinigungsmaßnahme durchgeführt werden kann. Die vorliegende Erfindung ist also mit solchen Produktionsstillständen befasst, die man als "Kurzstillstände" zu Revisions-, Reparatur-, Reinigungs- oder Wartungszwecken in Teilen der Anlage oder z. B. infolge eines zeitlich begrenzten Mangels an Einsatzstoffen oder Hilfsstoffen bezeichnen kann. Die vorliegende Erfindung ermöglicht es, dass bei einer derartigen Maßnahme nicht die gesamte Produktionsanlage außer Betrieb genommen werden muss. Vielmehr ermöglicht es die vorliegende Erfindung, dass nicht von der Revisions-, Reparatur-, Wartungs- oder Reinigungsmaßnahme betroffene Anlagenteile bzw. die entsprechenden Verfahrensschritte in sog. "Kreislauffahrweise" weiterbetrieben werden können (freilich ohne, dass weiteres Isocyanat gebildet wird). Damit bleibt eine Komplettabschaltung der Anlage auf besondere (seltene) Fälle wie etwa den der vollständigen Anlagenrevision beschränkt.

Der Begriff *"Außerbetriebnahme"* umfasst demnach erfindungsgemäß bei Vorhandensein von n Anlagenteilen im Sinne der vorliegenden Erfindung (siehe hierzu auch den folgenden Absatz), wobei n eine natürliche Zahl ist, die Außerbetriebnahme von maximal n - 1 dieser Anlagenteile. Erfindungsgemäß wird also mindestens ein Anlagenteil nicht "außer Betrieb genommen" (d. h. mindestens ein Anlagenteil wird nicht vollständig stillgelegt). Bevorzugt befasst sich die vorliegende Erfindung mit dem Fall der Außerbetriebnahme von 1 bis 2 Anlagenteilen, besonders bevorzugt von 1 Anlagenteil. Erfindungsgemäß wird daher bei Außerbetriebnahme eines Anlagenteils (oder mehrerer Anlagenteile, jedoch nicht aller Anlagenteile) in jedem Fall die Bildung weiteren Produkts unterbrochen (da der Massenstrom m₂ auf null reduziert wird und daher kein weiteres Isocyanat mehr produziert werden kann).

Unter "*Kreislauffahrweise*" wird im Rahmen dieser Erfindung verstanden, dass der Ausgangstrom eines Anlagenteils letztlich wieder als Eingangsstrom dieses Anlagenteils verwendet wird. Dies kann so geschehen, dass der Ausgangsstrom unmittelbar wieder in denselben Anlagenteil zurückgeführt wird. Es ist auch möglich (und bevorzugt), dass der Ausgangsstrom des ausgehenden Anlagenteils erst nach Durchlaufen eines weiteren Anlagenteils oder mehrerer weiterer Anlagenteile in den ausgehenden Anlagenteil zurückgeführt wird, wobei das weitere Anlagenteil oder die weiteren Anlagenteile dem ausgehenen Anlagenteil strömungstechnisch vor- oder nachgeschaltet sein können. Dabei ist mit "Anlagenteil" das dem jeweiligen Schritt (I) bis (IX), insofern diese durchgeführt werden, entsprechende Anlagenteil einer Anlage zur Herstellung von Isocyanat (und bevorzugt auch Phosgen) durch das erfindungsgemäße Verfahren gemeint. Umfasst der jeweilige Schritt (I) bis (IX) mehrere Apparate, so kann jeder dieser Apparate als Anlagenteil aufgefasst werden. Eine erfindungsgemäße Kreislauffahrweise kann also bspw. die gesamte Reaktionsstrecke 1000 oder auch nur Teile davon umfassen. Es ist ebenfalls möglich, dass Anlagenteile aus mehreren Schritten von einer Kreislauffahrweise umfasst sind.

Die *Destillationsvorrichtung 2100* (die sog. "Entphosgenierkolonne"), die *Destillationsvorrichtung 2200* (die sog. "Lösungsmittelkolonne"), die *Destillationsvorrichtung 2300* (der sog. "Lösungsmittelstripper"), die *Destillationsvorrichtung 2400* sowie die optional vorhandene *Einrichtung 2410* (die sog. "Polymerabtrennung"), die *Absorptionsvorrichtung 2500* (der sog. "Phosgenabsorber"), die *Absorptionsvorrichtung 2600* (die sog. "HCl-Absorptionskolonne"), die *Abgasreinigungsvorrichtung 3000* und die *Phosgenerzeugungsvorrichtung 4000* können grundsätzlich so ausgestaltet werden und im Regelbetrieb betrieben werden wie aus dem Stand der Technik bekannt. In apparativer Hinsicht wird es im Allgemeinen bei bestehenden Produktionsanlagen jedoch erforderlich sein, zusätzliche Rohrleitungen, Ventile und dgl. vorzusehen, um die erfindungsgemäßen Kreislauffahrweisen realisieren zu können. Dies und andere Aspekte der Erfindung werden weiter unten noch näher erläutert.

Es versteht sich von selbst, dass die Anlagenteile neben den zuvor explizit aufgeführten Apparaten auch Peripheriegeräte wie etwa Pumpen, Wärmeaustauscher und dergleichen mehr umfassen können.

Ausführungsformen der Erfindung werden nachfolgend beschrieben. Sie können beliebig miteinander kombiniert werden, sofern sich aus dem Kontext nicht eindeutig das Gegenteil ergibt.

Die Herstellung von Isocyanaten **im Regelbetrieb** lässt sich exemplarisch wie folgt zusammenfassen (vgl. hierzu auch FIG. 1):
a) Kernvorgang des Schritts I): das Amin wird mit Phosgen in einem Lösungsmittel zum entsprechenden Isocyanat umgesetzt, und das erhaltene rohe Verfahrensprodukt wird in einen flüssigen, das Roh-Isocyanat und Lösungsmittel (sowie Spuren an Phosgen und Chlorwasserstoff) enthaltenden Strom (60) und einen gasförmigen, Phosgen und Chlorwasserstoff (sowie Spuren an Lösungsmittel) enthaltenden Strom (70) aufgetrennt.
b) Kernvorgang des Schrittes II): Abtrennung weiteren bei der Reaktion entstandenen Chlorwasserstoffes zusammen mit nicht umgesetztem Phosgen aus dem Strom 60 in der sog. Entphosgenierkolonne 2100,
c) Kernvorgang des Schrittes III): Abtrennung von Lösungsmittel aus dem in Schritt II) erhaltenen flüssigen Strom 80 in der sog. Lösungsmittelkolonne 2200.
d) Kernvorgang des Schrittes IV): Abtrennung von Phosgen aus dem in Schritt III) erhaltenen gasförmigen Strom 110, bevorzugt nach dessen Verflüssigung in einem Kondensator (2310), im sog. Lösungsmittelstripper 2300.
e) Kernvorgang des Schrittes V): Abtrennung von Lösungsmittel aus dem im Schritt III) erhaltenen flüssigen Strom 100 mittels Destillation (2400), optional umfassend die Abtrennung polymerer Isocyanatfraktionen (141), unter Erhalt des Isocyanatstroms 140.
f) Kernvorgang des Schrittes VI): Absorption der gasförmigen Ströme aus den Schritten I), II) und III) in einem Lösungsmittel im sog. Phosgenabsorber 2500.
g) Kernvorgang des Schrittes VII) (optional): Absorption des in Schritt VI) erhaltenen Gasstroms 170 in Wasser oder verdünnter Salzsäure in der sog. HCl-Absorptionskolonne 2600.
h) Kernvorgang des Schrittes VIII) (optional): Reinigung der Abgasströme aus Schritt VII), bevorzugt der Abgasströme aus allen Schritten, in einer Abgasreinigungsvorrichtung 3000.
i) Kernvorgang des Schrittes IX) (optional): Herstellung von Phosgen aus Chlor und Kohlenstoffmonoxid in einer Phosgenerzeugungsvorrichtung 4000.

Die kontinuierliche Produktion des Isocyanats **in a)** erfolgt in einer Reaktionsstrecke nach einem aus dem Stand der Technik bekannten Verfahren. Geeignete Verfahren sind beispielsweise in EP 2 077 150 B1, EP 1 616 857 A1, EP 1 873 142 A1, EP 0 716 079 B1 oder EP 0 314 985 B1 beschrieben. Konzentrationen und Mengenströme der Edukte Amin und Phosgen werden dabei jedoch bevorzugt so gewählt, dass sich in der Mischzone ein molares Verhältnis von Phosgen zu primären Aminogruppen von 1,1 : 1 bis 30 : 1, besonders bevorzugt von 1,25 : 1 bis 3 : 1, einstellt. Alle Verfahren für die Produktion eines Isocyanats in der Flüssigphase liefern ein rohes Verfahrensprodukt, das in eine Flüssigphase (60), enthaltend neben dem gewünschten Isoyanat gelösten Chlorwasserstoff, überschüssiges gelöstes Phosgen und Lösungsmittel, und eine Gasphase (70), enthaltend Chlorwasserstoffgas, überschüssiges gasförmiges Phosgen und gasförmiges Lösungsmittel, zerfällt bzw. aufgetrennt wird. Erfindungsgemäß umfasst ist auch eine Ausführungsform bei der der erhaltene Roh-Isocyanat-Strom 60 vor der weiteren Verarbeitung in b) eine Vorrichtung zur Spaltung von Carbaminsäurechlorid durchläuft.

Die weitere Abtrennung von Chlorwasserstoff und Phosgen aus dem flüssigen Roh-Isocyanat-Strom 60 in der sog. Entphosgenierkolonne 2100 **in b)** kann nach einem beliebigen aus dem Stand der Technik bekannten Verfahren erfolgen, bevorzugt wie in DE-A-10260084 beschrieben.

Die weitere Abtrennung von Lösungsmittel aus so erhaltenen flüssigen Isocyanat-Strom 80 in der sog. Lösungsmittelkolonne 2200 **in c)** kann nach einem beliebigen aus dem Stand der Technik bekannten Verfahren erfolgen, bevorzugt wie in EP 1 854 783 B1 beschrieben.

Die Abtrennung von Phosgen aus dem so erhaltenen gasförmigen Lösungsmittel-Strom 110, bevorzugt nach dessen Verflüssigung in einem Kondensator (2310), im sog. Lösungsmittelstripper 2300 **in d)** kann nach einem beliebigen aus dem Stand der Technik bekannten Verfahren erfolgen, bevorzugt wie in EP 1 854 783 B1 beschrieben.

Die Abtrennung von Lösungsmittel aus dem im Schritt III) erhaltenen flüssigen Isocyanat-Strom 100 **in e),** ggf. umfassend eine Polymerabtrennung, kann nach einem beliebigen aus dem Stand der Technik bekannten Verfahren erfolgen. Geeignete Verfahren sind beschrieben in EP 1 854 783 A2 und EP 1 506 957 A1, oder auch in EP 1 371 635 B1.

Die Absorption der gasförmigen Ströme aus den Schritten I), II) und III) in einem Lösungsmittel im sog. Phosgenabsorber 2500 **in f)** kann nach einem beliebigen aus dem Stand der Technik bekannten Verfahren erfolgen, bevorzugt wie in DE-A-10260084 oder EP 2 093 215 A1 beschrieben.

Die Absorption des so erhaltenen HCl-Gasstroms 170 in Wasser oder verdünnter Salzsäure in der sog. HCl-Absorptionskolonne 2600 zur Gewinnung von Salzsäure **in g)** kann nach einem beliebigen aus dem Stand der Technik bekannten Verfahren erfolgen. Bevorzugt sind Verfahrensweisen, wie in EP 2 021 275 B1 und EP 1 743 882 B1 beschrieben.

Der Verfahrensschritt der Prozessabgasbehandlung **in h)** kann gemäß einem aus dem Stand der Technik bekannten Verfahren erfolgen.

Die kontinuierliche Produktion des Phosgens **in i)** kann grundsätzlich nach allen aus dem Stand der Technik bekannten Verfahren durchgeführt werden. Für die Ausführung dieses Schritts des erfindungsgemäßen Verfahrens kann ein "Kaltvereiniger" entsprechend EP 1 640 341 B1 oder ein "Heißvereiniger" entsprechend EP 0 134 506 B1 benutzt werden. Bei der sogenannten - bevorzugt eingesetzten - Heißvereinigung (siehe EP 0 134 506 B1) wird Phosgen durch Umsetzung von Chlor mit Kohlenmonoxid in Aktivkohle als Katalysator enthaltenen Rohrreaktoren unter gleichzeitiger Nutzung der anfallenden Reaktionswärme zur Erzeugung von Dampf umgesetzt. Dabei wird in einem ersten, gekörnte Aktivkohle enthaltenen Rohrreaktor mit einem lichten Rohrdurchmesser von maximal 100 mm 95 Vol.-% bis 98 Vol.-% des eingesetzten Chlors mit überschüssigem Kohlenmonoxid bei Reaktionstemperaturen von über 250 °C zu Phosgen umgesetzt. Die hierbei anfallende Reaktionswärme wird durch Verdampfungskühlung einer bei 150 °C bis 320 °C siedenden Flüssigkeit oder mit einer nicht-siedenden Flüssigkeit abgeführt, deren Temperatur am Reaktoraustritt mittels Zwangsumlaufpumpen und Temperatursteuerung bei 150 °C bis 320 °C gehalten wird. Der den Reaktor verlassende flüssige oder dampfförmige Wärmeträger wird in einem mit Wasser als Kühlmedium beschickten Wärmetauscher unter Erzeugung von Dampf kondensiert und / oder auf eine unter der Temperatur des Wärmeträgers am Reaktoraustritt liegende Temperatur abgekühlt und in den Reaktor zurückgeführt. Die den Reaktor verlassenden Reaktionsgase werden auf eine Temperatur von 50 °C bis 120 °C abgekühlt und anschließend in einen gekörnte Aktivkohle enthaltenden zweiten Reaktor geleitet, dessen Temperatur auf 50 °C bis 100 °C thermostatisch eingestellt und in dem die Umsetzung zu Ende geführt wird, so dass das den zweiten Reaktor verlassende Phosgen einen Restchlorgehalt von weniger als 50 ppmv aufweist. Das am Kopf des Reaktors austretende Phosgen wird, wie oben beschrieben, kondensiert.

Die erfindungsgemäße **Vorgehensweise bei Außerbetriebnahme eines oder mehrerer Anlagenteile** wird nachfolgend näher beschrieben:
Durch das Abstellen von m₂, also des Massenstroms an Amin, das optional mit Lösungsmittel verdünnt ist, in die Mischeinrichtung der Reaktionsstrecke aus Schritt I), wird sichergestellt, dass während der Unterbrechung, die, wie oben beschrieben, zum Zwecke der Revision, Wartung, Reparatur und/oder Reinigung eines Teiles der Herstellungsanlage durchgeführt wird oder durch einen Mangel an Rohstoff(en) und/oder Hilfsstoffe(n) verursacht wurde, die Reaktion in Schritt I) nicht weiter stattfindet. Hierbei ist es insbesondere bevorzugt, dass bei der Abstellung der Zufuhr von Amin die Zufuhr von Phosgen und Lösungsmittel nicht gleichzeitig mit unterbrochen wird. Vielmehr ist es bevorzugt, die Zufuhr des Lösungsmittels, das zur Verdünnung des Amins und des Phosgens dient und die Zufuhr von Phosgen, das mit Lösungsmittel verdünnt ist, zeitverzögert (vorzugsweise mindestens 15 min, weiter bevorzugt mindestens 30 min und besonders bevorzugt mindestens 60 min nachdem m₂ gleich null ist) zu unterbrechen. Damit wird gewährleistet, dass die Amin-Rohrleitung, die Mischeinrichtung und der Reaktor von Amin freigespült werden. Sollte die Unterbrechung länger als 3 Stunden dauern, dann kann auch Phosgen abgestellt werden, sodass dann nur noch Lösungsmittel über den Phosgenweg in die Mischeinrichtung und den Reaktionsraum fließt. Nachdem die Phosgenrohrleitung Phosgen-frei ist, kann man optional die Mischeinrichtung umfahren und lässt Lösungsmittel direkt in den ersten Reaktor laufen. Dann kann die Reaktionsstrecke des Schritts (I) in Kreislauffahrweise gebracht werden, d. h. den ausgetragenen, Lösungsmittel, Phosgen und Isocyanat enthaltenden Strom als Eingangsstrom der Entphosgenierkolonne aus Schritt (II) zu verwenden und von dort, wie bereits beschrieben, über die Destillation aus Schritt (V), die Strippkolonne aus Schritt (VI) und zurück zum ersten Reaktor der Reaktionsstrecke in Kreislauffahrweise zu fahren. Damit kann vorteilhafterweise erreicht werden, dass zum einen gegebenenfalls die Bildung von Nebenprodukten verhindert werden kann und zum anderen beispielsweise ein Verklumpen des Reaktionsgemisches unterbleibt. Damit kann effizient ein Verunreinigen des gewünschten Produktes, ein Verstopfen von Anlagenteilen wie beispielsweise Rohrleitungen, Ventilen und Pumpen, sowie Anbackungen und das Erzeugen von Ausschuss vermieden werden.

Wie bereits erwähnt, ist es möglich, die Isocyanat-Produktion in zwei oder mehr parallel geschalteten Produktionsstraßen (also Anlagen umfassend mindestens die Anlagenteile aus I) bis VI)) durchzuführen. Es ist ebenfalls denkbar, dass nur Teile einer Produktionsstraße (z. B. die Reaktorlinie umfassend die Mischeinrichtung 1100 und der Reaktionsraum 1200) mehrfach vorhanden sind und parallel betrieben werden. In solchen Fällen können zur erfindungsgemäßen Ausgestaltung einer Produktionsunterbrechung zunächst in einer Produktionsstraße bzw. Reaktorlinie ein Anlagenteil oder mehrere Anlagenteile außer Betrieb genommen werden und die anderen Anlagenteile dieser Produktionsstraße(n) bzw. Reaktorlinie(n), insofern erforderlich, in erfindungsgemäßer Kreislauffahrweise betrieben werden. Nicht von der Außerbetriebnahme betroffene Produktionsstraßen können weiter betrieben werden. Ebenfalls weiterbetrieben werden können im Falle mehrerer parallel betriebener Reaktorlinien innerhalb einer Produktionsstraße alle nicht von einer Produktionsunterbrechung betroffenen Reaktorlinien und die übrigen Anlagenteile der betroffenen Produktionsstraße. Alternativ ist es im Rahmen der vorliegenden Erfindung aber auch möglich, in allen Isocyanat-Produktionsstraßen bzw. Reaktorlinien, insofern erforderlich, die Produktion zu unterbrechen und nicht vollständig außer Betrieb genommene Anlagenteile gleichzeitig oder zeitnah nacheinander in Kreislauffahrweise zu überführen.

Ein weiterer Gegenstand der vorliegenden Erfindung ist daher eine **Anlage (10000) zur Herstellung von Isocyanaten (1) in der Flüssigphase,** umfassend die Anlagenteile
I) eine **Reaktionsstrecke (1000)** umfassend
   I.1) eine Einrichtung (1020) zur Bereitstellung eines Amins (2), bevorzugt in Form einer Aminlösung (20) in einem Lösungsmittel (4),
   I.2) eine Einrichtung (1030) zur Bereitstellung von Phosgen (3), bevorzugt in Form einer Phosgenlösung (30) in einem Lösungsmittel (4),
   I.3) eine Einrichtung (1040) zur Bereitstellung eines Lösungsmittels (4),
   I.4) eine Mischeinrichtung (1100) zur Vermischung von Amin (2) oder Aminlösung (20) mit Phosgen (3) oder Phosgenlösung (30) und optional weiterem Lösungsmittel (4) und
   I.5) einen strömungstechnisch nach der Mischeinrichtung angeordneten Reaktionsraum (1200) zur Durchfühung der Phosgenierung, dem optional eine Abscheidereinrichtung (1210) nachgeschaltet ist, wobei der Reaktionsraum oder die Abscheidervorrichtung mit Abfuhrleitungen für einen flüssigen Strom (60) und einen gasförmigen Strom (70) versehen sind;
II) eine **Destillationsvorrichtung (2100 - "Entphosgenierkolonne")** zur Auftrennung des flüssigen Stroms (60) in einen flüssigen Strom (80) und einen gasförmigen Strom (90);
III) eine **Destillationsvorrichtung (2200 - "Lösungsmittelkolonne")** zur Trennung des flüssigen Stroms (80) in einen gasförmigen Strom (110) und einen flüssigen Strom (100);
IV) eine **Destillationsvorrichtung (2300 - "Lösungsmittelstripper")** zur Trennung des gasförmigen Stroms (110), bevorzugt nach dessen Verflüssigung in einem **Kondensator (2310),** in einen flüssigen Strom (120) und einen gasförmigen Strom (130);
V) eine **Destillationsvorrichtung (2400)** zur Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, optional umfassend eine vorgeschaltete **Einrichtung zur Polymerabtrennung (2410 - "Polymerabtrennung", PMA)** zur Abtrennung von polymeren Isocyanatfraktionen (141);
VI) eine **Vorrichtung zur Absorption (2500 - "Phosgenabsorber")** der gasförmigen Ströme (70), (90) und (130) in Lösungsmittel unter Erhalt eines flüssigen Stroms (160) und eines gasförmigen Stroms (170);
VII) optional (und bevorzugt) eine **Vorrichtung zur Absorption (2600 - "HCl-Absorptionskolonne")** des gasförmigen Stroms (170) in Wasser;
VIII) optional (und bevorzugt) eine **Vorrichtung zur Aufarbeitung von Abgasströmen (3000),** welche zur Aufarbeitung von Abgasströmen mindestens aus VII), bevorzugt zur Aufarbeitung von Abgasströmen aus allen vorhandenen Anlagenteilen, ausgestaltet ist;
IX) optional (und bevorzugt) eine **Vorrichtung (4000) zur Herstellung von Phosgen,** welche mit der Einrichtung (1030) verbunden ist;
wobei
**die Anlage (10000) derart ausgestaltet ist, dass** bei einer Außerbetriebnahme eines oder mehrerer der Anlagenteile I) bis IX), insofern diese vorhanden sind,
der Massenstrom m₂ auf null reduziert wird und in mindestens einem der nicht außer Betrieb genommenen Anlagenteile der Ausgangsstrom dieses mindestens einen nicht außer Betrieb genommenen Anlagenteils
(i) in den jeweiligen Anlagenteil oder
(ii) in einen strömungstechnisch davor oder dahinter liegenden Anlagenteil geführt und von dort, optional über weitere nicht außer Betrieb genommene Anlagenteile, in den ausgehenden Anlagenteil
zurückgeführt werden kann (sog. "Kreislauffahrweise").

Die Ausgestaltung der Anlage zur Unterbrechung der Aminzufuhr bei Außerbetriebnahme eines oder mehrerer Anlagenteile wird dabei bevorzugt durch prozessleittechnische Einrichtungen realisiert. Geeignete Soft- und Hardware-Produkte sind kommerziell erhältlich und dem Fachmann bekannt.

In einer bevorzugten Ausführungsform der vorliegenden Erfindung können die Anlagenteile unabhängig voneinander auf aus rückgeführten Ausgangsströmen bestehenden Eingangsströmen geschaltet werden. In einer weiteren bevorzugten Ausführungsform können verschiedene Anlagenteile gleichzeitig auf aus rückgeführten Ausgangsströmen bestehenden Eingangsströmen geschaltet werden. Dabei ist es bevorzugt, dass in jedem anderen, nicht außer Betrieb genommenen Anlagenteil der Ausgangsstrom, wie weiter oben erläutert, als Eingangsstrom dieses oder eines anderen Anlagenteils verwendet werden kann und so letztlich in den ausgehenden Anlagenteil zurückgeführt wird. Weiterhin ist es bevorzugt, dass jedes nicht außer Betrieb genommene Anlagenteil auch Teil von zwei unterschiedlichen Kreislauffahrweisen sein kann. Wenn die Anlage ein Anlagenteil zur Phosgenerzeugung 4000 umfasst (Phosgenerzeugungsvorrichtung, IX), so ist es bevorzugt, dass dieses Anlagenteil bei einer Unterbrechung des Verfahrens lediglich abgestellt, aber nicht auf Kreislauffahrweise eingestellt wird. Für das Anlagenteil der Abgasreinigungsanlage 3000 (VIII) gilt, dass dieses Anlagenteil bevorzugt bei jedweder Unterbrechung des Verfahrens in Betrieb bleibt (Ausnahme: Totalstillstand für z. B. einen Revisionsstillstand). Bei Ausfall der Abgasreinigungsanlage 3000 (VIII) gilt, dass die komplette Anlage mit all ihren Anlagenteilen abgestellt wird.

### Die erfindungsgemäße Anlage wird nachfolgend näher erläutert:

Vorzugsweise umfasst die **Reaktionsstrecke 1000 (I)** einen Aminvorlagetank, eine absperrbare Amindosierleitung und eine absperrbare Lösungsmitteldosierleitung zum Amin/Lösungsmittel-Mischer (1020), eine Rohrleitung für das Amin/Lösungsmittel-Gemisch zum Mischapparat von Phosgen- und Aminstrom (1100), einen Verflüssiger (nicht gezeigt) für das in dem Phosgengenerator (4000) erzeugte Phosgen, einen Vorlagetank zur Herstellung eines Phosgen/Lösungsmittel-Gemisches (1030), eine absperrbare Phosgen/Lösungsmittel-Dosierleitung zum Mischapparat von Phosgen und Amin (1100) sowie eine absperrbare Lösungsmitteldosierleitung (nicht gezeigt) in die Verbindung zwischen Mischapparat der Phosgen- und Amin-haltigen Eingangsströme (1100) und dem beheizbaren Reaktionsraum (1200) der Reaktionsstrecke (1000).

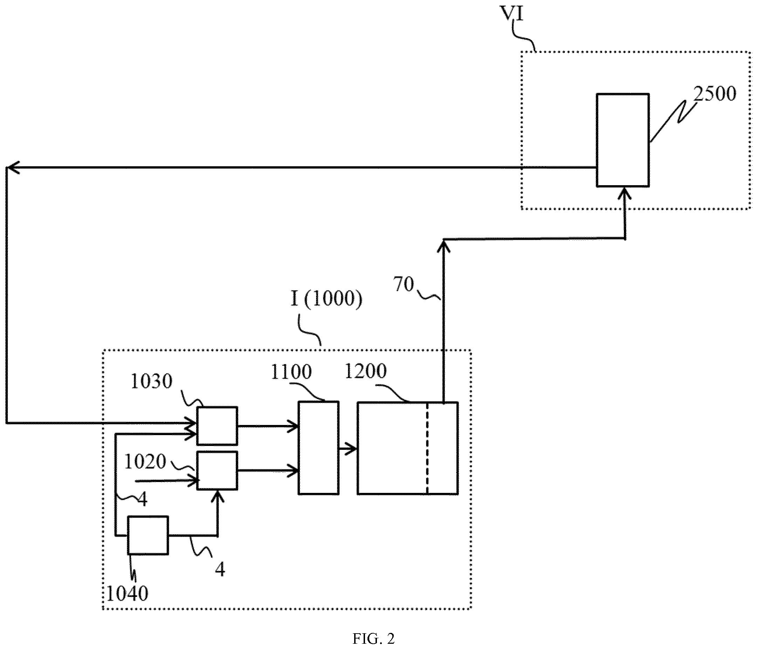

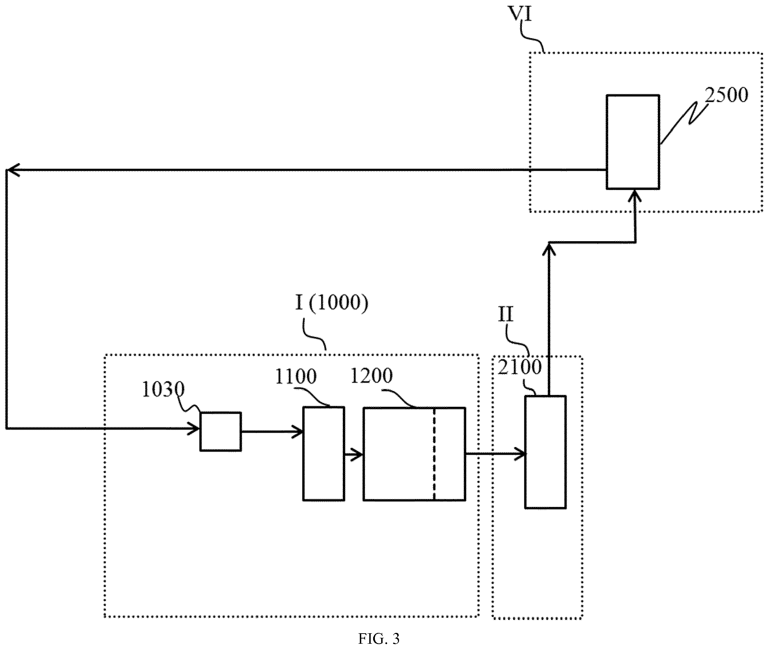

Es ist bevorzugt, die erfindungsgemäße Anlage so auszugestalten (insbesondere bevorzugt durch prozessleittechnische Einrichtungen) und so zu betreiben, dass bei Außerbetriebnahme eines oder mehrerer von der Reaktionsstrecke I) verschiedener Anlagenteile (zunächst) nur der Aminstrom in den Mischapparat der Reaktionsstrecke I) abgestellt wird. Die Amindosierleitung wird vorzugsweise mit einem von Phosgen und Isocyanat weitgehend befreiten Lösungsmittelstrom (120) von Amin freigespült, und zwar für mindestens 10 min (in bevorzugter Ausgestaltung der erfindungsgemäßen Anlage entsprechend dem Durchfluss von einem Sechstel der Lösungsmittelmenge, die pro Stunde bei Nennkapazität durch die Amindosierleitung gefahren wird), bevorzugt mindestens 20 min (in bevorzugter Ausgestaltung der erfindungsgemäßen Anlage entsprechend dem Durchfluss von einem Drittel der Lösungsmittelmenge, die pro Stunde bei Nennkapazität durch die Amindosierleitung gefahren wird). Der Phosgen/Lösungsmittelstrom (30) läuft vorzugsweise 60 min (in bevorzugter Ausgestaltung der erfindungsgemäßen Anlage entsprechend dem Durchfluss der Menge an Phosgen/Lösungsmittelstrom, die pro Stunde bei Nennkapazität durch die Phosgendosierleitung gefahren wird), bevorzugt mindestens 3 Stunden (in bevorzugter Ausgestaltung der erfindungsgemäßen Anlage entsprechend dem Durchfluss der dreifachen Menge an Phosgen/Lösungsmittelstrom, die pro Stunde bei Nennkapazität durch die Phosgendosierleitung gefahren wird), weiter, um die Umsetzung des Amins in der Reaktionsstrecke sicherzustellen. Wenn kein anderer Abnehmer für Phosgen bereit steht, muss, sofern vorhanden, bei Abstellung der Aminzufuhr auch der Phosgengenerator (4000) außer Betrieb genommen werden. Sollte der Produktionsstillstand länger als 12 Stunden dauern, dann wird auch das Phosgen/Lösungsmittel-Gemisch (30) abgestellt und die Phosgen/Lösungsmittel-Dosierleitung mit einem von Phosgen und Isocyanat weitgehend befreiten Lösungsmittel (160) durch die Reaktionsstrecke I) gespült. Bis zu einer Betriebsunterbrechung von 12 Stunden wird der Phosgen/Lösungsmittelstrom (30) zum Mischapparat 1100, durch den Reaktionsraum 1200, dann einerseits über die Gasphase (70) zur Phosgenabsorption (VI) und andererseits über den Flüssigablauf (60) des Reaktionsraums zum Entphosgenierer (II) und von dort über dessen Gasphase ebenfalls zur Phosgenabsorption (VI) geführt. In der Phosgenabsorption (VI) werden die gesammelten Gasphasen kondensiert, und restlicher Chlorwasserstoff wird abgetrennt. Die flüssige Phase wird zum Phosgenlösungstank 1030 und von dort zurück zum Mischapparat 1100 der Reaktionsstrecke I) geführt, wodurch die Kreislauffahrweise etabliert ist (vgl. **FIG. 2** **und** **3**).

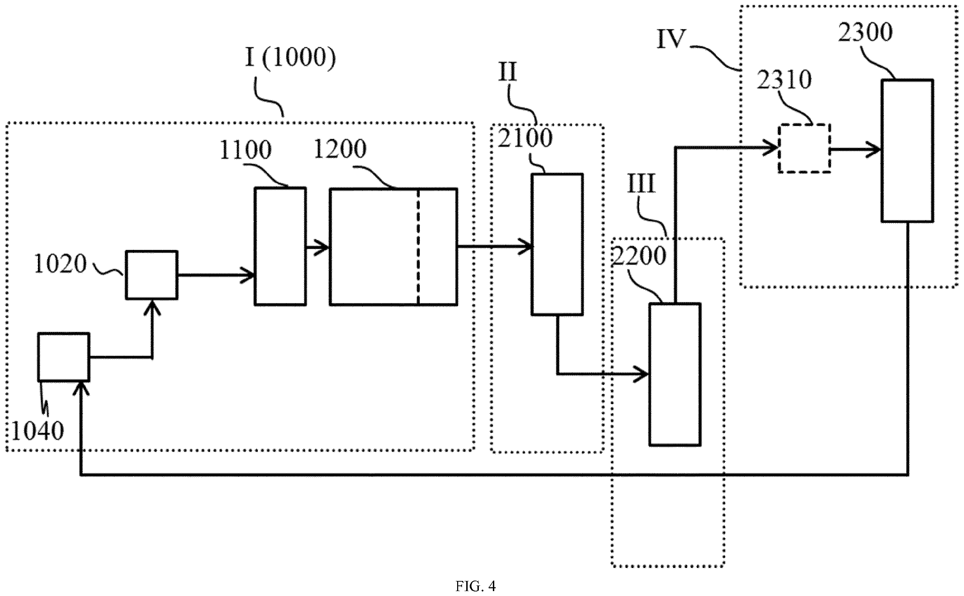

Die flüssige Phase des Entphosgenierers (II) wird zur Lösungsmitteldestillation (III) geführt, und das Lösungsmittel-haltige Kopfprodukt wird über die Lösungsmittelreinigung (IV) als flüssige Phase, die weitestgehend von Phosgen und Isocyanaten befreit ist, zum Lösungsmitteltank (1040) ebenfalls als Kreislauf zurück zum Mischapparat (1100) der Reaktionsstrecke I) gefahren (**FIG. 4**), während die gasförmige Phosgen- und Lösungsmittel-haltige Phase der Lösungsmittelreinigung (IV) zur Phosgenabsorption (VI) ebenfalls im Kreis gefahren wird.

Bei Arbeiten am Mischapparat kann dieser außer Betrieb genommen werden und die Kreislauffahrweise mit dem Lösungsmittel aus der Lösungsmittelreinigung (IV), über den Lösungsmitteltank (1040) und dann hinter dem abgesperrten Mischapparat in den Einlauf des Reaktionsraums 1200 und über die Entphosgenierkolonne (II), und die Lösungsmitteldestillation (III) zurück zur Lösungsmittelreinigung (IV) betrieben werden. Verdampfte Anteile des im Kreis geführen Lösungsmittels werden über die Gasphase der Entphosgenierkolonne (II) und die flüssige Phase des Phosgenabsorbers (VI) in den Phosgenlösungstank (1030) gefahren.

Des Weiteren ist es bevorzugt, dass die **Destillationsvorrichtung 2100 (II)** der erfindungsgemäßen Anlage, in der das Roh-Isocyanat (60) aus I) in eine flüssige, organische Phase (80), umfassend Roh-Isocyanat und Lösungsmittel und eine gasförmige Phase (90), umfassend Phosgen und Spuren Lösungsmittel sowie Chlorwasserstoff, getrennt wird, eine Entphosgenierkolonne mit Verdampfer umfasst.

Die erfindungsgemäße Anlage ist bevorzugt so ausgestaltet (insbesondere bevorzugt durch prozessleittechnische Einrichtungen) und wird bevorzugt so betrieben, dass bei Außerbetriebnahme eines oder mehrerer von der Entphosgenierkolonne (II) verschiedener Anlagenteile dieses Anlagenteil weiter in Betrieb bleibt, um bis zu einer Betriebsunterbrechung von 12 Stunden den Flüssigablauf aus dem Reaktionsraum 1200 aufzunehmen, wobei die Gasphase, die nach Abstellung des Aminstromes zunehmend Chlorwasserstoff-frei wird, über den Phosgenabsorber (VI), den Phosgenlösungstank 1030, die Mischeinrichtung 1100 und den Reaktionsraum 1200 zurück zur Entphosgenierkolonne (II) im Kreis gefahren wird. Dabei wird die flüssige, organische Phase, die nach Abstellung des Aminstromes zunehmend Roh-Isocyanat-frei wird, über die Destillationsvorrichtung (III), die Lösungsmittelreinigung (IV) und den Lösungsmitteltank (1040) und von dort über den Mischer und den Reaktionsraum in die Entphosgenierkolonne (II) im Kreis gefahren (**FIG. 4**).

Bevorzugt umfasst die **Destillationsvorrichtung 2200 (III)** der erfindungsgemäßen Anlage, in der das Roh-Isocyanat (80) aus II) in eine flüssige, Roh-Isocyanat und Lösungsmittel enthaltende Phase (100) und eine gasförmige, Lösungsmittel und restliches Phosgen enthaltende Phase (110), getrennt wird, zwei hintereinandergeschaltete Destillationskolonnen (in FIG. 1 nicht gezeigt), die an einem Vakuumsystem angeschlossen sind und mittels des Vakuumsystems bei Unterdruck betrieben und in die Vorrichtung zur Abgasreinigung (XIII) entlüftet werden, mit jeweils einem Verdampfer, einem Brüdenkondensationssystem und einem Wäscher, der mit Lösungsmittel beaufschlagt wird, um Mitriss von Roh-Isocyanat in die Lösungsmittelreinigung (IV) zu verhindern.

Die erfindungsgemäße Anlage ist bevorzugt so ausgestaltet (insbesondere bevorzugt durch prozessleittechnische Einrichtungen) und wird bevorzugt so betrieben, dass bei Außerbetriebnahme eines oder mehrerer von der Destillationsvorrichtung (III) verschiedener Anlagenteile sie weiter im Unterdruck in Betrieb bleibt, um bis zu einer Betriebsunterbrechung von 12 Stunden den Flüssigablauf der Destillationsvorrichtung (II) aufnehmen zu können, wobei die von Spuren an Isocyanat befreite Gasphase kondensiert und über die Lösungsmittelreinigung (IV) und den daran angeschlossenen Lösungsmitteltank (1040), über den Mischer und den Reaktionsraum in den Trennapparat (II) zurück zur Destillationsvorrichtung (III) im Kreis gefahren wird. Dabei wird die flüssige, organische Phase über einen Roh-Isocyanat-Zwischenlagertank, der lediglich für eine Kreislauffahrweise von Schritt III) und/oder Schritt II) und/oder Schritt V) vorgesehen ist, und von dort in die Einlaufleitung zwischen der Destillationsvorrichtung (II) und der Destillationsvorrichtung (III) zurück in die Destillationsvorrichtung (III) im Kreis gefahren. Alternativ kann die flüssige, organische Phase über die erste Kolonne (2410) der Isocyanatreinigung (V), über den Roh-Isocyanat-Zwischenlagertank und von dort in die Einlaufleitung zwischen der Destillationsvorrichtung (II) und der Destillationsvorrichtung (III) zurück in die Destillationsvorrichtung (III) im Kreis gefahren werden.

Bevorzugt umfasst die **Destillationsvorrichtung 2300 (IV)** der erfindungsgemäßen Anlage, in der das vorgereinigte Lösungsmittel (110) aus Schritt III) von restlichem Phosgen befreit wird, eine mit einem Vakuumsystem versehene Strippkolonne mit Pumpenvorlage und Verdampfer, einer Pumpenvorlage für die Destillatausspeisung und einem Brüdenkondensationssystem, die mittels des Vakuumsystems bei Unterdruck betrieben und in die Vorrichtung zur Abgasreinigung (VIII) entlüftet wird.

Die erfindungsgemäße Anlage ist bevorzugt so ausgestaltet (insbesondere bevorzugt durch prozessleittechnische Einrichtungen) und wird bevorzugt so betrieben, dass bei Außerbetriebnahme eines oder mehrerer von der Destillationsvorrichtung (IV) verschiedener Anlagenteile sie weiter im Unterdruck in Betrieb bleibt, um bis zu einer Betriebsunterbrechung von 12 Stunden die in 2310 kondensierten Brüden aus (III) aufnehmen zu können, wobei das von Resten an Phosgen befreite Lösungsmittel über den Lösungsmitteltank (1040), die Mischeinrichtung 1100 und den Reaktionsraum 1200 in die Destillationsvorrichtung (II) und von dort über die Destillationsvorrichtung (III) zurück zur Destillationsvorrichtung (IV) im Kreis gefahren wird (vgl. **FIG. 4**). Alternativ kann das von Resten an Phosgen befreite Lösungsmittel über den Roh-Isocyanat-Zwischenlagertank aus (III), in die Einlaufleitung zwischen der Destillationsvorrichtung (II) und der Destillationsvorrichtung (III), über die Destillationsvorrichtung (III) zurück in die Lösungsmittelreinigung (IV) im Kreis gefahren werden.

In der **Destillationsvorrichtung (V)** der erfindungsgemäßen Anlage wird das Roh-Isocyanat (100) aus Schritt III) von Spuren an Lösungsmittel und gegebenenfalls Nebenkomponenten befreit und in das/oder die Isocyanat(e) der gewünschten Reinheit und Zusammensetzung aufgetrennt. Die Destillationsvorrichtung (V) umfasst mindestens die **Destillationsvorrichtung 2400** und in besonderen Ausführungsformen ebenfalls die **Einrichtung zur Abtrennung polymerer Isocyanatfraktionen 2410.**

In einer besonderen Ausgestaltung der Erfindung, die insbesondere für die **Herstellung von Di- und Polyisocyanaten der Diphenylmethanreihe** (nachfolgend summarisch MDI) geeignet ist, umfasst die Destillationsvorrichtung (V) die **Einrichtung zur Polymerabtrennung (2410).** In dieser Ausführungsform wird das Roh-Isocyanat aus III) (Strom 100 in FIG. 1) über eine Einspeisepumpe (nicht gezeigt in den Abbildungen) in diese erste Destillationseinrichtung (2410) mit Vakuumsystem geführt (durchgezogen gezeichnete Leitung für Strom 100 in FIG. 1; die gestrichelt gezeichnete Leitung für Strom 100 in FIG. 1 ist in dieser Ausführungsform nicht vorhanden). Die Einrichtung (2410) umfasst ferner einen Verdampfer (nicht gezeigt in den Abbildungen), eine mit Packungen gefüllte Kolonne, eine Pumpenvorlage für den Rücklauf auf die Packung der Kolonne (nicht gezeigt in den Abbildungen), eine Entlüftung (nicht eingezeichnet in den Abbildungen) in die Vorrichtung zur Abgasreinigung (VIII), ein Brüdenkondensationssystem mit einem Brüdenabzug für restliche Spuren an Lösungsmittel (ebenfalls nicht eingezeichnet in den Abbildungen), eine Destillat-Entnahmestelle für monomore Isocyanatfraktionen 142 (d. h. im Falle der Herstellung von Di- und Polyisocyanaten der Diphenylmethanreihe für Methylendiphenyldiisocyanat [MMDI - "monomeres MDI"]). Außerdem umfasst die Einrichtung (2410) einen Dampferzeuger und eine Kreislaufpumpe (beide nicht gezeigt in den Abbildungen) zum Produkttank (2430) zur Aufnahme des Sumpf-Ausgangsstroms der Einrichtung zur Polymerabtrennung (2410). Dieser Sumpf-Ausgangsstrom enthält die polymeren Isocyanatfraktionen (141), also im Falle der Herstellung von Di- und Polyisocyanaten der Diphenylmethanreihe ein Gemisch aus Polymethylen-Polyphenylen-Polyisocyanat [PMDI - "polymeres MDI"] und Methylendiphenyldiisocyanat [MMDI - "monomeres MDI"].

Die Destillationsvorrichtung (V) umfasst in dieser Ausführungsform ferner eine Pumpenvorlage mit Kreislaufpumpe (nicht gezeigt in den Abbildungen), in welche die monomeren Isocyanatfraktionen (142) aus der Destillat-Entnahmestelle der Einrichtung zur Polymerabtrennung (2410) eingespeist werden. Von dort wird der Strom (142) in eine zweite **Destillationsvorrichtung (2400)** zur Gewinnung von zusätzlichen Isocyanat-Zusammensetzungen und Isocyanat-Qualitäten, d. h. im Falle der beispielhaft genannten Herstellung von Di- und Polyisocyanaten der Diphenylmethanreihe zur Gewinnung von (polymerfreien) Methylendiphenyldiisocyanat-Fraktionen (MMDI-Fraktionen), geleitet. Die Vorrichtung zur Reinigung und ggf. auch Auftrennung der monomeren Isocyanatfraktionen (2400) muss nicht wie in den Abbildungen gezeigt aus lediglich einer einzigen Destillationskolonne bestehen. Vielmehr kann 2400 in besonderen Ausführungsformen für mehrere, bevorzugt 4 bis 10 Destillationskolonnen stehen, die in Reihe und optional zum Teil auch parallel geschaltet sind, wobei diese Destillationskolonnen jeweils, wie für die Einrichtung 2410 beschrieben, ausgestattet sind. In diesen besonderen Ausführungsformen fallen dann mehrere Fraktionen (mit unterschiedlicher Isomerenzusammensetzung) an gereinigtem Isocyanat-Strom 140 an (etwa im Falle der beispielhaft genannten Herstellung von Di- und Polyisocyanaten der Diphenylmethanreihe eine Fraktion 140-1 aus überwiegend 4,4'-Diphenylmethandiisocyanat und eine Fraktion 140-2 aus einem Gemisch aus im Wesentlichen 2,4'- und 4,4'-Diphenylmethandiisocyanat). Gereingtes Isoycanat 140 (oder 140-1, 140-2, ...) wird in den angeschlossenen Produkttank 2440 geleitet (im Falle mehrerer IsocyanatFraktionen 140-1, 140-2, ... in mehrere Produkttanks 2440-1, 2440-2, ...). Mögliche Ausgestaltungen der Vorrichtung 2400 im Falle der Herstellung von Di- und Polyisocyanaten der Diphenylmethanreihe sind in DE 31 450 10 A1, FIG. 2 und FIG. 3 beschrieben. Die Beschränkung auf eine einzige Destillationskolonne 2400, einen einzigen Brüdenstrom 150 und einen einzigen Produkttank 2440 in den Abbildungen ist also lediglich der zeichnerischen Vereinfachung geschuldet. Die nicht zu kondensierenden Brüden (150) der Destillationsvorrichtung 2400 werden in das Abgassystem geleitet.

Es ist auch möglich, die **Destillationsvorrichtung (2400)** als Trennwandkolonne auszuführen, wobei dann verschiedene monomere Isocyanatfraktionen (im Falle der Herstellung von Di- und Polyiscoyanaten der Diphenylmethanreihe Fraktionen mit den Isomeren des MMDI in unterschiedlicher Zusammensetzung) an verschiedenen Stellen der Kolonne gewonnen werden. Es können auch mehrere Trennwandkolonnen in Serie geschaltet werden, ggf. auch kombiniert mit Kolonnen ohne Trennwand, wie in EP 1 475 367 B1 beschrieben.

In einer anderen besonderen Ausgestaltung der Erfindung, die insbesondere für die **Herstellung von Toluylendiisocyanat (TDI)** geeignet ist, wird auf die Einrichtung (2410) verzichtet (d. h. der Strom 100 wird direkt in die Destillationsvorrichtung 2400 geleitet; vgl. die gestrichelt gezeichnete Leitung für Strom 100 in FIG. 1), und die **Destillationsvorrichtung (2400)** wird als Trennwandkolonne ausgestaltet. Gereinigtes Isocyanat (140), im Falle der Herstellung von TDI also Rein-TDI, wird der Kolonne (2400) als Seitenstrom entnommen, während im Sumpf der Kolonne ein Gemisch aus polymeren Anteilen ("Rückstand") und monomerem Isocyanat (TDI im beispielhaft genannten Fall) als Strom (143) anfällt. In dieser Ausführungsform ist also die Abtrennung polymerer Isocyanatfraktionen apparativ in die Destillationsvorrichtung 2400 integriert. Es ist möglich, aus Strom 143 monomeres Isocyanat durch Aufkonzentration zurückzugewinnen. Alternativ kann Strom 143 auch zur Rückgewinnung des korrespondierenden Amins hydrolysiert werden. Eine rein thermische Verwertung des Strom 143 durch Verbrennung ist ebenfalls möglich. Ein Strom 143 im zuvor geschilderten Sinne eines Rückstandsstroms gibt es in der weiter oben beschriebenen Ausführungsform umfassend die Polymerabtrennung 2410 nicht (selbstverständlich können auch in dieser Ausführungsform umfassend die Polymerabtrennung 2410 in der Vorrichtung 2400 Sumpfströme anfallen; diese umfassen dann aber hochsiedende Monomerfraktionen 140 und/oder hochsiedende Verunreinigungen - die polymeren Isocyanatfraktionen werden in 2410 als Strom 141 abgetrennt).

Es ist bevorzugt, die erfindungsgemäße Anlage (insbesondere bevorzugt durch prozessleittechnische Einrichtungen) so auszugestalten und so zu betreiben, dass bei Außerbetriebnahme eines oder mehrerer von der Destillationsvorrichtung (V) verschiedener Anlagenteile weiter das Vakuum aufrecht erhalten wird und bevorzugt 20 Minuten nach Abstellung der Aminzufuhr in die Reaktionsstrecke I), besonders bevorzugt 40 Minuten (je nach Produktionslast) nach Abstellung der Aminzufuhr in die Reaktionsstrecke I), die Dampfzufuhr zum Verdampfer der Destillationskolonne abgestellt wird, um bis zu einer Betriebsunterbrechung von 12 Stunden den Ablauf aus (III) aufnehmen zu können. Dabei wird der sich abkühlende Ablauf der Destillationskolonne statt zum Endprodukttank auf Kreislauffahrweise umgeschaltet und über den Roh-Isocyanat-Zwischenlagertank aus (III), von dort in die Einlaufleitung zwischen Destillationsvorrichtung (II) und der Destillationsvorrichtung (III), über die Destillationsvorrichtung (III) zurück nach (V) im Kreis gefahren.

Vorzugsweise umfasst die **Absorptionsvorrichtung 2500 (VI),** in der die Brüden aus der Reaktionsstrecke I) (70), dem Entphosgenierer II) (90) und der Lösungsmittelreinigung IV) (130) gesammelt werden, und in dem die vereinigten Brüden in eine flüssige, organische Phase (160), umfassend Phosgen und Lösungsmittel und eine gasförmige Phase (170), umfassend Chlorwasserstoff und Spuren an Phosgen sowie Lösungsmittel, aufgetrennt werden, mehrere hintereinandergeschaltete Wärmeaustauscher, die mit gekühltem Lösungsmittel als Kühlmedium betrieben werden, in denen das gasförmige Phosgen und Lösungsmittel der gesammelten drei Brüdenströme gekühlt und kondensiert werden, und eine mit Lösungsmittel betriebene Waschkolonne, in der aus dem verbliebenen gasförmigen Chlorwasserstoff Spuren an Phosgen und Lösungsmittel ausgewaschen werden.

Es ist bevorzugt, die erfindungsgemäße Anlage so auszugestalten (insbesondere bevorzugt durch prozessleittechnische Einrichtungen) und so zu betreiben, dass bei einer Außerbetriebnahme eines oder mehrerer von der Absorptionsvorrichtung (VI) verschiedener Anlagenteile die Vorrichtung VI, wie oben bei der Reaktionsstrecke in Schritt I) beschrieben, in Kreislauffahrweise weiterbetrieben wird, wobei die Chlorwasserstoffkonzentration der Brüden aus den Schritten I), II) und IV) stetig abnimmt, da der Aminstrom erfindungsgemäß abgestellt ist und nur noch in der Reaktionsstrecke I) verbliebenes Amin zu Roh-Isocyanat und Chlorwasserstoff abreagiert. Die Kühlung der Wärmeaustauscher wird Aufrecht erhalten. Die Lösungsmittelaufgabe zur Waschkolonne der Absorptionsvorrichtung 2500 wird stetig in der Menge reduziert und nach 12 Stunden mit dem Abstellen der kompletten Anlage ebenfalls abgestellt.

Des Weiteren ist es bevorzugt, dass die **Absorptionsvorrichtung 2600 (VII)** der erfindungsgemäßen Anlage, in der der gasförmige Chlorwasserstoffstrom (170), der noch Spuren an Phosgen und Lösungsmittel enthält, mit HCl-saurem Wasser (nicht gezeigt) und frischem, kaltem Kondensat aus Dampf (180) in einem exothermen Vorgang als Salzsäure absorbiert wird, eine Absorptionskolonne 2600, einen Anschluss zur Vorrichtung zur Abgasreinigung (VIII) und einen Sumpfausspeisekühler umfasst. Die Spuren an Lösungsmittel und Phosgen werden über Kopf der Absorptionskolonne (Strom 200) in die Vorrichtung zur Abgasreinigung (VIII) geführt. Das HCl-saure Wasser wird aus der Vorrichtung zur Abgasreingung (VIII), die einen mit Aktivkohle betriebenen Turm umfasst, der im Unterdruck mit Frischwasser berieselt wird, an dem auch die Absorptionsvorrichtung VII) angeschlossen ist, entnommen. Zur Zwischenlagerung der Salzsäure hängt an der Absorptionskolonne 2600 ein Salzsäuretank (2610), der im regulären Betrieb der Anlage genutzt wird. Zusätzlich hängt ein weiterer Tank (2620), enthaltend schwache Salzsäure, an der Absorptionskolonne 2600, der das HCl-saure Wasser aus der Vorrichtung zur Abgasreinigung 3000 (VIII) aufnimmt. Nach der Abstellung des Aminstroms (m₂ = 0) klingt die Entstehung von Chlorwasserstoff ab und damit die Produktion von Salzsäure.

Es ist bevorzugt, die erfindungsgemäße Anlage (insbesondere bevorzugt durch prozessleittechnische Einrichtungen) so auszugestalten und so zu betreiben, dass bei Außerbetriebnahme eines oder mehrerer von der Absorptionsvorrichtung VII verschiedener Anlagenteile der Absorptionsapparat während der gesamten Betriebsunterbrechung in Betrieb bleibt, um mit dem Prozessabgasvernichtungssystem, das ebenfalls während der gesamten Betriebsunterbrechung in Betrieb bleibt, über den Ablauf des Absorptionsapparates auf den schwache Salzsäure enthaltenden Behälter und über dessen Ablauf zurück zum Absorptionsapparat in Kreislauffahrweise gestellt zu werden.

Bevorzugt umfasst die optionale **Vorrichtung zur Abgasreinigung 3000 (VIII)** ("Prozessabgasvernichtung") der erfindungsgemäßen Anlage, in der die Phosgen-haltigen Prozessabgase der Vakuumsysteme aus mindestens Schritt VII), bevorzugt aus allen Anlagenteilen mit Vakuumsystemen sowie die inerten Gase aus den Druckhaltungen und den Abgasen der Tank-Beschleierungen vernichtet werden, einen mit Aktivkohle im Unterdruck betriebenen Apparat mit einer über einen Ventilator betriebenen Entlüftung zu einer thermischen Abluftreinigung ("TAR").

Es ist bevorzugt, die erfindungsgemäße Anlage (insbesondere bevorzugt durch prozessleittechnische Einrichtungen) so auszugestalten und so zu betreiben, dass bei Außerbetriebnahme eines oder mehrerer von der Prozessabgasvernichtung (VIII) verschiedener Anlagenteile das Prozessabgasvemichtungssystem unbedingt in Betrieb bleiben muss, um während der gesamten Betriebsunterbrechung die inerten Gase aus den Druckhaltungen und den Abgasen der Tank-Beschleierungen aufzunehmen und aufzuarbeiten, wobei die Flüssigphase aus der Chlorwasserstoffabsorptionskolonne 2600 (VII) statt zum Salzsäuretank zum Behälter für die schwache Salzsäure umgelegt wird, wobei die schwache Salzsäure über den mit Aktivkohle betriebenen Apparat des Prozessabgasvernichtungssystems, über die Chlorwasserstoffabsorptionskolonne und zurück zu dem Behälter mit der schwachen Salzsäure im Kreis gefahren wird. Eine wichtige Grundlage des Prozesses ist, dass der mit Aktivkohle betriebene Turm des Prozessabgasvernichtungssystems beim Betreiben der Isocyanat-Anlage immer als Erstes in Betrieb genommen wird und als Letztes abgestellt wird.

Bevorzugt umfasst die optionale **Phosgenerzeugungsvorrichtung 4000 (IX)** der erfindungsgemäßen Anlage, in der Frischphosgen aus Chlor (220) und Kohlenmonoxid (230) als Einsatzstoffe hergestellt wird, einen Mischapparat für die Einsatzstoffe, einen mit Aktivkohle als Katalysator bestückten Apparat mit Dosierleitungen für das Einsatzstoff-Gemisch, einem Kühlkreislaufsystem und einem Phosgenverflüssiger (nicht gezeigt) mit Austrittsleitung zum Phosgenlösungstank (1030), in dem das Phosgen/Lösungsmittel-Gemisch für die Phosgenierung in der Reaktionsstrecke I) hergestellt wird. Das Abgas des Phosgenverflüssigers ist bevorzugt mit der TAR verbunden.

Es ist bevorzugt, die erfindungsgemäße Anlage (insbesondere bevorzugt durch prozessleittechnische Einrichtungen) so auszugestalten und so zu betreiben, dass bei Außerbetriebnahme eines oder mehrerer von dem Phosgengenerator IX) verschiedener Anlagenteile dieser mit der Abstellung der Aminzufuhr zur Reaktionsstrecke I) ebenfalls abgestellt wird.

Diese bevorzugten Ausführungsformen stehen natürlich nur exemplarisch für eine Vielzahl möglicher Kreislauffahrweisen, deren genaue Ausgestaltung von den konkreten Gegebenheiten einer Produktionsanlage abhängt, aber im Sinne der vorliegenden Erfindung einfach an diese konkreten Gegebenheiten angepasst werden kann. Ein gemeinsames Merkmal aller denkbaren Kreislauffahrweisen ist jedoch, dass, nach Abreagieren der in Apparaten und Rohrleitungen vorhandenen Restmengen an Amin, kein Produkt (Isocyanat 1) die Anlage verlässt, sofern es sich um eine einstrassige Isocyanat-Produktionsanlage handelt.

Sollten zwei oder mehr Isocyanat-Reaktorlinien parallel betrieben werden, dann könnte, muss aber nicht, Produkt die Anlage verlassen, wenn z. B. die Anlage mit Teillast gefahren wird.

In der erfindungsgemäßen Anlage und den erfindungsgemäßen Verfahren kann dabei jedes Anlagenteil beispielsweise per Hand auf Kreislauffahrweise eingestellt werden. In einer bevorzugten Ausführungsform erfolgt das Umstellen auf Kreislauffahrweise, das Anfahren sowie die Überwachung aller Schritte über eine zentrale Steuerungsanlage, die insbesondere bevorzugt prozessleittechnische Einrichtungen umfasst.

Ein weiterer Gegenstand der vorliegenden Erfindung ist ein **Verfahren zum Betrieb einer Anlage zur Herstellung von Isocyanaten in der Flüssigphase**, umfassend die folgenden Anlagenteile:
I) eine **Reaktionsstrecke (1000)** umfassend
   I.1) eine Einrichtung (1020) zur Bereitstellung eines Amins (2), bevorzugt in Form einer Aminlösung (20) in einem Lösungsmittel (4),
   I.2) eine Einrichtung (1030) zur Bereitstellung von Phosgen (3), bevorzugt in Form einer Phosgenlösung (30) in einem Lösungsmittel (4),
   I.3) eine Einrichtung (1040) zur Bereitstellung eines Lösungsmittels (4),
   I.4) eine Mischeinrichtung (1100) zur Vermischung von Amin (2) oder Aminlösung (20) mit Phosgen (3) oder Phosgenlösung (30) und optional weiterem Lösungsmittel (4) und
   I.5) einen strömungstechnisch nach der Mischeinrichtung angeordneten Reaktionsraum (1200) zur Durchfühung der Phosgenierung, dem optional eine Abscheidereinrichtung (1210) nachgeschaltet ist, wobei der Reaktionsraum oder die Abscheidervorrichtung mit Abfuhrleitungen für einen flüssigen Strom (60) und einen gasförmigen Strom (70) versehen sind;
II) eine **Destillationsvorrichtung (2100 - "Entphosgenierkolonne")** zur Auftrennung des flüssigen Stroms (60) in einen flüssigen Strom (80) und einen gasförmigen Strom (90);
III) eine **Destillationsvorrichtung (2200 - "Lösungsmittelkolonne")** zur Trennung des flüssigen Stroms (80) in einen gasförmigen Strom (110) und einen flüssigen Strom (100);
IV) eine **Destillationsvorrichtung (2300 - "Lösungsmittelstripper")** zur Trennung des gasförmigen Stroms (110), bevorzugt nach dessen Verflüssigung in einem **Kondensator (2310),** in einen flüssigen Strom (120) und einen gasförmigen Strom (130);
V) eine **Destillationsvorrichtung (2400)** zur Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, optional umfassend eine vorgeschaltete **Einrichtung zur Polymerabtrennung (2410 - "Polymerabtrennung", PMA)** zur Abtrennung von polymeren Isocyanatfraktionen (141);
VI) eine **Vorrichtung zur Absorption (2500 - "Phosgenabsorber")** der gasförmigen Ströme (70), (90) und (130) in Lösungsmittel unter Erhalt eines flüssigen Stroms (160) und eines gasförmigen Stroms (170);
VII) optional (und bevorzugt) eine **Vorrichtung zur Absorption (2600 - "HCl-Absorptionskolonne")** des gasförmigen Stroms (170) in Wasser;
VIII) optional (und bevorzugt) eine **Vorrichtung zur Aufarbeitung von Abgasströmen (3000),** welche zur Aufarbeitung von Abgasströmen mindestens aus VII), bevorzugt zur Aufarbeitung von Abgasströmen aus allen vorhandenen Anlagenteilen, ausgestaltet ist;
IX) optional (und bevorzugt) eine **Vorrichtung (4000) zur Herstellung von Phosgen,** welche mit der Einrichtung (1030) verbunden ist;
wobei
bei einer Produktionsunterbrechung die folgenden Schritte durchlaufen werden:
(i) Unterbrechen der Zufuhr von Amin (2) in die Mischeinrichtung 1100;
(ii) Sofern vorhanden, Abschalten der Vorrichtung (4000) zur Herstellung von Phosgen;
(iii) Verringerung der Phosgenzufuhr in die Mischeinrichtung (1100) auf bevorzugt einen Wert im Bereich von 10 % bis 50 % der Phosgenzufuhr im Regelbetrieb;
(iv) Verringerung der Lösungsmittelzufuhr in die Mischeinrichtung (1100) auf bevorzugt einen Wert im Bereich von 10 % bis 50 % der Lösungsmittelzufuhr im Regelbetrieb, bevorzugt durch Verringerung der Lösungsmittelzufuhr in die Einrichtungen 1020 und 1030;
(v) Fahren mindestens eines Anlagenteiles so, dass der Ausgangsstrom des jeweiligen Anlagenteils
   (v)(i) in den jeweiligen Anlagenteil oder
   (v)(ii) in einen strömungstechnisch davor oder dahinter liegenden Anlagenteil geführt und von dort, optional über weitere nicht außer Betrieb genommene Anlagenteile, in den ausgehenden Anlagenteil
   zurückgeführt wird (sog. "Kreislauffahrweise").

Ist die Produktionsunterbrechung die Folge der anstehenden Außerbetriebnahme eines Anlagenteils oder mehrerer Anlagenteile I) bis IX), insofern diese vorhanden sind, so werden im Anschluss an Schritt (v) die folgenden Schritte durchlaufen:
(vi) Außerbetriebnahme mindestens eines Anlagenteils;
(vii) Erforderlichenfalls Öffnen des in Schritt (vi) außer Betrieb genommenen mindestens einen Anlagenteils;
(viii) Durchführen einer Instandhaltungs-, Reinigungs- und/oder Reparaturmaßnahme in dem mindestens einen in Schritt (vi) außer Betrieb genommenen Anlagenteil;
(ix) Erforderlichenfalls Schließen und optional Inertisieren des mindestens einen in Schritt (vi) außer Betrieb genommenen Anlagenteils.

Dabei ist die Anlage des Verfahrens vorzugsweise die Anlage gemäß der vorliegenden Erfindung. Mit diesem erfindungsgemäßen Verfahren kann vorteilhafterweise die Anlage bei den oben beschriebenen Unterbrechungen (Außerbetriebnahme einzelner Anlagenteile) in Kreislauffahrweise betrieben und damit die erfindungsgemäßen Vorteile und Effekte erzielt werden. Ganz besonders vorteilhaft und daher bevorzugt werden in Schritt (v) möglichst alle Anlagenteile, die sich auf Kreislauffahrweise schalten lassen und nicht in irgendeiner Art vom Produktionsstillstand betroffen sind, in Kreislauffahrweise gebracht, wobei jedoch die Vorrichtung zur Aufarbeitung von Abgasströmen 3000 weiterbetrieben wird und die Vorrichtung 4000 zur Phosgenerzeugung von der Kreislauffahrweise ausgenommen wird.

In einer bevorzugten Ausführungsform umfasst das Verfahren bei Außerbetriebnahme mindestens eines Anlagenteils die weiteren Schritte:
(x) Anfahren des mindestens einen in Schritt (vi) außer Betrieb genommenen Anlagenteils,
(xi) Sofern vorhanden, Anfahren der Vorrichtung 4000 zur Phosgenerzeugung,
(xii) Starten, bevorzugt in dieser Reihenfolge, der Zufuhr von Lösungsmittel, Phosgen und Amin in der Reaktionsstrecke 1000 (I).

In einer anderen Ausführungsform, die dann vorteilhaft ist, wenn lediglich Rohstoffmangel herrscht, aber keine Wartungsarbeiten anstehen, werden in Schritt (v) alle Anlagenteile, bevorzugt mit Ausnahme des Phosgengenerators 4000, welcher bevorzugt, wie weiter oben beschrieben, abgestellt wird, so betrieben, dass der Ausgangstrom des jeweiligen Anlagenteils als Eingangsstrom des jeweiligen Anlagenteils verwendet wird, oder dass der Ausgangstrom des jeweiligen Anlagenteils als Eingangsstrom eines davor oder dahinter liegenden Anlagenteils verwendet werden kann und in Kreislauffahrweise der Ausgangsstrom dieses Anlagenteils als Eingangsstrom des ausgehenden Anlagenteiles zurückgefahren wird, wobei die Kreislauffahrweise auch über mehrere Anlagenteile erfolgen kann und ein Anlagenteil auch in mehr als einer Kreislauffahrweise eingebunden sein kann. In diesem Fall schließen sich an Schritt (v) die folgenden Schritte an:
xiii) Warten auf Einsatz- oder Hilfsstoffe, und, sobald diese eingetroffen sind,
(xiv) Starten, bevorzugt in dieser Reihenfolge, der Zufuhr von Lösungsmittel, Phosgen und Amin in der Reaktionsstrecke 1000 (I).

Im Folgenden sollen dazu beispielhaft das Einstellen der gesamten Anlage (mit Ausnahme des Prozessabgassystems VIII, welches in Betrieb bleibt, und der Phosgenerzeugung IX, welche abgestellt wird) auf Kreislauffahrweise und ebenso beispielhaft das Wiederanfahren der Anlage aus der Kreislauffahrweise heraus in den Normalbetrieb beschrieben werden. Davon abweichend ist es selbstverständlich auch möglich, nur bestimmte der Anlagenteile I bis VII in Kreislauffahrweise zu betreiben und die übrigen bspw. für Wartungsarbeiten außer Betrieb zu nehmen.

Im ersten Schritt wird die Zufuhr von Amin in die Reaktionsstrecke 1000 gestoppt.

Im zweiten Schritt wird, sofern vorhanden, die Phosgenherstellung abgestellt, und die restliche Anlage wird sukkzesive in Kreislauffahrweise gestellt. Wird Phosgen nicht in der Anlage selbst hergestellt, sondern von einer externen Quelle bezogen, wird die Zufuhr aus dieser Quelle unterbrochen.

Im dritten Schritt werden für bis zu 3 Stunden, aber für mindestens 1 Stunde, das Phosgen/Lösungsmittel-Gemisch 30 und das Lösungsmittel 4 des Amin/Lösungsmittel-Gemisches 20 zur restlichen Umsetzung des noch vorhandenen Amins und zur Verdünnung der Reaktionslösung in der Reaktionsstrecke weiter in die Mischeinrichtung 1100 und von dort in den Reaktionsraum 1200 und die Entphosgenierkolonne 2100 gefahren.

Die Lösungsmittelzufuhr auf dem Phosgenweg und dem Aminweg läuft weiter, und die Reaktionsstrecke (I) wird über den Phosgenlösungstank (1030) mit Phosgen-haltigem Lösungsmittel aus dem Phosgenabsorber (VI) beschickt und so eine Kreislauffahrweise I) → VI) → I) eingestellt (vgl. **FIG. 2**).

Gleichzeitig wird die Reaktionsstrecke (I) über den Phosgenlösungstank 1030 mit Phosgen-haltigem Lösungsmittel aus dem Phosgenabsorber (VI) *und der Entphosgenierung (II)* beschickt und so eine Kreislauffahrweise I) → II) → VI) → I) eingestellt (vgl. **FIG. 3**).

Gleichzeitig wird die Reaktionsstrecke (I) mit Phosgen-haltigem Lösungsmittel über die Entphosgenierung (II), die Lösungsmitteldestillation (III), die Lösungsmittelreinigung (IV), den Lösungsmitteltank (1040) und die Einrichtung 1020 (in welche zu diesem Zeitpunkt kein Amin 2 mehr eingespeist wird) zurück zum Mischer (1100) der Reaktionsstrecke (vgl. **FIG. 4**) oder zur Anbindung des Mischers an den Reaktionsraum 1200 (nicht gezeigt) in Kreislauffahrweise gestellt (Kreislauffahrweise I) → II) → III) → IV) → I)).

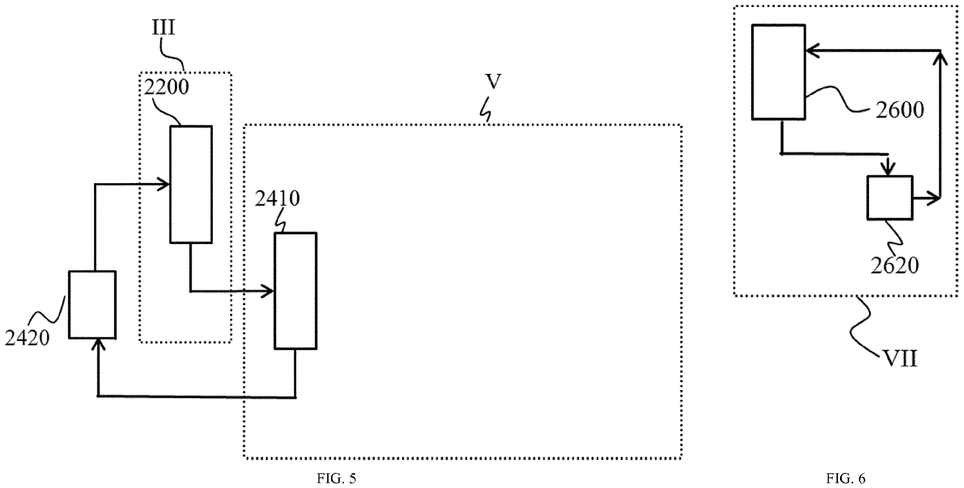

Gleichzeitig wird das verdünnte Roh-Isocyanat aus der Lösungsmitteldestillation (III) über die Destillation des Roh-Isocyanates (V) (ohne Beheizung) durch die erste Destillationskolonne 2410 in den Roh-Polymer-Isocyanat-Tank (2420) zurück auf die Einlaufleitung der Lösungsmitteldestillation (III) im Kreis gefahren (Kreislauffahrweise III) → V) → III); vgl. **FIG. 5**).

Gleichzeitig lässt man den Abgasweg vom Phosgenabsorber (VI) zur Chlorwasserstoffabsorption (VII) und von dieser zur Prozessabgasvernichtung (VIII) offen und stellt die Chlorwasserstoffabsorption mit schwacher Salzsäure über den Schwachsäure-Tank (2620) auf Kreislauffahrweise (Kreislauf VII) → VII); vgl. **FIG. 6**).

Nach Durchführung dieser Schritte befinden sich sämtliche Anlagenteile außer VIII (Prozessabgasvernichtung) und IX (Phosgenerzeugung) in Kreislauffahrweise. Nun ist es möglich, bspw. Wartungsarbeiten am Phosgengenerator durchzuführen oder auch einfach bspw. durch Rohstoffmangel bedingte Wartezeiten zu überbrücken, ohne dazu die gesamte Anlage vollständig außer Betrieb nehmen zu müssen. Im Falle von Wartungsarbeiten wird der Phosgengenerator (oder, falls ein anderes Anlagenteil betroffen ist und deshalb nur Teile der Anlagenteile I bis VIII in Kreislauffahrweise betrieben werden, dieses besagte Anlagenteil entleert, gereinigt und gegebenenfalls für die anstehende Maßnahme geöffnet. Dann wird die Instandhaltungsmaßnahme durchgeführt und der Phosgengenerator (oder das andere Anlagenteil) wieder geschlossen, gegebenenfalls inertisiert und mit Hilfsstoffen und Einsatzmaterialien befüllt und zur Wiederinbetriebnahme vorbereitet.

Während der Kreislauffahrweise wird das Vakuumsystem von Lösungsmitteldestillation und Lösungsmittelstripper Aufrecht erhalten. Reaktionsstrecke, Entphosgenierer, Lösungsmitteldestillation und Lösungsmittelreinigung werden weiter beheizt. Das Prozessabgassystem bleibt in Betrieb.

### Das Wiederanfahren der Anlage aus der Kreislauffahrweise heraus kann beispielsweise wie folgt ablaufen:

Die Behälter und Apparate sind mit verdünnter Produktionslösung beladen. Das Vakuumsystem von Lösungsmitteldestillation (III) und Lösungsmittelstripper (IV) läuft. Die Reaktionsstrecke (I), der Entphosgenierer (II), die Lösungsmitteldestillation (III) und der Lösungsmittelstripper (IV) sind beheizt, und das Prozessabgassystem läuft. Um die Anlage wieder anzufahren, wird **zuerst die Phosgenherstellung (IX) angefahren.**

Sobald Phosgen in genügender Menge zur Verfügung steht, wird **als zweites die Phosgenlösung im Phosgen/Lösungsmittel-Tank (1030) aufkonzentriert.**

Dann wird **als drittes der Amin-Weg geöffnet.** Die Phosgenierungsreaktion startet unmittelbar unter Bildung von Roh-Isocyanat und Chlorwasserstoff. Nun ist das Betriebssegment der Reaktionsstrecke angestellt und die Brüden (70) des Reaktionsraums 1200 (oder der nachgeschalteten Abscheidervorrichtung 1210), enthaltend Phosgen, Chlorwasserstoff und Lösungsmittel, werden zum Phosgenabsorber (IV) geleitet, während die flüssige Phase (80), enthaltend Roh-Isocyanat, Lösungsmittel und Phosgen, zum Entphosgenierer (III) geleitet wird.

Sobald die Brüden aus (I) im Phosgenabsorber (VI), umfassend einen Trennapparat, drei Brüdenleitungen für die Brüden (70, 90 und 130), eine Ausspeiseleitung für die flüssige Phase (160) und eine ausgehende Leitung (170) zur Chlorwasserstoffabsorption, ankommen, werden die vereinigten frischen Brüden aus der Reaktionsstrecke (I), dem Entphosgenierer (II) und dem Lösungsmittelstripper (IV) aufgetrennt in eine flüssige, organische Phase (160), umfassend Phosgen und Lösungsmittel, die in den Phosgenlösungstank (1030) aus Schritt I) läuft, und eine gasförmige Phase (170), umfassend Chlorwasserstoff mit Spuren an Lösungsmittel und Phosgen, die in die Chlorwasserstoffabsorption (VII) geführt wird. Die Gasphase der Chlorwasserstoffabsorption (200) wird in die Prozessabgasvernichtung (VIII) geleitet.

Sobald die gasförmige Phase aus (VI) in der Chlorwasserstoffabsorption (VII), umfassend einen Absorptionsapparat, einen Wärmeaustauscher zum Kühlen der flüssigen Phase, einen Wärmeaustauscher zum Kondensieren der Gasphase, eine Gaszuführleitung, zwei Dosierleitung für die wässrigen Phasen, einen Salzsäurelagertank und einen Lagertank für schwache Salzsäure mit entsprechenden Leitungen, eintrifft, wird das Chlorwasserstoffgas der gasförmigen Phase aus (VI) mit saurem Wasser aus Schritt (VII) und zusätzlichem Kondensatwasser (180) in einer exothermen Reaktion als Salzsäure absorbiert und in den Salzsäuretank (2610) gefahren. Das Abgas 200 geht zur Prozessabgasvernichtung (VIII).

Sobald die Reaktionslösung (60) aus (I) in die Entphosgenierung (II), umfassend eine Dosierleitung für Roh-Isocyanat, einen Trennapparat und einen Wärmeaustauscher, gepumpt wird, wird die Reaktionslösung in eine gasförmige Phase (90), enthaltend Phosgen, Lösungsmittel und Chlorwasserstoff, die in die Phosgenabsorption (VI) geführt wird, und eine flüssige Phase (80), enthaltend Roh-Isocyanat, Lösungsmittel und Spuren an Phosgen, die in die Lösungsmitteldestillation (III) gepumpt wird, aufgetrennt.

Sobald das Roh-Isocyanat 80 aus (II) in die Lösungsmitteldestillation (III), umfassend eine Destillationskolonne mit Vakuumsystem, eine Pumpenvorlage für das Gemisch aus Roh-Isocyanat und Lösungsmittel, einem Wärmeaustauscher für dieses Gemisch, einen Trennapparat zur Trennung von Lösungsmittel und Roh-Isocyanat, einem Wärmeaustauscher zur Kondensation der Lösungsmittelbrüden, gepumpt wird, wird das Roh-Isocyanat (80) im ersten Schritt vom größten Teil an Lösungsmittel und Spuren von Phosgen befreit, wobei diese in IV) kondensiert (2310) und zur Lösungsmittelreinigung (2300) geschickt werden, und im zweiten Schritt das so vorgereinigte Roh-Isocyanat bis auf Spuren von restlichem Lösungsmittel und Phosgen befreit und als Strom 100 in die Rein-Destillation (V) gepumpt. Schritt (III) wurde hier in einer zweistufigen Ausgestaltung geschildert; in den Zeichnungen ist der Einfachheit halber Schritt (III) als einstufige Destillation dargestellt, die grundsätzlich bei entsprechender Auslegung auch möglich ist.

Sobald das kondensierte Lösungsmittel mit Spuren von Phosgen und Roh-Isocyanat aus (III) in die Lösungsmittelreinigung (IV), umfassend eine Pumpenvorlage, eine Trennkolonne mit Vakuumsystem, einen Wärmeaustauscher zur Vorerwärmung, einen Wärmeaustauscher zur Brüdenerzeugung, einen Wärmeaustauscher zur Kondensation der Brüden und einen Wärmeaustauscher zur Kühlung des Lösungsmittels, gepumpt wird, wird das Lösungsmittel mit Spuren von Phosgen und Roh-Isocyanat von Phosgen befreit. Die so gebildeten Brüden (130), enthaltend Phosgen und Lösungsmittel, werden in die Phosgenabsorption (VI) geführt, und das so gereinigte Lösungsmittel (120) wird anschließend in den Lösungsmitteltank (1040) gepumpt, wo es zur Verfügung steht für die Versorgung des Aminweges in der Reaktionsstrecke (I).

Sobald das bis auf Spuren von restlichem Lösungsmittel und Phosgen befreite Roh-Isocyanat aus (III) in die Rein-Destillation (V), umfassend eine Destillationskolonne 2400 mit Vakuumsystem und Trennapparat, einen Wärmeaustauscher zum Verdampfen, einer Pumpenvorlage und einen Wärmeaustauscher zum Kondensieren, und optional eine vorgeschaltete Destillationskolonne 2410 mit Vakuumsystem und Trennapparat, einen Wärmetauscher zum Verdampfen, einer Pumpenvorlage und einen Wärmeaustauscher zum Kondensieren, für eine optionale Abtrennung polymerer Isocyanatfraktionen, gepumpt wird, wird das Roh-Isocyanat 100 von Spuren an Lösungsmittel und z. B. im Fall von MDI von Phenylisocyanat und anderen Nebenprodukten, z. B. im Fall von TDI von unerwünschten Oligomeren und Polymeren, befreit, und in die gewünschten Isomeren getrennt. Auf diese Weise wird das gewünschte Isocyanat 1 in der gewünschten Reinheit und Homologen- bzw. Isomerenverteilung erhalten (Ströme 140 und 141).

Das Prozessabgasvemichtungssystem (VIII), umfassend einen mit Aktivkohle gefüllten und im Unterdruck betriebenen Apparat mit Ventilator zu einer angeschlossenen TAR, einer Pumpenvorlage mit Pumpe und Dosierleitungen und einer Gaszuführungsleitung, war auch während der Kreislauffahrweise in Betrieb und muss daher nicht erneut angefahren werden.

Die komplette Isocyanat-Produktionsanlage 10000 läuft jetzt bevorzugt mit reduzierter Last (Anfahrlast) und kann nun auf die gewünschte Sollproduktion hochgefahren werden. Es ist hier besonders bevorzugt die Produktionsanlage mit reduzierter Last anzufahren, da ansonsten die benötigten Temperaturprofile für die Phosgenierung, die Chlorwasserstoff-Aufarbeitung, die Phosgenabsorption und die verschiedenen Destillationseinrichtungen wegen z. B. unzulänglichen Temperaturprofilen nicht schnell genug zur Verfügung stehen könnten, was zu unvollständigen Reaktionen, vermehrter Nebenproduktbildung und mangelhafter Aufarbeitung des Produktes führen würde.

Durch das erfindungsgemäße Verfahren ergeben sich die folgenden Vorteile:
i) Erhöhung der Produktivität, weil sich die Verfügbarkeit der Anlage erhöht, da sich der Zeitbedarf für das Abfahren und Wiederanfahren der Anlage für den Produktionsstillstand stark minimiert.
ii) Investkosten in eine größere Anlagenkapazität entfallen.
iii) Investkosten in einen größeren Endprodukttank zur Abpufferung von längeren Stillstandszeiten entfallen.
iv) Vermeidung von überflüssigen Abfallprodukten, die bei unvollständiger Reaktion während des Abfahrvorganges entstehen.
v) Die Reinigung des Lösungsmittels, z. B. die Entfernung von Phosgen und Spuren an Isocyanaten, begünstigt die Produktqualität und ermöglicht ein reibungsloses Anfahren der Reaktion bei der Wiederaufnahme des Verfahrens.
vi) In vielen Fällen kommt es zur Einsparung von Energie, weil die zum Wiederanfahren benötigten Vorbereitungen für die ausgeschalteten Anlagenteile wie das Aufwärmen der Einsatz- und Hilfsstoffe oder das Aufwärmen des Equipments etc. entfallen.
vii) In vielen Fällen kommt es zur Einsparung von Hilfsstoffen wie Kondensat und Stickstoff.
viii) Die Reparaturanfälligkeit von Pumpen oder Kompressoren wird vermindert, da, wenn diese bei einem Stillstand abgestellt werden, bei jedem Wiederanfahren deren Lager oder Abdichtungen leiden. Somit werden Folgereparaturen vermieden, was sich wiederum positiv auf die Produktivität der Anlage und die Instandhaltungskosten auswirkt.

Der Erfolg der erfindungsgemäßen Vorgehensweise ist für den Fachmann überraschend, weil er, um grundsätzlich Energie einzusparen und um sich auf die anstehenden Instandhaltungsmaßnahmen im Produktionsstillstand konzentrieren zu können, viel eher dazu tendieren würde, die Gesamtanlage abzustellen, zumal für das erfindungsgemäße Verfahren bzw. für die erfindungsgemäße Anlage Zusatzinvestitionen in rückführende Rohrleitungen samt Pumpen, Umbauten an den Apparaten sowie zusätzliche Prozessleittechnik in Kauf genommen werden.Im Folgenden soll die vorliegende Erfindung durch weitere Beispiele verdeutlicht werden.

### Beispiele

### Allgemeine Bedingungen für die Herstellung eines Gemisches aus Methylendiphenyldiisocyanat und Polymethylenpolyphenylenpolyisocyanat (nachfolgend summarisch MDI) im Regelbetrieb - vgl. auch FIG. 1

4,3 t/h eines Gemisches aus Methylendiphenyldiamin und Polymethylenpolyphenylenpolyamin (nachfolgend summarisch MDA; 2) einer Temperatur von 110 °C werden mit 11 t/h Monochlorbenzol (MCB; 4) einer Temperatur von 30 °C als Lösungsmittel über einen statischen Mischer **(1020)** zu einer 28%igen MDA-Lösung **(20)** vermischt. Phosgen (3) wird bereitgestellt über einen Phosgengenerator und einen Phosgenverflüssiger (IX). Im Anschluss wird das Phosgen (3) in einem Phosgenlösungstank (1030) mit Strom 160, der überwiegend aus MCB (4) besteht, auf eine 35%ige Phosgenlösung (30) eingestellt. Stündlich werden 24 Tonnen 35%ige Phosgenlösung (30) einer Temperatur von 0 °C mit 4,3 Tonnen MDA (2) in Form der 28%igen MDA-Lösung (20) einer Temperatur von 45 °C in einer adiabaten Reaktion, wie in EP 1 873 142 B1 beschrieben, umgesetzt. Nach der Durchmischung der beiden Rohstofflösungen im Mischaggregat (1100) wird die erhaltene Reaktionslösung (50) mit einer Temperatur von 85 °C über eine Suspensionsleitung in einen beheizten Phosgenierturm (1200) gefahren. Am Kopf des Phosgenierturmes liegen ein absoluter Druck von 1,6 bar und eine Temperatur von 111 °C vor. Der bei der Reaktion entstandene Chlorwasserstoff wird zusammen mit Spuren an Phosgen und MCB als Gasstrom (70) abgeführt. Das flüssige Reaktionsgemisch (60) wird dem Phosgenierturm (1200) entnommen und der Aufarbeitungssequenz (II ff.) zugeführt. Dazu wird es zunächst als Seitenstrom in eine beheizte Entphosgenierkolonne (II) eingeleitet. Bei einer Kopftemperatur von 116 °C und einem absoluten Druck von 1,6 bar wird über Kopf Phosgen mit Spuren MCB und Chlorwasserstoff abgeführt (90). Phosgen wird in einer Phosgenabsorptionskolonne (2500, VI) absorbiert und in den Phosgenlösungstank (1030) gefahren, und Chlorwasserstoff wird in einen Chlorwasserstoffabsorber 2600 (VII) und dann zur weiteren Verwendung in einen Salzsäuretank 2610 geleitet. Nach Abtrennung von Chlorwasserstoff und überschüssigem Phosgen aus der Isocyanat enthaltenden Reaktionslösung (60) wird eine Isocyanat-Rohlösung (80) erhalten, die aus dem Sumpf der Entphosgenierkolonne 2100 ausgetragen und mit einer Temperatur von 155 °C in eine erste Destillationsstufe der Lösungsmitteldestillation (III) gefahren wird, um sie vom Lösungsmittel MCB zu befreien. Der absolute Druck am Kopf dieser Lösungsmitteldestillationskolonne beträgt 600 mbar bei einer Sumpftemperatur von 145 °C. Über Kopf wird MCB gasförmig abgezogen (110), wobei dieser MCB-Gasstrom in einem Luftkühler (2310) kondensiert wird. 2,5 t/h dieses kondensierten Lösungsmittels wird in eine Waschkolonne (nicht gezeigt in FIG. 1) gesprüht, um einen möglichen Mitriss von Isocyanat in die Vakuumleitungen zu verhindern. Das restliche kondensierte MCB von 20 t/h wird zu einem Lösungsmittelstripper 2300 gepumpt, in dem das MCB von Phosgen befreit wird, wobei die Phosgen-haltigen Brüden (130) kondensiert und in den Phosgenabsorber 2500 gepumpt werden, und das Phosgen-freie MCB aus dem Sumpf des Strippers (120) in den Lösungsmitteltank (1040) gepumpt wird. Das Roh-MDI (100) wird aus dem Sumpf der Kolonne 2200 ausgetragen und in einer zweiten Destillationskolonne (nicht gezeigt) bis auf 1 % vom restlichen MCB befreit. Der absolute Druck am Kopf dieser Lösungsmitteldestillationskolonne beträgt 70 mbar bei einer Sumpftemperatur von 150 °C. Über Kopf wird MCB gasförmig abgezogen, wobei dieser MCB-Gasstrom kondensiert wird und in den Sumpf der ersten Destillationskolonne zurückgeführt wird. Anschließend wird in einem Gegenstromverdampfer bei einem absoluten Druck von 20 mbar und einer Kopftemperatur von 170 °C das Produkt von Nebenkomponenten wie Phenylisocyanat und restlichem MCB befreit. Dabei fallen als Sumpfprodukt 5,4 t/h MDI an, das mittels weiterer Destillationsschritte (2410 / 2400) zu MDI der gewünschten Reinheit (polymere Fraktionen 141 / monomere Fraktionen 140) aufgearbeitet und anschließend zur weiteren Verwendung in die entsprechenden MDI-Produkttanks (2430 / 2440) gefahren wird.

### Allgemeine Bedingungen für die Herstellung von Phosgen im Regelbetrieb für Kaltvereiniger:

In einem Mischrohr werden kontinuierlich 810 Nm³/h Chlor und 955 Nm³/h Kohlenmonoxid bei 18 °C und einem Druck von 1,8 bar (absolut) gemischt. Es wird ein Überschuss an Kohlenmonoxid, bezogen auf Chlor, eingesetzt, sodass nach der vollständigen Umsetzung des Chlors noch 9 % Kohlenmonoxid im Phosgen verbleiben. Das Mischgas aus Chlor und Kohlenmonoxid wird in einen am Boden befindlichen Verteiler eines Rohrbündel-Phosgengenerators gefahren. In den Rohren über dem Verteiler befinden sich als Katalysator 2 Tonnen Aktivkohle (Norit RB4C). An diesem Katalysator reagiert das Mischgas in einer stark exothermen Reaktion zu Phosgen ab. Die Reaktion wird über einen Wasserkreislauf mittels Wassersiedekühlung gekühlt. Die Temperatur des Phosgens in der Austrittsleitung des Generators beträgt 55 °C und der Druck liegt bei 1,53 bar (absolut). An diesem Punkt wird die Vollständigkeit der Reaktion überwacht, indem der Restchlorgehalt und der Kohlenmonoxidgehalt kontinuierlich gemessen werden. Das so hergestellte, gasförmige, überschüssiges Kohlenmonoxid enthaltende Phosgen wird dann in einem Phosgenverflüssiger bei -17 °C kondensiert. Das Sumpfprodukt aus dem Phosgenverflüssiger läuft in einen Phosgenlösungstank (1030). Überschüssiges Kohlenmonoxid kondensiert nicht und wird über Kopf in einen nachgeschalteten baugleichen zweiten Phosgengenerator gefahren und dort mit einer entsprechenden Menge an Chlor beaufschlagt, sodass wiederum nach vollständiger Umsetzung des Chlors noch 9 % an Kohlenmonoxid im Phosgen verbleiben. Auch nach dem zweiten Phosgengenerator wird die Vollständigkeit der Reaktion überwacht, indem der Restchlorgehalt und der Kohlenmonoxidgehalt kontinuierlich gemessen werden. Das so hergestellte Phosgen wird in einem zweiten Phosgenverflüssiger bei -17 °C kondensiert. Das Sumpfprodukt aus dem zweiten Phosgenverflüssiger läuft ebenfalls in den Phosgenlösungstank. Als Kopfprodukt wird überschüssiges Kohlenmonoxid, dem auch Spuren an Phosgen anhaften, in eine Abgasschiene geleitet, dort von Phosgen befreit und anschließend in einer thermischen Abluftreinigung verbrannt. Somit kommen pro Stunde 4,2 Tonnen Phosgen im Phosgenlösungstank an.

Im Phosgenlösungstank 1030 kann das Phosgen bei Bedarf mit einem Lösungsmittel vermischt werden. Für einen Herstellungsprozess für Isocyanate wird das Phosgen im Phosgenlösungstank mit Monochlorbenzol zu einer 35%igen Phosgenlösung vermischt und bei -2 °C zur weiteren Verwendung entnommen. (In anderen großtechnischen Verfahren kann Phosgen auch als reines Flüssigphosgen zum Einsatz kommen.)

### Beispiel 1 (Vergleichsbeispiel): Kurzstillstand der MDI-Anlage mit Komplettabstellung der Anlage, Reparaturmaßnahme und Wiederanstellung der MDI-Anlage.

Der Kurzstillstand der Anlage diente dazu, einen defekten Wärmeaustauscher in der Phosgenverflüssigung in der Phosgen-Herstellung zu wechseln. Hierfür wird die MDI-Anlage komplett abgefahren, also Reaktionsstrecke, Entphosgenierer, Lösungsmittel-Destillation, Lösungsmittel-Reinigung, Rein-Destillation, Chlorwasserstoff-Absorption und Prozessabgasvernichter. Die Energiezufuhren sind während der Reparaturmaßnahme abgestellt. Nach der Maßnahme wird wieder angefahren, wobei die komplette Anlage vorab inertisiert, befüllt und aufgeheizt werden musste.

### Durchführung der Komplettabstellung der Anlage:

Die Herstellung von 5,4 t/h MDI in kontinuierlicher Fahrweise wird, wie in den allgemeinen Bedingungen beschrieben, bei Nennlast durchgeführt. Die Reaktionsstrecke wird abgefahren, indem zuerst die MDA-Zufuhr abgestellt wird. MCB aus der MDA-Lösungszufuhr und die Phosgenlösung aus dem Phosgenlösungstank laufen für eine Stunde mit der vorab eingestellten Nennlastmenge weiter. Anschließend wird die Phosgenzufuhr abgestellt, und mit zweistündigem Spülen mit Lösungsmittel wird die Reaktionsstrecke von Phosgen befreit. Die Mischeinrichtung kühlt nach Abstellung des MDA-Stromes direkt ab. Die Temperatur des Phosgenierreaktors wird mittels technischer Heizung während dieser Zeit bei 110 °C gehalten. Nach den 2 Stunden wird die Beheizung abgestellt und man lässt die Phosgenieranlage abkühlen, wobei der Phosgenierreaktor befüllt mit Lösungsmittel stehen bleibt und der Anlagendruck sich entsprechend einstellt. Die Reaktionsstrecke ist nun außer Betrieb. Die Abstellung der Phosgenierung hat insgesamt 3 Stunden gedauert.

Nach Abstellung des MDA-Stromes wird sofort die Phosgenherstellung abgestellt. Sobald restliches MDA in der Phosgenierung abreagiert ist, muss die Phosgenherstellung abgestellt sein, da nur eine kleine Lagerkapazität für Phosgen bereitsteht, weil man den Phosgenbestand in der Anlage so gering wie möglich halten möchte. Spätestens nach 15 Minuten müssen daher die Phosgengeneratoren abgestellt sein. Beim Abfahren dieser Anlage wird zuerst der zweite Phosgengenerator außer Betrieb genommen, indem die Chlorzufuhr gestoppt wird, während der Kohlenmonoxidstrom weiter laufen gelassen wird und zur Abgasentsorgung der TAR geführt wird. Nachdem die Chlorzufuhr in den zweiten Phosgengenerator geschlossen wurde, wird die Zufuhr von Chlor und Kohlenmonoxid zum ersten Phosgengenerator gleichzeitig gedrosselt. Die Menge der beiden Einsatzstoffe, die während der Abfahrzeit von 15 Minuten, in das Mischrohr eingeleitet werden, wird stufenlos von 545 m³/h auf 0 Nm³/h Kohlenmonoxid und stufenlos von 455 m³/h auf 0 Nm³/h Chlor, gesenkt. Die Temperatur im Mischrohr beträgt weiterhin 18 °C und der Druck fällt unmittelbar von 1,8 auf 1,2 bar absolut. Die exotherme Reaktion zu Phosgen in den Phosgengeneratoren ist sofort beendet. Die Reaktionswärme fällt weg und der Wasserkreislauf der Wassersiedekühlung stellt sich automatisch ab. Die Temperatur in der Austrittsleitung des Generators fällt nach 10 Minuten von 55 °C auf 18 °C und der Druck fällt von 1,53 bar auf 1,2 bar absolut. Die sich an die Phosgenherstellung anschließende Phosgenkondensation im Rohrbündelwärmetauscher der Phosgenverflüssiger stellt sich automatisch ab und die Kühlung des Rohrbündelwärmetauschers wird einseitig geschlossen, wobei sich der Wärmetauscher von -17 °C auf 18 °C erwärmt. Im danach liegenden Phosgenlösungstank verdünnt sich die 35%-ige Phosgenlösung, weil kein frisch hergestelltes Phosgen mehr nachkommt und der Phosgenlösungstank nur noch aus der Phosgenabsorption mit einem Gemisch aus Phosgen und Monochlorbenzol befüllt wird. Nun ist die Phosgenherstellung abgestellt. Die Anlageneingänge und Anlagenausgänge sind geschlossen, das Mischrohr steht unter Kohlenmonoxid und Chlor und in den Phosgengeneratoren befindet sich Phosgen. Die Abstellung der Phosgenherstellung hat insgesamt 15 Minuten gedauert.

Während der dreistündigen Abstellungsphase der Reaktionsstrecke läuft deren flüssige Phase weiterhin durch den Entphosgenierer, dessen flüssige Phase läuft zur Lösungsmitteldestillation und dessen gasförmige Phase ist auf die Phosgenabsorption geschaltet. Während der dreistündigen Spülung wird der Entphosgenierer mit 15 bar Dampf auf 130 °C beheizt. Nach dem Spülvorgang wird der Dampf zum Entphosgenierer geschlossen, und die Entphosgenierung steht und kühlt ab. Der Brüdenweg zum Phosgenabsorber bleibt offen. Wenn kein Lösungsmittel MCB mehr im Sumpf des Entphosgenierers vorliegt, wird die Ausschleusepumpe zur Lösungsmitteldestillation abgestellt. Die Entphosgenierung steht ca. 5 Minuten nach der Reaktionsstrecke.

Während der dreistündigen Abstellung der Reaktionsstrecke läuft deren flüssige Phase weiterhin durch den Entphosgenierer und von dort in die Lösungsmitteldestillation. Während der dreistündigen Spülung wird die Lösungsmitteldestillation weiter betrieben. Die Isocyanat-Rohlösung, die aus dem Sumpf der Entphosgenierkolonne ausgetragen und mit einer Temperatur von 155 °C in die erste Destillationsstufe gefahren wird, verdünnt sich während der 3 Stunden bis nur noch Lösungsmittel MCB ankommt. Der absolute Druck am Kopf dieser Lösungsmitteldestillationskolonne ändert sich mit abnehmender Roh-MDI-Konzentration von 600 mbar auf 800 mbar und die Sumpftemperatur sinkt von 150 °C auf 120 °C. Über Kopf wird MCB gasförmig abgezogen und in einem Luftkühler kondensiert. 2,5 t/h dieses kondensierten Lösungsmittels wird in eine Waschkolonne gesprüht, um anfänglich einen möglichen Mitriss von Isocyanat in das Kondensationssystem zu verhindern. Die restlichen kondensierten, fallenden Mengen an Lösungsmittel werden über eine Vorlage mittels einer Austragepumpe in die Lösungsmittelreinigung gepumpt. Die flüssige Phase mit schwindendem Roh-MDI-Anteil wird aus dem Sumpf der Kolonne ausgetragen und in die zweite Destillationskolonne, zum Spülen dieser, gefahren. Der absolute Druck am Kopf dieser Lösungsmitteldestillationskolonne beträgt 200 mbar bei einer Sumpftemperatur von 110 °C. Über Kopf wird MCB gasförmig abgezogen, wobei dieser MCB-Gasstrom kondensiert wird und in den Sumpf der ersten Destillationskolonne zurückgeführt wird. Der Sumpf der zweiten Destillationskolonne wird über einen Gegenstromverdampfer, dessen Beheizung mit Ausbleiben von Roh-MDI etwa 30 Minuten nach Abstellung des MDA-Stromes in die Reaktionsstrecke abgestellt wird, kalt zur Rein-Destillation gepumpt. Die Lösungsmitteldestillation steht 2 Stunden nachdem die Spülung in der Reaktionsstrecke beendet ist.

Während der dreistündigen Spülung der Reaktionsstrecke läuft deren flüssige Phase weiterhin durch den Entphosgenierer, durch die Lösungsmitteldestillation und von dort in die Rein-Destillation. Während der dreistündigen Spülung wird die Rein-Destillation weiter betrieben. Die Isocyanat-Rohlösung, die aus dem Sumpf der Lösungsmitteldestillation ausgetragen und mit einer Temperatur von 120 °C in die erste Stufe der Rein-Destillation gefahren wird, verdünnt sich schon nach 30 Minuten soweit mit Lösungsmittel MCB, dass die Beheizung der Rein-Destillation abgestellt werden muss. Der absolute Druck am Kopf dieser Rein-Destillation ändert sich mit abnehmender Roh-MDI-Konzentration von 6 mbar auf 200 mbar und die Sumpftemperatur sinkt von 220 °C auf 100 °C. Mit der Einstellung der Beheizung ist die Destillation beendet und der Sumpf der Rein-Destillation wird in einen MDI-Produkttank gepumpt. Die Rein-Destillation wird zusammen mit der Lösungsmitteldestillation abgestellt.

Während der dreistündigen Spülung der Reaktionsstrecke läuft deren flüssige Phase weiterhin durch den Entphosgenierer, durch die Lösungsmitteldestillation und von dort in die Rein-Destillation. Während der dreistündigen Spülung wird die Lösungsmittelreinigung weiter betrieben. Mit dem Abstellen der Lösungsmitteldestillation fällt kein kondensierendes Lösungsmittel aus der Gasphase der Lösungsmitteldestillation mehr an. Der Einlauf über eine Vorlage mittels einer Austragepumpe in die Lösungsmittelreinigung wird geschlossen, die Austragepumpe abgestellt, sowie die Kreislaufpumpe der Lösungsmittelreinigung abgestellt und die Beheizung des Verdampfers der Lösungsmittelreinigung geschlossen.

Solange noch Brüden aus der Reaktionsstrecke, dem Entphosgenierer und die kondensierten Brüden aus der Lösungsmittelreinigung anfallen, läuft auch die Phosgenabsorption weiter. Nach dem Abstellen der Reaktionsstrecke, des Entphosgenierers und der Lösungsmittelreinigung kann auch die Phosgenabsorption abgefahren werden. Der Einlauf der gasförmigen Phase der Reaktionsstrecke und des Entphosgenierers in den Phosgenabsorber bleiben offen. Solange die Lösungsmittelreinigung in Betrieb ist, läuft deren Destillat in den Phosgenabsorber. Der Weg des Abgases des Phosgenabsorbers zur Chlorwasserstoffabsorption bleibt solange offen, wie die Prozessabgasvernichtungsanlage läuft. Der Druck des Phosgenabsorbers bleibt während der ganzen Abstellungsphase konstant bei 1,6 bar absolut und die Temperatur bleibt konstant bei 0 °C. Das MCB (-17 °C), das auf den Kopf der Phosgenabsorption zum Waschen des Abgases, aufgegeben wird, wird abgestellt. Die Kühlung der Phosgenabsorption wird 1 Stunde nach der Abstellung der Lösungsmittelreinigung abgestellt. Nun steht die Phosgenabsorption und erwärmt sich auf Umgebungstemperatur.

Solange die Phosgenabsorption betrieben wird, bleibt die Chlorwasserstoffabsorption in Betrieb. Die Chlorwasserstoff-haltige Gasphase aus der Reaktionsstrecke und dem Entphosgenierer, die in den Phosgenabsorber gehen und von dort in die Chlorwasserstoffabsorption, verliert rasch an Chlorwasserstoff. Das Chlorwasserstoffgas der gasförmigen Phase aus der Phosgenabsorption wird in einem Absorptionsapparat mit saurem Wasser aus der Prozessabgasvernichtung und zusätzlichem Kondensatwasser in einer exothermen Reaktion zu Salzsäure absorbiert und in den Salzsäuretank gefahren. Das Abgas geht weiterhin zur Prozessabgasvernichtung. Innerhalb von 30 Minuten sinkt die ankommende Chlorwasserstoffmenge soweit, dass nur noch schwache Salzsäure entsteht, die in einen dafür vorgesehenen schwache Salzsäuretank umgestellt wird. Gleichzeitig werden die Mengen an Kondensat von 3,2 t/h auf 1 t/h und schwacher Säure von 5,4 t/h auf 3 t/h zur Chlorwasserstoffabsorption stark gedrosselt. Sobald die Phosgenabsorption abgestellt ist, wird die Kondensat- und schwache Säure-Aufgabe auf den Chlorwasserstoffabsorber abgestellt. Der Weg zum Salzsäuretank ist geschlossen. Der Weg zum schwache Salzsäuretank bleibt offen. Der Weg von Phosgenabsorber über Chlorwasserstoffabsorption und einschließlich Prozessabgasvernichtung bleibt offen. Die Chlorwasserstoffabsorption steht 30 Minuten nach dem Phosgenabsorber.

Nun wird das Vakuumsystem abgestellt, indem die Vakuumpumpen der Lösungsmitteldestillation und der Lösungsmittelreinigung sowie der Rein-Destillation abgestellt werden. Die Anlagenteile werden mit Stickstoff auf Normaldruck belüftet. Diese Vorgänge dauern 1 Stunde.

Nach dem Vakuumsystem wird die Kälteanlage (Ammoniakkälteanlage), mit der MCB auf -17 °C tiefgekühlt wird, um den Phosgenabsorber, den Phosgenverflüssiger und das Vakuumsystem zu betreiben, abgefahren. Dieser Vorgang dauert 30 Minuten.

Nun wird die Prozessabgasvemichtungsanlage abgestellt, indem zuerst der Abgasweg der Phosgenabsorption zur Chlorwasserstoffabsorption und dann von der Chlorwasserstoffabsorption zur Prozessabgasvernichtung geschlossen wird. Die Wasseraufgabe auf den mit Aktivkohle gefüllten und im Unterdruck bei 980 mbar absolut betriebenen Apparat wird gestoppt. Der Ventilator wird abgestellt. Der Wasserkreislauf mit Pumpe und Pumpenvorlage wird abgestellt. Diese Vorgänge dauern 1 Stunde.

Zuletzt wird die termische Abgasreinigung außer Betrieb genommen, indem der Prozessabgasweg geschlossen wird, die Erdgasflamme abgestellt wird, der Wäscherkreislauf der Rauchgaswäsche abgestellt wird und am Ende wird der Abgasventilator abgestellt. Dieser Vorgang dauert 30 Minuten.

Jetzt steht die komplette MDI-Anlage. Der Anlagendruck wird auf Umgebungsdruck gestellt. Die Restentleerungen aller Anlagenteile werden geöffnet, um Reststoffe aus der Anlage abzulassen. Die Komplettabstellung mit Entleerung und Spülung sämtlicher Apparate, Pumpen und Rohrleitungen hat insgesamt 24 Stunden gedauert. Die Teilanlage der Phosgenherstellung wird, wie unten beschrieben, auf die Instandhaltungsmassnahme vorbereitet.

### Durchführung der Instandhaltungsmaßnahme

In dem Phosgenverflüssiger musste ein defekter Wärmetauscher ersetzt werden. Wie oben beschrieben, steht die Phosgenerzeugung 15 Minuten nachdem die Aminzufuhr in die Reaktionsstrecke geschlossen wurde. Die sich an die Phosgenherstellung anschließende Phosgenkondensation im Rohrbündelwärmetauscher der Phosgenverflüssiger stellt sich, wie oben beschrieben, automatisch ab. Nachdem die MDI-Anlage steht, wird der Rohrbündelwärmetauscher produktseitig im Ein- und Austritt geschlossen. Lösungsmittel MCB wird angeschlossen und der Apparat wird damit für 3 Stunden gespült. Anschließend wird das MCB 2 Stunden mit Stickstoff ausgeblasen. Der Rohrbündelwärmetauscher wird auch im Ein- und Austritt der Kühlmittelseite zeitgleich geschlossen. Das Kühlmittel MCB wird mit Stickstoff leergeblasen. Nun wird der Rohrbündelwärmetauscher gewechselt. Nach Einbau des Ersatzrohrbündelwärmetauschers wird die Dichtigkeit mit Stickstoff geprüft. Der Zeitbedarf dafür beträgt 8 Stunden.

Der Wechsel des defekten Wärmetauschers dauerte insgesamt 13 Stunden und die Gesamtanlage ist nach 37 Stunden wieder betriebsbereit. In modernen, automatisierten Produktionsanlagen spielt die Personalstärke für die Vorbereitung des Wechsels des Wärmetauschers, nämlich Teilentleerung der Anlage, Montage der Spülleitungen zur Reinigung des defekten Wärmetauschers mit Zu- und Ablauf, eine wichtige Rolle. In diesem Fall wird ein zusätzlicher Produktionsmitarbeiter benötigt. Handwerker zur Demontage und Montage der Rohrleitungen und für den Wärmetauscherwechsel selbst werden ebenfalls benötigt.

### Durchführung der Wiederanstellung der MDI-Anlage:

Nach der 13-stündigen Reparaturarbeit am Phosgenverflüssiger ist die Phosgen-Herstellung, nachdem die Feststellung der Dichtigkeit mit Stickstoff erfolgte und die Ventile auf der Produkt- und Kühlmittelseite des Rohrbündelwärmetauschers geöffnet wurden, zum Anfahren der Anlage bereit. Nun kann die Wiederanstellung der MDI-Anlage mit der sukkzessiven Inbetriebnahme der einzelnen Anlagenteile beginnen.

### Anfahren der Prozessabgasvernichtungsanlage:

Zuerst wird die termische Abgasreinigung (TAR) in Betrieb genommen, indem der Abgasventilator und dann der Wäscherkreislauf der Rauchgaswäsche angestellt werden, dann die Erdgasflamme entzündet wird und zuletzt der Prozessabgasweg geöffnet wird. Diese Vorgänge dauern 16 Stunden.

Nun wird die Prozessabgasvernichtungsanlage angestellt, indem zuerst der Wasserkreislauf mit Pumpe und Pumpenvorlage und der Ventilator angestellt werden. Dann wird mit der Wasseraufgabe auf den mit Aktivkohle gefüllten und im Unterdruck bei 980 mbar absolut betriebenen Apparat begonnen. Abschließend werden die Abgaswege von der Chlorwasserstoffabsorption zur Prozessabgasvernichtung und von der Phosgenabsorption zur Chlorwasserstoffabsorption geöffnet. Diese Vorgänge dauern 2 Stunden.

### Anfahren der Kältesysteme und Hilfssysteme:

Die Kreisläufe der Lösungsmittel-Hilfssysteme werden zuerst in Betrieb genommen, indem die Kreislaufpumpen, die die verschiedenen Wärmetauscher der 3 Lösungsmittel-Hilfssysteme, die sich in der Temperatur unterscheiden (kalt: -17 °C, kühl: 20 °C bis 30 °C, warm: 50 °C), temperieren, angefahren werden. Dann wird die Kälteanlage (Ammoniakkälteanlage), mit der MCB auf -17 °C tiefgekühlt wird, um die Wärmetauscher des Phosgenabsorbers, des Phosgenverflüssigers, der Lösungsmitteldestillation und des Vakuumsystems zu betreiben, angefahren. Am Ende wird die Vakuumerzeugung in Betrieb genommen, indem die Vakuumpumpen angestellt werden. Diese Vorgänge dauern 4 Stunden.

### Anfahren der Lösungsmitteldestillation:

Die Destillationskolonnen werden bis 50 % Stand mit Lösungsmittel MCB befüllt und deren MCB-Umpumpung über den Verdampfer mittels Kreislaufpumpen in Betrieb genommen. Der Vakuumweg wird geöffnet und der Verdampfer wird beheizt. Der absolute Druck am Kopf der ersten Lösungsmitteldestillationskolonne erreicht 800 mbar absolut und eine Sumpftemperatur von 123 °C stellt sich ein, wobei das Lösungsmittel anfängt zu destillieren. Über Kopf wird MCB gasförmig abgezogen und in einem Luftkühler kondensiert. 2,5 t/h dieses kondensierten Lösungsmittels wird in eine Waschkolonne gesprüht, um später den möglichen Mitriss von Isocyanat in das Kondensationssystem zu verhindern. Die restlichen kondensierten Mengen an Lösungsmittel laufen in die Vorlage der Lösungsmittelreinigung. Die flüssige Phase aus der ersten Destillationskolonne wird aus dem Sumpf der Kolonne ausgetragen und in die zweite Destillationskolonne gefahren. Der Vakuumweg wird geöffnet und der Verdampfer wird beheizt. Der absolute Druck am Kopf dieser Lösungsmitteldestillationskolonne erreicht 80 mbar absolut und eine Sumpftemperatur von 60 °C bei der das Lösungsmittel MCB anfängt zu sieden. Über Kopf wird MCB gasförmig abgezogen, wobei dieser MCB-Gasstrom kondensiert wird und in den Sumpf der ersten Destillationskolonne zurückgeführt wird. Zur weiteren Inbetriebnahme der Lösungsmitteldestillation muss die Lösungsmittelreinigung angestellt werden. Sobald diese angestellt ist, läuft Lösungsmittel in den Lösungsmitteltank. Aus dem Tank wird das Lösungsmittel über **die Reaktionsstrecke** und den **Entphosgenierer** in die Lösungsmitteldestillation gefahren, wobei die Beheizung der Reaktionsstrecke und des Entphosgenierers angestellt werden. Nun ist die Lösungsmitteldestillation betriebsbereit und läuft über die Lösungsmittelreinigung, den Lösungsmitteltank, die Reaktionsstrecke und den Entphosgenierer im Kreis. Dieser Vorgang dauert 8 Stunden. Sobald die Phosgenierung in der Reaktionstrecke gestartet wird, entsteht Roh-MDI, das über den Entphosgenierer in der Lösungsmitteldestillation ankommt. Wenn das Roh-MDI ankommt wird als Brüden über Kopf das Lösungsmittel MCB abgetrennt und aus dem Sumpf der Lösungsmitteldestillation wird das vom größten Teil an Lösungsmittel befreite Roh-MDI zur Rein-Destillation gepumpt. Der absolute Druck am Kopf der ersten Lösungsmitteldestillationskolonne wird bei 500 mbar absolut und eine Sumpftemperatur von 145 °C wird eingeregelt. Der absolute Druck am Kopf der zweiten Lösungsmitteldestillationskolonne wird bei 70 mbar absolut eingeregelt und eine Sumpftemperatur von 150 °C wird eingestellt.

### Anfahren der Lösungsmittelreinigung:

Mit dem Anstellen der Lösungsmitteldestillation fällt kondensierendes Lösungsmittel aus der Gasphase der Lösungsmitteldestillation an. Der Vakuumweg wird geöffnet. Der Einlauf über die Vorlage mittels der in Betrieb genommenen Austragepumpe in die Lösungsmittelreinigung wird geöffnet. Die Kreislaufpumpe der Lösungsmittelreinigung wird angestellt und die Beheizung des Verdampfers der Lösungsmittelreinigung wird geöffnet. Bei einem absoluten Druck von 500 mbar und einer Temperatur von 107 °C werden Brüden erzeugt, die kondensiert der Phosgenabsorption zugeführt werden, womit die **Phosgenabsorption,** durch Aufgabe dieser kondensierten Brüden auf deren Waschkolonne, gestartet, aber noch nicht angefahren wird. Der Phosgen-freie Sumpf der Lösungsmittelreinigung wird in den Lösungsmitteltank gepumpt. Diese Vorgänge dauern 4 Stunden.

### Anfahren der Phosgenabsorption:

Sobald die kondensierten Brüden aus der Lösungsmittelreinigung ankommen, werden die Ventile der Lösungsmittel-Hilfssysteme (kühl und kalt) geöffnet, um auf die heißen Brüden, die beim Anfahren der Reaktionsstrecke entstehen, vorbereitet zu sein. Das kalte MCB, das auf den Kopf der Phosgenabsorption zum Waschen des Abgases, aufgegeben wird, wird angestellt. Der Ablauf der Phosgenabsorption ist auf den Phosgenlösungstank geschaltet. Anschließend wird der Weg vom Phosgenlösungstank zur Reaktionsstrecke geöffnet. Die Phosgenabsorption ist jetzt bereit, um auch die Brüden der Reaktionsstrecke und des Entphosgenierers aufzunehmen. Mit dem Start der Phosgenierung kommt Chlorwasserstoff, der über den offenen Abgasweg zur Chlorwasserstoffabsorption weitergeführt wird, an. Das überschüssige Phosgen wird kondensiert und ausgewaschen und läuft mit dem kondensierten Lösungsmittel zum Phosgenlösungstank ab. Der Druck des Phosgenabsorbers bleibt während der ganzen Anfahrphase konstant bei 1,6 bar absolut und die Temperatur wird auf 0 °C abgesenkt.

### Anfahren der Phosgenherstellung:

Die Phosgen-Anlage wird angefahren, indem in das mit Kohlenmonoxid und Chlor befüllte Mischrohr um 1 Minute zeitversetzt Kohlenmonoxid und Chlor gefahren werden. Die Menge der beiden Einsatzstoffe, die während der Anfahrzeit t von 45 Minuten, für Chlor entsprechend nur 44 Minuten, in das Mischrohr eingeleitet werden, wird stufenlos von 0 Nm³/h auf 545 Nm³/h Kohlenmonoxid und stufenlos von 0 Nm³/h auf 455 Nm³/h Chlor, hochgezogen. Nach 45 Minuten ergibt sich im Phosgen ein Gehalt an Kohlenmonoxid von 9 %. Die Temperatur im Mischrohr beträgt 18 °C und der Druck stellt sich unmittelbar auf 1,8 bar absolut ein. Kohlenmonoxid und zeitversetzt das Mischgas aus Chlor und Kohlenmonoxid tritt in den 18 °C warmen Innenraum des Rohrbündel-Phosgengenerator ein. Die Reaktion zu Phosgen springt direkt an und ist stark exotherm. Die Reaktionswärme wird über einen Wasserkreislauf mittels Wassersiedekühlung abgeführt. Die Temperatur des Phosgens in der Austrittsleitung des Generators liegt nach 5 Minuten bei 55 °C und der Druck liegt bei 1,57 bar absolut. Das so hergestellte Phosgen wird, wie in den allgemeinen Herstellungsbedingungen beschrieben, im Phosgenverflüssiger kondensiert und im Phosgenlösungstank gesammelt. Der Phosgenverflüssiger ist betriebsbereit, wenn die Wärmeaustauscher mit -17 °C kaltem MCB gekühlt sind und der Abgasstrom des Verflüssigers mit -17 °C kaltem MCB gewaschen wird. Nach 45 Minuten werden diese Mengenströme auf einen Eduktestrom von 810 Nm³/h Chlor und 955 Nm³/h Kohlenmonoxid erhöht. Nun läuft die Phosgenherstellung und die Phosgenkonzentration im Phosgenlösungstank wird sukkzessive innerhalb von 6 Stunden auf eine 35%ige Phosgenlösung aufkonzentriert.

### Start der Phosgenierung in der Reaktionsstrecke:

Der Phosgenierturm ist mit Lösungsmittel bis zur Höhe des Überlaufes befüllt und mit Hilfe eines Wärmeträgers wurde der Phosgenierturm bereits auf 105 °C aufgeheizt. Sobald die Phosgenlösungskonzentration 25 Prozent erreicht, wird der Aminweg zur Reaktionsstrecke geöffnet. Die Aminkonzentration beträgt 18 Prozent. Während des Anfahrens wird ein stöchiometrischer Überschuss von Phosgen zu MDA von 140 Prozent eingestellt. Die Reaktionsstrecke wird mit einer Last von 15 % der Nennlast in Betrieb genommen. Neben Roh-MDI entsteht bei der Reaktion auch sofort Chlorwasserstoff, der zusammen mit überschüssigem Phosgen und anteiligem Lösungsmittel den Gasweg zum Phosgenabsorber nimmt. Nach einer Stunde wird die MDA-Lösungszufuhr auf eine Last von 25 % der Nennlast erhöht, was einer Produktionsleistung von 1,35 t/h (MDI) entspricht. Die beiden Mengenströme werden erst auf die Nennlast von 5,4 t/h MDI erhöht, wenn die Reindestillation spezifikationsgerechtes Endprodukt in den Produktionstank ausspeist. Dieser Vorgang dauert 12 Stunden. Wenn die Reaktionsstrecke auf Nennlast läuft, wird ein stöchiometrischer Phosgen- zu MDA-Überschuss von 100 Prozent eingestellt. Die MDA-Konzentration in Lösungsmittel wird dann auf 28 Prozent eingestellt. Die Phosgenkonzentration in der Phosgenlösung hat mittlerweile 35 Prozent erreicht.

### Anfahren des Entphosgenierers:

Der Entphosgenierer steht mit Lösungsmittel MCB aus dem Lösungsmitteltank, über die Reaktionsstrecke zur Lösungsmitteldestillation und Lösungsmittelreinigung im Kreislauf. Sobald aus dem Überlauf des Phosgenierturms der Reaktionsstrecke erstes Roh-MDI, Lösungsmittel und Phosgen ankommt, wird dieser bei 1,6 bar absolut auf eine Solltemperatur von 157 °C im Entphosgenierersumpf eingefahren und ist damit in Betrieb. Der Brüdenweg zum Phosgenabsorber war die ganze Zeit offen. Phosgen und Spuren an Lösungsmittel verlassen über den Gasweg den Entphosgenierer und entphosgeniertes Roh-MDI wird zur Lösungsmitteldestillation gepumpt.

### Anfahren der Chlorwasserstoff-Absorption:

Die Abgaswege vom Phosgenabsorber kommend und zur Prozessabgasvernichtung hin sind offen. Zuerst wird die Kondensat- und schwache Säure-Aufgabe auf den Chlorwasserstoffabsorber angestellt. Der Weg zum Salzsäuretank ist geschlossen. Der Weg zum schwache Salzsäuretank bleibt offen. Sobald erster Chlorwasserstoff nach dem Start der Phosgenierung seinen Weg über den Phosgenabsorber in die Chlorwasserstoffabsorption findet, wird durch Einregeln von Kondensat und schwacher Salzsäure aus dem schwache Salzsäuretank die Salzsäurekonzentration im Ablauf des Chlorwasserstoffabsorbers auf 31 Prozent eingestellt. Der Weg der Salzsäure zum Salzsäuretank wird geöffnet und der Weg zum schwache Salzsäuretank wird geschlossen. Nun ist die Chlorwasserstoff-Absorption in Betrieb. Dieser Vorgang dauert 2 Stunden und läuft parallel zur Inbetriebnahme der Phosgenierung.

### Anfahren der Rein-Destillation:

Sobald der absolute Druck am Kopf der zweiten Lösungsmitteldestillationskolonne bei 70 mbar absolut eingeregelt und eine Sumpftemperatur von 120 °C erreicht wurde, wird der Sumpf dieser zweiten Lösungsmitteldestillationskolonne auf den Einlauf der Rein-Destillation umgeschaltet. Bei einem Stand von 60 Prozent in der ersten Kolonne der Rein-Destillation wird die Kreislaufpumpe in Betrieb genommen und über den Verdampfer wird das Roh-MDI weiter aufgeheizt. Bei einem Vakuum von 6 mbar absolut und einer Sumpftemperatur von 220 °C wird das Roh-Isocyanat von Spuren an Lösungsmittel und von Phenylisocyanat befreit. Der Sumpf der ersten Kolonne der Rein-Destillation wird in die zweite Kolonne der Rein-Destillation gefahren. Auch hier wird die Kreislaufpumpe in Betrieb genommen und über den Verdampfer wird das Roh-MDI weiter beheizt. In dieser zweiten Kolonne wird bei 6 mbar absolut und 220 °C Sumpftemperatur das monomere MDI über Kopf der Kolonne vom polymeren MDI, das im Sumpf der Kolonne abgezogen wird, abgetrennt. Das polymere Sumpfprodukt wird in einen MDI-Produkttank gepumpt. Das monomere MDI, das am Kopf der Kolonne anfällt, wird in weiteren Kolonnen in die gewünschte Zusammensetzung der Isomeren getrennt, um dann in die jeweiligen Produkttanks gefahren zu werden.

Nun läuft die MDI-Anlage mit 25% der Nennlast. Das Hochziehen der Produktionsanlage auf Nennlast, das in einer modernen Produktionsanlage automatisiert ist, dauert nochmals 6 Stunden. Es ist zwingend erforderlich, die Produktionsanlage mit reduzierter Last anzufahren, da ansonsten die benötigten Temperaturprofile für die Phosgenierungsreaktion, die Lösungsmitteldestillation, die Lösungsmittelreinigung und die Rein-Destillation nicht schnell genug zur Verfügung stehen. Dieses würde zu unvollständigen Reaktionen, vermehrten Nebenprodukten und mangelhafter Aufarbeitung des Produktes führen. Zudem ist es wichtig die Teilanlage der Phosgenherstellung zeitlich gesehen so anzufahren, dass Phosgen zur Verfügung steht, wenn die Phosgenierungsreaktion gestartet werden soll. Wenn besagte Phosgenherstellung auch andere Abnehmer beliefert, dann muss die Produktionslast dieser Anlage nur rechtzeitig hochgefahren werden.

### Bilanz des Energie-, Hilfsstoff- und Zeitbedarfes für das Ab- und Anfahren der Anlage inklusive der Reinigungsmaßnahme:

Der gesamte Zeitbedarf für die Maßnahme betrug 81 Stunden. Dies gilt für den Fall, wenn genügend Personal vorhanden ist und keine technischen Schwierigkeiten auftreten. Der Zeitbedarf für die Reparaturmaßnahme an sich betrug 13 Stunden. Für das Abfahren wurden 24 Stunden benötigt. Das Anfahren dauerte weitere 44 Stunden.

Insgesamt gingen so 437,4 Tonnen an Produktion von MDI verloren.

### Beispiel 2 (erfindungsgemäß): Kurzstillstand der MDI-Anlage mit Kreislauffahrweise der nicht von der Reparaturmaßnahme betroffenen Anlagenteile, Reparaturmaßnahme und Wiederanstellung der MDI-Anlage.

Der Kurzstillstand der Anlage diente dazu, einen defekten Wärmeaustauscher in der Phosgenverflüssigung in der Phosgen-Herstellung zu wechseln. Hierfür wird die Phosgenherstellung komplett abgefahren und die anderen Anlagenteile wie Reaktionsstrecke, Entphosgenierer, Lösungsmittel-Destillation, Lösungsmittel-Reinigung, Rein-Destillation, Chlorwasserstoff-Absorption und Prozessabgasvemichter in Kreislauffahrweise gestellt. Die Energien während der Reparaturarbeiten sind nur im Bereich der Phosgenherstellung abgestellt. Das Vakuumsystem bleibt in Betrieb. Nach den Reparaturarbeiten wird wieder angefahren, wobei nur der gewechselte Wärmetauscher in der Phosgenherstellung inertisiert und mit Kühlmedium befüllt werden muss.

### Durchführung der Komplettabstellung der Phosgenherstellung und Einstellung der restlichen Anlagenteile der MDI-Anlage auf Kreislauffahrweise:

Die Phosgenherstellung wird, wie in Beispiel 1 beschrieben, abgestellt. Gleichzeitig beginnt die Einstellung der MDI-Anlage auf Kreislauffahrweise mit dem Abstellen des Eingangsstromes von MDA in die Mischeinrichtung der Reaktionsstrecke. Dazu wird die MDA-Versorgung der Reaktionsstrecke gestoppt und der MDA-Weg vom MDA-Vorratstank mit MCB für 10 Minuten von MDA frei gespült. Die Reaktionsstrecke der Phosgenierungsanlage wird mit Phosgenlösung gespült, wobei noch im Phosgenierturm befindliches MDA zu Roh-MDI abreagiert. Dabei verdünnt sich die Roh-MDI-Lösung. Die Reaktionswärme fällt nach Stoppen der MDA-Zufuhr nicht mehr an. Die Temperatur des Phosgenierreaktors wird mittels technischer Heizung bei 110 °C gehalten. Phosgenlösung aus dem Phosgenlösungstank, die sich, wie gesagt, immer weiter verdünnt, wird durch die Reaktionsstrecke gefahren und die Kreislauffahrweise stellt sich über den Entphosgenierer, den Phosgenabsorber und den Phosgenlösungstank zurück zum Mischer der Reaktionsstrecke ein. Der Druck in der Reaktionsstrecke verbleibt während der Kreislauffahrweise bei 1,4 bar (absolut).

Sobald die Phosgenierung in der Reaktionsstrecke mit dem Einstellen der MDA-Zufuhr beendet ist, wird der Entphosgenierer, wie oben beschrieben, mit der Reaktionsstrecke und dem Phosgenabsorber in Kreislauffahrweise gestellt. Während der Kreislauffahrweise läuft die flüssige Phase der Reaktionsstrecke weiterhin durch den Entphosgenierer, dessen flüssige Phase läuft zur Lösungsmitteldestillation und dessen gasförmige Phase ist auf die Phosgenabsorption geschaltet. Dazu wird der Entphosgenierer weiterhin mit Dampf beheizt, wobei die Sumpftemperatur bei 150 °C gehalten wird. Die Konzentration an Roh-MDI im Entphosgenierer wird niedriger.

Sobald die Phosgenierung in der Reaktionsstrecke mit dem Einstellen der MDA-Zufuhr beendet ist, wird der Phosgenabsorber, wie oben beschrieben, mit der Reaktionsstrecke und dem Entphosgenierer in Kreislauffahrweise gestellt. Der Einlauf der gasförmigen Phase der Reaktionsstrecke und des Entphosgenierers in den Phosgenabsorber bleiben offen. Da auch die Lösungsmittelreinigung weiter in Betrieb ist, läuft deren Destillat ebenfalls in den Phosgenabsorber. Der Weg des Abgases des Phosgenabsorbers zur Chlorwasserstoffabsorption bleibt ebenfalls offen. Der Druck des Phosgenabsorbers verbleibt während der Kreislauffahrweise konstant bei 1,6 bar absolut und die Temperatur bleibt konstant bei 0 °C. Das MCB (-17 °C), das auf den Kopf der Phosgenabsorption zum Waschen des Abgases, aufgegeben wird, wird von 15 Tonnen pro Stunde auf 4 Tonnen pro Stunde reduziert. Die Kühlung der Phosgenabsorption wird in Betrieb gehalten.

Somit ist mit der Abstellung der MDA-Zufuhr in die Mischeinrichtung eine erste Kreislauffahrweise der Reaktionsstrecke, gasförmig aus dem Phosgenierturm in den Phosgenabsorber und flüssig aus dem Phosgenierturm in den Entphosgenierer und gasförmig aus dem Entphosgenierer in die Phosgenabsorption und von dort flüssig über den Phosgenlösungstank zurück zum Mischer der Reaktionsstrecke der MDI-Anlage etabliert.

Eine zweite Kreislauffahrweise etabliert sich nach Abstellung der MDA-Zufuhr in die Mischeinrichtung der Reaktionsstrecke aus dem Einlauf des Sumpfes des Entphosgenierers in die Lösungsmittel-Destillation, über die Lösungsmittel-Reinigung, den Lösungsmitteltank, über die Reaktionsstrecke zurück zum Entphosgenierer.

Die Lösungsmittel-Destillation wird in Kreislauffahrweise weiterhin beheizt. Die Isocyanat-Rohlösung, die aus dem Sumpf der Entphosgenierkolonne ausgetragen und mit einer Temperatur von 150 °C in die erste Destillationsstufe gefahren wird, verdünnt sich während der Kreislauffahrweise bis nur noch Lösungsmittel MCB ankommt. Der absolute Druck am Kopf dieser Lösungsmitteldestillationskolonne ändert sich mit abnehmender Roh-MDI-Konzentration von 600 mbar auf 800 mbar und die Sumpftemperatur sinkt von 150 °C auf 120 °C. Über Kopf wird MCB gasförmig abgezogen und in einem Luftkühler kondensiert. 2,5 t/h dieses kondensierten Lösungsmittels wird in eine Waschkolonne gesprüht, um anfänglich einen möglichen Mitriss von Isocyanat in das Kondensationssystem zu verhindern. Die restlichen kondensierten, fallenden Mengen an Lösungsmittel werden über eine Vorlage mittels einer Austragepumpe in die Lösungsmittelreinigung gepumpt. Die flüssige Phase mit schwindendem Roh-MDI-Anteil wird aus dem Sumpf der Kolonne ausgetragen und in die zweite Destillationskolonne gefahren. Der absolute Druck am Kopf dieser zweiten Lösungsmitteldestillationskolonne beträgt 200 mbar bei einer Sumpftemperatur von 110 °C. Über Kopf wird MCB gasförmig abgezogen, wobei dieser MCB-Gasstrom kondensiert wird und in den Sumpf der ersten Destillationskolonne zurückgeführt wird. Der Sumpf der zweiten Lösungsmittel-Destillationskolonne wird während der Kreislauffahrweise weiterhin ausgeschleust.

Die Lösungsmittel-Reinigung wird in Kreislauffahrweise weiterhin beheizt. Aus der Lösungsmitteldestillation fällt weiterhin kondensierendes Lösungsmittel aus der Gasphase der Lösungsmitteldestillation an. Der Einlauf über eine Vorlage mittels einer Austragepumpe in die Lösungsmittelreinigung bleibt offen, die Austragepumpe, sowie die Kreislaufpumpe der Lösungsmittelreinigung bleiben in Betrieb und die Beheizung des Verdampfers der Lösungsmittelreinigung wird Aufrecht erhalten. Solange die Lösungsmittelreinigung in Betrieb ist, läuft deren Destillat in den Phosgenabsorber. Der Sumpf der Lösungsmittel-Reinigung, bestehend aus inertem Lösungsmittel MCB, wird kondensiert und läuft in den Lösungsmitteltank.

Diese zweite Kreislauffahrweise wird in Betrieb gehalten, indem das Lösungsmittel aus dem Lösungsmitteltank zur Reaktionsstrecke und von dort in den Entphosgenierer und von dort zurück in den Einlauf der Lösungsmittel-Destillation gefahren wird.

Somit ist mit der Abstellung der MDA-Zufuhr in die Mischeinrichtung eine zweite Kreislauffahrweise der Reaktionsstrecke, flüssig aus dem Phosgenierturm in den Entphosgenierer und flüssig aus dem Entphosgenierer in die Lösungsmittel-Destillation und von dort gasförmig über Kopf, dann kondensiert, in die Lösungsmittel-Reinigung, von dort aus der flüssigen Phase in den Lösungsmitteltank und von dort zurück zum Mischer der Reaktionsstrecke der MDI-Anlage, etabliert.

Eine dritte Kreislauffahrweise wird mit dem Sumpf der zweiten Destillationskolonne der Lösungsmittel-Destillation aufgebaut. Dazu wird dieser Sumpf als Einlauf über einen Gegenstromverdampfer, dessen Beheizung mit Ausbleiben von Roh-MDI etwa 30 Minuten nach Abstellung des MDA-Stromes in die Reaktionsstrecke abgestellt wird, kalt zur Polymerabtrennung (2410) gepumpt. Die Beheizung der Rein-Destillation wird ebenfalls abgestellt. Der Sumpf aus der Rein-Destillation, der hauptsächlich aus einem Gemisch aus Lösungsmittel MCB und Roh-MDI besteht, wird in einen MDI-Rohware-Lagertank gepumpt (nicht eingezeichnet). Aus dem MDI-Rohware-Lagertank wird das leicht MDI-haltige Lösungsmittel MCB zurück in den Einlauf der ersten Destillationskolonne (2200) der Lösungsmittel-Destillation gepumpt. Damit ist die dritte Kreislauffahrweise etabliert.

Der Abgasweg ist ausgehend von der gasförmigen Phase des Phosgenabsorbers in die Chlorwasserstoff-Absorption und von dort über die gasförmige Phase in die Prozessabgasvernichtung inklusive TAR geöffnet. Eine vierte Kreislauffahrweise wird etabliert, indem der Ablauf der Chlorwasserstoff-Absorption vom Salzsäure-Tank auf den schwache Salzsäure-Tank gestellt wird. Von dort wird die schwache Salzsäure zurück zum Kopf des Absorbers der Chlorwasserstoff-Absorption zurück gefahren.

Die noch verbliebene letzte Teilanlage der Prozessabgasvernichtung muss nicht auf Kreislauffahrweise gestellt werden, da ihr Normalbetrieb bereits einer Kreislauffahrweise entspricht. Der Prozessabgasweg ist, wie oben beschrieben, ausgehend von der gasförmigen Phase des Phosgenabsorbers in die Chlorwasserstoff-Absorption und von dort über die gasförmige Phase in die Prozessabgasvernichtung inklusive TAR geöffnet. Normalbetrieb bedeutet erstens, dass die termische Abgasreinigung (TAR) in Betrieb ist, der Abgasventilator und der Wäscherkreislauf der Rauchgaswäsche laufen und die Erdgasflamme entzündet ist und zweitens, dass die Prozessabgasvemichtungsanlage in Betrieb ist, der Wasserkreislauf mit Pumpe und Pumpenvorlage und der Ventilator laufen und die Wasseraufgabe auf den mit Aktivkohle gefüllten und im Unterdruck bei 980 mbar absolut betriebenen Apparat läuft.

Der einzige Unterschied zum Normalbetrieb ist, dass der schwache Salzsäure-Tank, der durch die kontinuierliche Wasseraufgabe auf den mit Aktivkohle gefüllten Apparat der Prozessabgasvernichtung und dessen Ablauf auf den schwache Salzsäure-Tank vollläuft, zur Kläranlage ausgespeist wird.

Die gesamte MDI-Anlage läuft jetzt bis auf die abgestellte Phosgenherstellung in Kreislauffahrweise. Die Vorbereitung (Einstellen Kreislauffahrweise von Reaktionsstrecke, Entphosgenierer, Lösungsmittel-Destillation, Lösungsmittel-Reinigung, Rein-Destillation, Chlorwasserstoff-Absorption und Prozessabgasvemichter und das Abstellen der Phosgen-Herstellung) für die Reparaturmaßnahme dauerte ohne Spülung und Entleerung der Apparate, Pumpen und Rohrleitungen insgesamt 15 Minuten.

**Durchführung der Reparaturmaßnahme:** die Reparaturmaßnahme wurde, wie in Beispiel 1 beschrieben, durchgeführt.

### Vorbereitung zur Wiederinbetriebnahme der Anlage

Die Vorbereitungen zur Wiederinbetriebnahme der Anlage waren minimal, da schon fast alle Anlagenteile in Kreislauffahrweise laufen. Lediglich die Phosgenherstellung muss nach der Reparatur, wie in Beispiel 1 beschrieben, vorbereitet und anschließend in Betrieb genommen werden.

### Wiederinbetriebnahme der Anlage

Nachdem die Phosgenherstellung in Betrieb ist, kommt Phosgen im Phosgenlösungstank der Reaktionsstrecke an. Sobald die Phosgenlösungskonzentration 25 Prozent erreicht, was nach 45 min der Fall ist, wird der Aminweg zur Reaktionsstrecke geöffnet und die Phosgenierung wird, wie in Beispiel 1 beschrieben, angefahren. Die Wiederinbetriebnahme der anderen Teilanlagen wird ebenfalls, wie in Beispiel 1 (Vergleichsbeispiel) beschrieben, aus der Kreislauffahrweise heraus durchgeführt. Die Ausspeisung des Ablaufes des schwache Salzsäure-Tankes zur Kläranlage wird beendet und auf den Kopf des Absorbers der Chlorwasserstoff-Absorption umgeschaltet, sobald erste Salzsäure aus Chlorwasserstoff in der Chlorwasserstoff-Absorption gebildet wird. Sobald frisches Roh-MDI im Sumpf der Kolonne 2410 (Polymerabtrennung) anfällt, wird die Heizung der Kolonne in Betrieb genommen, und sobald der Sumpf von Lösungsmittel befreit ist, wird der Sumpf vom PMDI-Rohware-Lagertank (2420 in FIG. 5) auf den MDI-Lagertank 2430 umgestellt. Während der Kreislauffahrweise wurde die Kolonne (2400) nicht mehr über die Destillat-Entnahme der Kolonne (2410) gespeist, lief aber destillationsgerecht aus einem Lagertank (nicht eingezeichnet) weiter. Wenn bei Wiederinbetriebnahme frisches MMDI (Destillat-Entnahme der Kolonne 2410) in der Kolonne (2400) ankommt, wird die Destillation wieder auf Solllast eingefahren.

Wie in Beispiel 1 beschrieben, läuft die MDI-Anlage nun mit 25 % der Nennlast. Das Hochziehen der Produktionsanlage auf Nennlast, das in einer modernen Produktionsanlage automatisiert ist, dauert dann ebenfalls nochmals 2 Stunden.

Der Zeitaufwand für die gesamte Aktion (Abfahren, Ausführen der Maßnahme und Anfahren) betrug 16 Stunden. Der Zeitbedarf für die Reparaturmaßnahme an sich betrug 13 Stunden. Für das Abfahren wurden 15 min benötigt. Das Anfahren dauerte 2 Stunden und 45 min.

Insgesamt gingen so 86,4 Tonnen an Produktion von MDI verloren.

Somit ergab sich bei einer Nennlast von 129,6 Tagestonnen eine Mehrproduktion von 351 Tonnen MDI im Vergleich zu Beispiel 1 (Vergleichsbeispiel).

**Fazit:** es werden im erfindungsgemäßen Beispiel 2 mit Kreislauffahrweise prozentual 64 % Primärenergie (Dampf und Strom) und 80 % Stickstoff weniger verbraucht als bei einer Komplettabstellung der Anlage wie in Beispiel 1 (Vergleichsbeispiel). Zusätzlich ergibt sich eine stark verbesserte Produktivität der Anlage, da wegen des geringeren Zeitbedarfes für die ganze Aktion (Abfahren, Maßnahme und Anfahren) über 300 Tonnen an MDI mehr produziert werden konnten.

## Patentansprüche

1. Verfahren zur Herstellung von Isocyanaten (1), umfassend die Schritte:
I) Umsetzung eines Amins (2) mit Phosgen (3) in der Flüssigphase in einer Reaktionsstrecke (1000) umfassend
I.1) eine Einrichtung (1020) zur Bereitstellung eines Amins (2), bevorzugt in Form einer Aminlösung (20) in einem Lösungsmittel (4),
I.2) eine Einrichtung (1030) zur Bereitstellung von Phosgen (3), bevorzugt in Form einer Phosgenlösung (30) in einem Lösungsmittel (4),
I.3) eine Einrichtung (1040) zur Bereitstellung eines Lösungsmittels (4),
I.4) eine Mischeinrichtung (1100) zur Vermischung von Amin (2) oder Aminlösung (20) mit Phosgen (3) oder Phosgenlösung (30) und optional weiterem Lösungsmittel (4) und
I.5) einen strömungstechnisch nach der Mischeinrichtung angeordneten Reaktionsraum (1200), dem optional eine Abscheidereinrichtung (1210) nachgeschaltet ist,
wobei
das Amin (2), bevorzugt in Form einer Aminlösung (20), mit einem Massenstrom m₂ aus der Einrichtung 1020 und
das Phosgen (3), bevorzugt in Form einer Phosgenlösung (30), mit einem Massenstrom m₃ aus der Einrichtung 1030 sowie
optional Lösungsmittel (4) aus der Einrichtung 1040 mit einem Massenstrom m₄ in die Mischeinrichtung 1100 geführt und dort vermischt werden,
die erhaltene Mischung im nachgeschalteten Reaktionsraum umgesetzt wird und
in einen flüssigen, das Roh-Isocyanat und Lösungsmittel enthaltenden Strom (60) und einen gasförmigen, Phosgen und Chlorwasserstoff enthaltenden Strom (70) aufgetrennt wird;
II) Trennung des flüssigen Stroms (60) aus Schritt I) in einen flüssigen, Lösungsmittel und Roh-Isocyanat enthaltenden Strom (80) und einen gasförmigen, Phosgen und Chlorwasserstoff enthaltenden Strom (90) in einer Destillationsvorrichtung (2100);
III) Trennung des flüssigen Stroms (80) in einen gasförmigen, Lösungsmittel enthaltenden Strom (110) und eine flüssigen, Roh-Isocyanat enthaltenden Strom (100) in einer Destillationsvorrichtung (2200);
IV) Trennung des gasförmigen Stroms (110), bevorzugt nach dessen Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
V) Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, in einer Destillationsvorrichtung (2400), optional umfassend die Abtrennung von polymeren Isocyanatfraktionen in einer vorgeschalteten Einrichtung zur Polymerabtrennung (2410) als Strom (141);
VI) Absorption der gasförmigen Ströme (70), (90) und (130) in Lösungsmittel (4) unter Erhalt eines flüssigen, Lösungsmittel und Phosgen enthaltenden Stroms (160) und eines gasförmigen, Chlorwasserstoff enthaltenden Stroms (170) in einer Absorptionsvorrichtung (2500);
VII) optional und bevorzugt, Absorption des gasförmigen Stroms (170) in Wasser oder verdünnter Salzsäure in einer weiteren Absorptionsvorrichtung (2600);
VIII) optional und bevorzugt, Reinigung von Abgasströmen mindestens aus VII), bevorzugt Reinigung von Abgasströmen aus allen vorhandenen Anlagenteilen, in einer Vorrichtung zur Abgasreinigung (3000);
IX) optional und bevorzugt, Herstellung von Phosgen (3) aus Kohlenmonoxid und Chlor in einer Phosgenerzeugungs-Vorrichtung (4000), welche mit der Einrichtung (1030) zur Bereitstellung von Phosgen verbunden ist;
wobei
bei einer Außerbetriebnahme eines Anlagenteils oder mehrerer Anlagenteile aus den Schritten (I) bis (IX), insofern diese durchgeführt werden, der Massenstrom m₂ auf null reduziert wird und in mindestens einem der nicht außer Betrieb genommenen Anlagenteile der Ausgangsstrom dieses mindestens einen nicht außer Betrieb genommenen Anlagenteils
(i) in den jeweiligen Anlagenteil oder
(ii) in einen strömungstechnisch davor oder dahinter liegenden Anlagenteil geführt und von dort, optional über weitere nicht außer Betrieb genommene Anlagenteile, in den ausgehenden Anlagenteil
zurückgeführt wird.

2. Verfahren gemäß Anspruch 1, umfassend Schritt (IX).

3. Verfahren gemäß Anspruch 1 oder 2, insbesondere gemäß Anspruch 2, bei dem bei einer Außerbetriebnahme eines Anlagenteils oder mehrerer Anlagenteile aus den Schritten (I) bis (IX), insofern diese durchgeführt werden, insbesondere bei Außerbetriebnahme der Phosgenerzeugungs-Vorrichtung (4000) aus Schritt (IX), der gasförmige Ausgangstrom des Reaktionsraums (1200) oder, sofern vorhanden, der Abscheideeinrichtung (1210), als Eingangsstrom der Absorptionsvorrichtung 2500 verwendet wird, wobei der flüssige Ausgangstrom der Absorptionsvorrichtung 2500 über die Einrichtungen 1030 und 1100 in den Reaktionsraum (1200) und von dort, sofern vorhanden, in die Abscheideeinrichtung (1210) geführt wird.

4. Verfahren gemäß einem der Ansprüche 1 bis 3, insbesondere gemäß Anspruch 2 oder 3, bei dem bei einer Außerbetriebnahme eines Anlagenteils oder mehrerer Anlagenteile aus den Schritten (I) bis (IX), insofern diese durchgeführt werden, insbesondere bei Außerbetriebnahme der Phosgenerzeugungs-Vorrichtung (4000) aus Schritt (IX), der flüssige Ausgangstrom des Reaktionsraums (1200) oder, sofern vorhanden, der Abscheideeinrichtung (1210), als Eingangsstrom der Destillationsvorrichtung 2100 verwendet wird, wobei der gasförmige Ausgangsstrom der Destillationsvorrichtung 2100 als Eingangsstrom der Absorptionsvorrichtung 2500 verwendet wird, wobei der flüssige Ausgangstrom der Absorptionsvorrichtung 2500 über die Einrichtungen 1030 und 1100 in den Reaktionsraum (1200) und von dort, sofern vorhanden, in die Abscheideeinrichtung (1210) geführt wird.

5. Verfahren gemäß einem der Ansprüche 1 bis 4, insbesondere gemäß einem der Ansprüche 2 bis 4, bei dem bei einer Außerbetriebnahme eines Anlagenteils oder mehrerer Anlagenteile aus den Schritten (I) bis (IX), insofern diese durchgeführt werden, insbesondere bei Außerbetriebnahme der Phosgenerzeugungs-Vorrichtung (4000) aus Schritt (IX), der flüssige Ausgangstrom des Reaktionsraums (1200) oder, sofern vorhanden, der Abscheideeinrichtung (1210), als Eingangsstrom der Destillationsvorrichtung 2100 verwendet wird, wobei der flüssige Ausgangsstrom der Destillationsvorrichtung 2100 als Eingangsstrom der Destillationsvorrichtung 2200 verwendet wird, wobei der gasförmige Ausgangsstrom der Destillationsvorrichtung 2200, optional nach Kondensation im Kondensator 2310, als Eingangsstrom der Destillationsvorrichtung 2300 verwendet wird, wobei der flüssige Ausgangsstrom der Destillationsvorrichtung 2300 über die Einrichtungen 1040, 1020 und 1100 in den Reaktionsraum (1200) und von dort, sofern vorhanden, in die Abscheideeinrichtung (1210) geführt wird.

6. Verfahren gemäß einem der Ansprüche 1 bis 5, insbesondere gemäß einem der Ansprüche 2 bis 5, bei dem die Einrichtung zur Polymerabtrennung 2410 vorhanden ist und bei dem bei einer Außerbetriebnahme eines Anlagenteils oder mehrerer Anlagenteile aus den Schritten (I) bis (IX), insofern diese durchgeführt werden, insbesondere bei Außerbetriebnahme der Phosgenerzeugungs-Vorrichtung (4000) aus Schritt (IX), der Sumpf-Ausgangsstrom der Einrichtung zur Polymerabtrennung (2410), ggf. über einen Lagertank (2420) als Eingangsstrom der Destillationsvorrichtung 2200 verwendet wird, wobei der flüssige Ausgangsstrom der Destillationsvorrichtung 2200 in die Einrichtung zur Polymerabtrennung 2410 zurückgeführt wird.

7. Verfahren gemäß einem der Ansprüche 1 bis 6, insbesondere gemäß einem der Ansprüche 2 bis 6, bei dem Schritt (VII) durchgeführt wird und bei dem bei einer Außerbetriebnahme eines Anlagenteils oder mehrerer Anlagenteile aus den Schritten (I) bis (IX), insofern diese durchgeführt werden, insbesondere bei Außerbetriebnahme der Phosgenerzeugungs-Vorrichtung (4000) aus Schritt (IX), der flüssige Ausgangsstrom der Absorptionsvorrichtung 2600, ggf. über einen Lagertank (2620), wieder in die Absorptionsvorrichtung 2600 zurückgeführt wird.

8. Verfahren gemäß einem der Ansprüche 1 bis 7, bei dem das Amin (2) ausgewählt ist aus der Gruppe bestehend aus Methylendiphenyldiamin, Polymethylenpolyphenylenpolyamin, einem Gemisch aus Methylendiphenyldiamin und Polymethylenpolyphenylenpolyamin, Toluylendiamin, Xylylendiamin, Hexamethylendiamin, Isophorondiamin und Naphthyldiamin.

9. Anlage (10000) zur Herstellung von Isocyanaten (1) in der Flüssigphase, umfassend die Anlagenteile
I) eine Reaktionsstrecke (1000) umfassend
I.1) eine Einrichtung (1020) zur Bereitstellung eines Amins (2), bevorzugt in Form einer Aminlösung (20) in einem Lösungsmittel (4),
I.2) eine Einrichtung (1030) zur Bereitstellung von Phosgen (3), bevorzugt in Form einer Phosgenlösung (30) in einem Lösungsmittel (4),
I.3) eine Einrichtung (1040) zur Bereitstellung eines Lösungsmittels (4),
I.4) eine Mischeinrichtung (1100) zur Vermischung von Amin (2) oder Aminlösung (20) mit Phosgen (3) oder Phosgenlösung (30) und optional weiterem Lösungsmittel (4) und
I.5) einen strömungstechnisch nach der Mischeinrichtung angeordneten Reaktionsraum (1200) zur Durchfühung der Phosgenierung, dem optional eine Abscheidereinrichtung (1210) nachgeschaltet ist, wobei der Reaktionsraum oder die Abscheidervorrichtung mit Abfuhrleitungen für einen flüssigen Strom (60) und einen gasförmigen Strom (70) versehen sind;
II) eine Destillationsvorrichtung (2100) zur Auftrennung des flüssigen Stroms (60) in einen flüssigen Strom (80) und einen gasförmigen Strom (90);
III) eine Destillationsvorrichtung (2200) zur Trennung des flüssigen Stroms (80) in einen gasförmigen Strom (110) und einen flüssigen Strom (100);
IV) eine Destillationsvorrichtung (2300) zur Trennung des gasförmigen Stroms (110), bevorzugt nach dessen Verflüssigung in einem Kondensator (2310), in einen flüssigen Strom (120) und einen gasförmigen Strom (130);
V) eine Destillationsvorrichtung (2400) zur Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, optional umfassend eine vorgeschaltete Einrichtung zur Polymerabtrennung (2410) zur Abtrennung von polymeren Isocyanatfraktionen (141);
VI) eine Vorrichtung zur Absorption (2500) der gasförmigen Ströme (70), (90) und (130) in Lösungsmittel unter Erhalt eines flüssigen Stroms (160) und eines gasförmigen Stroms (170);
VII) optional (und bevorzugt) eine Vorrichtung zur Absorption (2600) des gasförmigen Stroms (170) in Wasser;
VIII) optional (und bevorzugt) eine Vorrichtung zur Aufarbeitung von Abgasströmen (3000), welche zur Aufarbeitung von Abgasströmen mindestens aus VII), bevorzugt zur Aufarbeitung von Abgasströmen aus allen vorhandenen Anlagenteilen, ausgestaltet ist;
IX) optional (und bevorzugt) eine Vorrichtung (4000) zur Herstellung von Phosgen, welche mit der Einrichtung (1030) verbunden ist;
wobei
die Anlage (10000) derart ausgestaltet ist, dass bei einer Außerbetriebnahme eines oder mehrerer der Anlagenteile I) bis IX), insofern diese vorhanden sind,
der Massenstrom m₂ auf null reduziert wird und in mindestens einem der nicht außer Betrieb genommenen Anlagenteile der Ausgangsstrom dieses mindestens einen nicht außer Betrieb genommenen Anlagenteils
(i) in den jeweiligen Anlagenteil oder
(ii) in einen strömungstechnisch davor oder dahinter liegenden Anlagenteil geführt und von dort, optional über weitere nicht außer Betrieb genommene Anlagenteile, in den ausgehenden Anlagenteil
zurückgeführt werden kann.

10. Anlage (10000) gemäß Anspruch 9, bei der die Reduktion des Massenstroms m₂ auf null und/oder die Zurückführung des Ausgangsstroms des mindestens einen nicht außer Betrieb genommenen Anlagenteils
(i) in den jeweiligen Anlagenteil oder
(ii) in einen strömungstechnisch davor oder dahinter liegenden Anlagenteil und von dort, optional über weitere nicht außer Betrieb genommene Anlagenteile, in den ausgehenden Anlagenteil
bei einer Außerbetriebnahme eines oder mehrerer der Anlagenteile I) bis IX), insofern diese vorhanden sind, durch prozessleittechnische Einrichtungen realisiert ist.

11. Verfahren zum Betrieb einer Anlage zur Herstellung von Isocyanaten in der Flüssigphase, umfassend die folgenden Anlagenteile:
I) eine Reaktionsstrecke (1000) umfassend
I.1) eine Einrichtung (1020) zur Bereitstellung eines Amins (2), bevorzugt in Form einer Aminlösung (20) in einem Lösungsmittel (4),
I.2) eine Einrichtung (1030) zur Bereitstellung von Phosgen (3), bevorzugt in Form einer Phosgenlösung (30) in einem Lösungsmittel (4),
I.3) eine Einrichtung (1040) zur Bereitstellung eines Lösungsmittels (4),
I.4) eine Mischeinrichtung (1100) zur Vermischung von Amin (2) oder Aminlösung (20) mit Phosgen (3) oder Phosgenlösung (30) und optional weiterem Lösungsmittel (4) und
I.5) einen strömungstechnisch nach der Mischeinrichtung angeordneten Reaktionsraum (1200) zur Durchfühung der Phosgenierung, dem optional eine Abscheidereinrichtung (1210) nachgeschaltet ist, wobei der Reaktionsraum oder die Abscheidervorrichtung mit Abfuhrleitungen für einen flüssigen Strom (60) und einen gasförmigen Strom (70) versehen sind;
II) eine Destillationsvorrichtung (2100) zur Auftrennung des flüssigen Stroms (60) in einen flüssigen Strom (80) und einen gasförmigen Strom (90);
III) eine Destillationsvorrichtung (2200) zur Trennung des flüssigen Stroms (80) in einen gasförmigen Strom (110) und einen flüssigen Strom (100);
IV) eine Destillationsvorrichtung (2300) zur Trennung des gasförmigen Stroms (110), bevorzugt nach dessen Verflüssigung in einem Kondensator (2310), in einen flüssigen Strom (120) und einen gasförmigen Strom (130);
V) eine Destillationsvorrichtung (2400) zur Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, optional umfassend eine vorgeschaltete Einrichtung zur Polymerabtrennung (2410) zur Abtrennung von polymeren Isocyanatfraktionen (141);
VI) eine Vorrichtung zur Absorption (2500) der gasförmigen Ströme (70), (90) und (130) in Lösungsmittel unter Erhalt eines flüssigen Stroms (160) und eines gasförmigen Stroms (170);
VII) optional (und bevorzugt) eine Vorrichtung zur Absorption (2600) des gasförmigen Stroms (170) in Wasser;
VIII) optional (und bevorzugt) eine Vorrichtung zur Aufarbeitung von Abgasströmen (3000), welche zur Aufarbeitung von Abgasströmen mindestens aus VII), bevorzugt zur Aufarbeitung von Abgasströmen aus allen vorhandenen Anlagenteilen, ausgestaltet ist;
IX) optional (und bevorzugt) eine Vorrichtung (4000) zur Herstellung von Phosgen, welche mit der Einrichtung (1030) verbunden ist;
wobei
bei einer Produktionsunterbrechung die folgenden Schritte durchlaufen werden:
(i) Unterbrechen der Zufuhr von Amin (2) in die Mischeinrichtung 1100;
(ii) Sofern vorhanden, Abschalten der Vorrichtung (4000) zur Herstellung von Phosgen;
(iii) Verringerung der Phosgenzufuhr in die Mischeinrichtung (1100) auf bevorzugt einen Wert im Bereich von 10 % bis 50 % der Phosgenzufuhr im Regelbetrieb;
(iv) Verringerung der Lösungsmittelzufuhr in die Mischeinrichtung (1100) auf bevorzugt einen Wert im Bereich von 10 % bis 50 % der Lösungsmittelzufuhr im Regelbetrieb, bevorzugt durch Verringerung der Lösungsmittelzufuhr in die Einrichtungen 1020 und 1030;
(v) Fahren mindestens eines Anlagenteiles so, dass der Ausgangsstrom des jeweiligen Anlagenteils
(v)(i) in den jeweiligen Anlagenteil oder
(v)(ii) in einen strömungstechnisch davor oder dahinter liegenden Anlagenteil geführt und von dort, optional über weitere nicht außer Betrieb genommene Anlagenteile, in den ausgehenden Anlagenteil
zurückgeführt wird.

12. Verfahren gemäß Anspruch 11, bei dem im Anschluss an Schritt (v) die folgenden Schritte durchlaufen werden:
(vi) Außerbetriebnahme mindestens eines Anlagenteils;
(vii) Erforderlichenfalls Öffnen des in Schritt (vi) außer Betrieb genommenen mindestens einen Anlagenteils;
(viii) Durchführen einer Instandhaltungs-, Reinigungs- und/oder Reparaturmaßnahme in dem mindestens einen in Schritt (vi) außer Betrieb genommenen Anlagenteil;
(ix) Erforderlichenfalls Schließen und optional Inertisieren des mindestens einen in Schritt (vi) außer Betrieb genommenen Anlagenteils.

13. Verfahren gemäß Anspruch 12, bei dem im Anschluss an Schritt (ix) die folgenden Schritte durchlaufen werden:
(x) Anfahren des mindestens einen in Schritt (vi) außer Betrieb genommenen Anlagenteils,
(xi) Sofern vorhanden, Anfahren der Vorrichtung 4000 zur Phosgenerzeugung,
(xii) Starten, bevorzugt in dieser Reihenfolge, der Zufuhr von Lösungsmittel, Phosgen und Amin in der Reaktionsstrecke 1000 (I).

14. Verfahren gemäß Anspruch 13, bei dem im Anschluss an Schritt (xii) die folgenden Schritte durchlaufen werden:
(xiii) Warten auf Einsatz- oder Hilfsstoffe, und, sobald diese eingetroffen sind,
(xiv) Starten, bevorzugt in dieser Reihenfolge, der Zufuhr von Lösungsmittel, Phosgen und Amin in der Reaktionsstrecke 1000 (I).

15. Verfahren gemäß einem der Ansprüche 11 bis 14, bei dem das Amin (2) ausgewählt ist aus der Gruppe bestehend aus Methylendiphenyldiamin, Polymethylenpolyphenylenpolyamin, einem Gemisch aus Methylendiphenyldiamin und Polymethylenpolyphenylenpolyamin, Toluylendiamin, Xylylendiamin, Hexamethylendiamin, Isophorondiamin und Naphthyldiamin.

## Claims

1. Process for preparing isocyanates (1), comprising the steps of:
I) reacting an amine (2) with phosgene (3) in the liquid phase in a reaction zone (1000) comprising
I.1) a unit (1020) for providing an amine (2), preferably in the form of an amine solution (20) in a solvent (4),
I.2) a unit (1030) for providing phosgene (3), preferably in the form of a phosgene solution (30) in a solvent (4),
I.3) a unit (1040) for providing a solvent (4),
I.4) a mixing unit (1100) for mixing amine (2) or amine solution (20) with phosgene (3) or phosgene solution (30) and optionally further solvent (4) and
I.5) a reaction space (1200) arranged downstream of the mixing unit, with a separator unit (1210) optionally connected downstream,
wherein
the amine (2), preferably in the form of an amine solution (20), with a mass flow rate m₂ from the unit 1020 and
the phosgene (3), preferably in the form of a phosgene solution (30), with a mass flow rate m₃ from the unit 1030 and
optionally solvent (4) from the unit 1040 with a mass flow rate m₄
are conducted into the mixing unit 1100 and mixed therein, and
the mixture obtained is converted in the downstream reaction space and
separated into a liquid stream (60) comprising crude isocyanate and solvent, and a gaseous stream (70) comprising phosgene and hydrogen chloride;
II) separating the liquid stream (60) from step I) into a liquid stream (80) comprising solvent and crude isocyanate, and a gaseous stream (90) comprising phosgene and hydrogen chloride in a distillation apparatus (2100);
III) separating the liquid stream (80) into a gaseous stream (110) comprising solvent and a liquid stream (100) comprising crude isocyanate in a distillation apparatus (2200);
IV) separating the gaseous stream (110), preferably after it has been liquefied in a condenser (2310), into a liquid stream (120) comprising solvent and a gaseous stream (130) comprising phosgene in a distillation apparatus (2300);
V) obtaining a liquid isocyanate stream (140) from the liquid stream (100), resulting in a gaseous stream (150) comprising secondary components and optionally solvent, in a distillation apparatus (2400), optionally comprising the removal of polymeric isocyanate fractions in an upstream unit for polymer removal (2410) as stream (141);
VI) absorbing the gaseous streams (70), (90) and (130) in solvent (4) to obtain a liquid stream (160) comprising solvent and phosgene, and a gaseous stream (170) comprising hydrogen chloride in an absorption apparatus (2500);
VII) optionally and preferably, absorbing the gaseous stream (170) in water or dilute hydrochloric acid in a further absorption apparatus (2600);
VIII) optionally and preferably, cleaning offgas streams at least from VII), preferably cleaning offgas streams from all the plant sections present, in an apparatus for offgas cleaning (3000);
IX) optionally and preferably, preparing phosgene (3) from carbon monoxide and chlorine in a phosgene generation apparatus (4000) connected to the unit (1030) for provision of phosgene;
wherein
in a shutdown of one or more plant sections from steps (I) to (IX), insofar as they are implemented, the mass flow rate m₂ is reduced to zero and, in at least one of the plant sections that is not being shut down, the output stream from this at least one plant section which is not being shut down
(i) is recycled into the respective plant section or
(ii) is conducted into a plant section which is upstream or downstream and thence, optionally via further plant sections that have not been shut down, recycled into the original plant section.

2. Process according to Claim 1, comprising step (IX) .

3. Process according to Claim 1 or 2, especially according to Claim 2, in which in a shutdown of one or more plant sections from steps (I) to (IX), insofar as they are implemented, especially in a shutdown of the phosgene generation apparatus (4000) from step (IX), the gaseous output stream from the reaction space (1200) or, if present, from the separation unit (1210) is used as input stream for the absorption apparatus 2500, wherein the liquid output stream from the absorption apparatus 2500 is conducted via units 1030 and 1100 into the reaction space (1200) and thence, if present, into the separation unit (1210).

4. Process according to any of Claims 1 to 3, especially according to Claim 2 or 3, in which in a shutdown of one or more plant sections from steps (I) to (IX), insofar as they are implemented, especially in a shutdown of the phosgene generation apparatus (4000) from step (IX), the liquid output stream from the reaction space (1200) or, if present, from the separation unit (1210) is used as input stream for the distillation apparatus 2100, wherein the gaseous output stream from the distillation apparatus 2100 is used as input stream for the absorption apparatus 2500, wherein the liquid output stream from the absorption apparatus 2500 is conducted via units 1030 and 1100 into the reaction space (1200) and thence, if present, into the separation unit (1210).

5. Process according to any of Claims 1 to 4, especially according to any of Claims 2 to 4, in which in a shutdown of one or more plant sections from steps (I) to (IX), insofar as they are implemented, especially in a shutdown of the phosgene generation apparatus (4000) from step (IX), the liquid output stream from the reaction space (1200) or, if present, from the separation unit (1210) is used as input stream for the distillation apparatus 2100, wherein the liquid output stream from the distillation apparatus 2100 is used as input stream for the distillation apparatus 2200, wherein the gaseous output stream from the distillation apparatus 2200, optionally after condensation in the condenser 2310, is used as input stream for the distillation apparatus 2300, wherein the liquid output stream from the distillation apparatus 2300 is conducted via units 1040, 1020 and 1100 into the reaction space (1200) and thence, if present, into the separation unit (1210).

6. Process according to any of Claims 1 to 5, especially according to any of Claims 2 to 5, in which the unit for polymer removal 2410 is present and in which, in a shutdown of one or more plant sections from steps (I) to (IX), insofar as they are implemented, especially in a shutdown of the phosgene generation apparatus (4000) from step (IX), the bottom output stream from the unit for polymer removal (2410), optionally via a storage tank (2420), is used as input stream for the distillation apparatus 2200, wherein the liquid output stream from the distillation apparatus 2200 is recycled into the unit for polymer removal 2410.

7. Process according to any of Claims 1 to 6, especially according to any of Claims 2 to 6, in which step (VII) is conducted and in which, in a shutdown of one or more plant sections from steps (I) to (IX), insofar as they are implemented, especially in a shutdown of the phosgene generation apparatus (4000) from step (IX), the liquid output stream from the absorption apparatus 2600, optionally via a storage tank (2620), is recycled back into the absorption apparatus 2600.

8. Process according to any of Claims 1 to 7, in which the amine (2) is selected from the group consisting of methylenediphenyldiamine, polymethylenepolyphenylenepolyamine, a mixture of methylenediphenyldiamine and polymethylenepolyphenylenepolyamine, tolylenediamine, xylylenediamine, hexamethylenediamine, isophoronediamine and naphthyldiamine.

9. Plant (1000) for preparation of isocyanates (1) in the liquid phase, comprising the following plant sections:
I) a reaction zone (1000) comprising
I.1) a unit (1020) for providing an amine (2), preferably in the form of an amine solution (20) in a solvent (4),
I.2) a unit (1030) for providing phosgene (3), preferably in the form of a phosgene solution (30) in a solvent (4),
I.3) a unit (1040) for providing a solvent (4),
I.4) a mixing unit (1100) for mixing amine (2) or amine solution (20) with phosgene (3) or phosgene solution (30) and optionally further solvent (4) and
I.5) a reaction space (1200) arranged downstream of the mixing unit, for conducting the phosgenation, with a separator unit (1210) optionally connected downstream, wherein the reaction space or the separator apparatus have been provided with outlet conduits for a liquid stream (60) and a gaseous stream (70);
II) a distillation apparatus (2100) for separating the liquid stream (60) into a liquid stream (80) and a gaseous stream (90);
III) a distillation apparatus (2200) for separating the liquid stream (80) into a gaseous stream (110) and a liquid stream (100);
IV) a distillation apparatus (2300) for separating the gaseous stream (110), preferably after it has been liquefied in a condenser (2310), into a liquid stream (120) and a gaseous stream (130);
V) a distillation apparatus (2400) for obtaining a liquid isocyanate stream (140) from the liquid stream (100), resulting in a gaseous stream (150) comprising secondary components and optionally solvent, optionally comprising an upstream unit for polymer removal (2410) for removal of polymeric isocyanate fractions (141);
VI) an apparatus for absorption (2500) of the gaseous streams (70), (90) and (130) in solvent to obtain a liquid stream (160) and a gaseous stream (170);
VII) optionally (and preferably) an apparatus for absorption (2600) of the gaseous stream (170) in water;
VIII) optionally (and preferably) an apparatus for workup of offgas streams (3000), configured for workup of offgas streams at least from VII), preferably for workup of offgas streams from all the plant sections present;
IX) optionally (and preferably) an apparatus (4000) for preparation of phosgene, connected to the unit (1030);
wherein
the plant (10000) is configured such that, in a shutdown of one or more of plant sections I) to IX), insofar as they are present,
the mass flow rate m₂ is reduced to zero and, in at least one of the plant sections that is not being shut down, the output stream of this at least one plant section which is not being shut down
(i) can be recycled into the respective plant section or
(ii) can be conducted into a plant section which is upstream or downstream and thence, optionally via further plant sections that have not been shut down, recycled into the original plant section.

10. Plant (10000) according to Claim 9, in which the reduction in the mass flow rate m₂ to zero and/or the recycling of the output stream from the at least one plant section which is not being shut down
(i) into the respective plant section or
(ii) into a plant section which is upstream or downstream and thence, optionally via further plant sections that have not been shut down, into the original plant section in a shutdown of one or more of plant sections I) to IX), insofar as they are present, is implemented by means of process control units.

11. Process for operating a plant for preparation of isocyanates in the liquid phase, comprising the following plant sections:
I) a reaction zone (1000) comprising
I.1) a unit (1020) for providing an amine (2), preferably in the form of an amine solution (20) in a solvent (4),
I.2) a unit (1030) for providing phosgene (3), preferably in the form of a phosgene solution (30) in a solvent (4),
I.3) a unit (1040) for providing a solvent (4),
I.4) a mixing unit (1100) for mixing amine (2) or amine solution (20) with phosgene (3) or phosgene solution (30) and optionally further solvent (4) and
I.5) a reaction space (1200) arranged downstream of the mixing unit, for conducting the phosgenation, with a separator unit (1210) optionally connected downstream, wherein the reaction space or the separator apparatus have been provided with outlet conduits for a liquid stream (60) and a gaseous stream (70);
II) a distillation apparatus (2100) for separating the liquid stream (60) into a liquid stream (80) and a gaseous stream (90);
III) a distillation apparatus (2200) for separating the liquid stream (80) into a gaseous stream (110) and a liquid stream (100);
IV) a distillation apparatus (2300) for separating the gaseous stream (110), preferably after it has been liquefied in a condenser (2310), into a liquid stream (120) and a gaseous stream (130);
V) a distillation apparatus (2400) for obtaining a liquid isocyanate stream (140) from the liquid stream (100), resulting in a gaseous stream (150) comprising secondary components and optionally solvent, optionally comprising an upstream unit for polymer removal (2410) for removal of polymeric isocyanate fractions (141);
VI) an apparatus for absorption (2500) of the gaseous streams (70), (90) and (130) in solvent to obtain a liquid stream (160) and a gaseous stream (170);
VII) optionally (and preferably) an apparatus for absorption (2600) of the gaseous stream (170) in water;
VIII) optionally (and preferably) an apparatus for workup of offgas streams (3000), configured for workup of offgas streams at least from VII), preferably for workup of offgas streams from all the plant sections present;
IX) optionally (and preferably) an apparatus (4000) for preparation of phosgene, connected to the unit (1030);
wherein
in the event of a production stoppage the following steps are run:
(i) stopping the feed of amine (2) into the mixing unit 1100;
(ii) if present, switching of the apparatus (4000) for preparation of phosgene;
(iii) reducing the phosgene feed into the mixing unit (1100), preferably to a value in the range from 10% to 50% of the phosgene feed in regular operation;
(iv) reducing the solvent feed into the mixing unit (1100), preferably to a value of 10% to 50% of the solvent feed in regular operation, preferably by reducing the solvent feed into the units 1020 and 1030;
(v) running at least one plant section such that the output stream from the respective plant section
(v) (i) is recycled into the respective plant section or
(v) (ii) is conducted into a plant section which is upstream or downstream and thence, optionally via further plant sections that have not been shut down, is recycled into the original plant section.

12. Process according to Claim 11, in which, after step (v), the following steps are run:
(vi) shutting down at least one plant section;
(vii) if necessary, opening the at least one plant section that has been shut down in step (vi) ;
(viii) conducting a maintenance, cleaning and/or repair measure in the at least one plant section that has been shut down in step (vi) ;
(ix) if necessary, closing and optionally inertizing the at least one plant section that has been shut down in step (vi).

13. Process according to Claim 12, in which, after step (ix), the following steps are run:
(x) starting up the at least one plant section shut down in step (vi),
(xi) if present, starting up the apparatus 4000 for phosgene production,
(xii) starting, preferably in this sequence, the supply of solvent, phosgene and amine in the reaction zone 1000 (I).

14. Process according to Claim 13, in which, after step (xii), the following steps are run:
(xiii) waiting for feedstocks or auxiliaries and, as soon as these have arrived,
(xiv) starting, preferably in this sequence, the supply of solvent, phosgene and amine in the reaction zone 1000 (I).

15. Process according to any of Claims 11 to 14, in which the amine (2) is selected from the group consisting of methylenediphenyldiamine, polymethylenepolyphenylenepolyamine, a mixture of methylenediphenyldiamine and polymethylenepolyphenylenepolyamine, tolylenediamine, xylylenediamine, hexamethylenediamine, isophoronediamine and naphthyldiamine.

## Revendications

1. Procédé pour la préparation d'isocyanates (1), comprenant les étapes :
I) transformation d'une amine (2) avec du phosgène (3) en phase liquide dans une section de réaction (1000) comprenant
I.1) un dispositif (1020) pour la mise à disposition d'une amine (2), préférablement sous forme d'une solution d'amine (20) dans un solvant (4),
I.2) un dispositif (1030) pour la mise à disposition de phosgène (3), préférablement sous forme d'une solution de phosgène (30) dans un solvant (4),
I.3) un dispositif (1040) pour la mise à disposition d'un solvant (4),
I.4) un dispositif de mélange (1100) pour le mélange de l'amine (2) ou de la solution d'amine (20) avec du phosgène (3) ou avec la solution de phosgène (30) et éventuellement du solvant (4) supplémentaire et
I.5) un espace de réaction (1200) disposé fluidiquement après le dispositif de mélange, en aval duquel est éventuellement disposé un dispositif de séparation (1210),
l'amine (2), préférablement sous forme d'une solution d'amine (20), étant évacuée du dispositif 1020 avec un flux massique m₂, et
le phosgène (3), préférablement sous forme d'une solution de phosgène (30), étant évacuée du dispositif 1030 avec un flux massique m₃, ainsi que
du solvant (4) étant éventuellement évacué du dispositif 1040 avec un flux massique m₄,
dans le dispositif de mélange 1100 et y étant mélangés,
le mélange résultant étant transformé dans l'espace de réaction disposé en aval et
étant séparé en un flux liquide (60) contenant l'isocyanate brut et du solvant et en un flux gazeux (70) contenant du phosgène et du chlorure d'hydrogène ;
II) séparation du flux liquide (60) de l'étape I) en un flux liquide (80) contenant du solvant et l'isocyanate brut et en un flux gazeux (90) contenant du phosgène et du chlorure d'hydrogène dans un dispositif de distillation (2100) ;
III) séparation du flux liquide (80) en un flux gazeux (110) contenant du solvant et en un flux liquide (00) contenant l'isocyanate brut dans un dispositif de distillation (2200) ;
IV) séparation du flux gazeux (110), préférablement après sa liquéfaction dans un condensateur (2310), en un flux liquide (120) contenant du solvant et en un flux gazeux (130) contenant du phosgène dans un dispositif de distillation (2300) ;
V) obtention d'un flux liquide d'isocyanate (140) à partir du flux liquide (100), un flux gazeux (150) contenant des sous-produits et éventuellement du solvant étant produit, dans un dispositif de distillation (2400), comprenant éventuellement la séparation de fractions d'isocyanate polymériques dans un dispositif (2410) de séparation de polymère disposé en amont en tant que flux (141) ;
VI) absorption des flux gazeux (70), (90) et (130) dans le solvant (4) avec obtention d'un flux liquide (160) contenant du solvant et du phosgène et d'un flux gazeux (170) contenant du chlorure d'hydrogène dans un dispositif d'absorption (2500) ;
VII) éventuellement et préférablement, absorption du flux gazeux (170) dans de l'eau ou dans de l'acide chlorhydrique dilué dans un autre dispositif d'absorption (2600) ;
VIII) éventuellement et préférablement, purification de flux du gaz d'échappement d'au moins VII), préférablement purification de flux du gaz d'échappement de toutes les parties d'installation en présence, dans un dispositif pour la purification de gaz d'échappement (3000) ;
IX) éventuellement et préférablement, préparation de phosgène (3) à partir de monoxyde de carbone et de chlore dans un dispositif de génération de phosgène (4000), qui est relié au dispositif (1030) pour la mise à disposition de phosgène ;
dans lequel
lors d'une mise hors service d'une partie d'installation ou de plusieurs parties d'installation des étapes (I) à (IX), pour autant que celles-ci sont réalisées, le flux massique m₂ est réduit à zéro et, dans au moins une des parties d'installation qui n'a pas été mis hors service, le flux sortant de cette au moins une partie d'installation qui n'a pas été mise hors service
(i) est recyclé dans la partie d'installation respective ou
(ii) est conduit dans une partie d'installation disposée fluidiquement avant ou après celle-ci et de là, éventuellement par l'intermédiaire d'autres parties d'installation qui n'ont pas été mises hors service, est recyclé dans la partie d'installation sortante.

2. Procédé selon la revendication 1, comprenant l'étape (IX).

3. Procédé selon la revendication 1 ou 2, en particulier selon la revendication 2, dans lequel lors d'une mise hors service d'une partie d'installation ou de plusieurs parties d'installation des étapes (I) à (IX), pour autant que celles-ci sont réalisées, en particulier lors de la mise hors service du dispositif de génération de phosgène (4000) de l'étape (IX), le flux gazeux sortant de l'espace de réaction (1200) ou, le cas échéant, du dispositif de séparation (1210), est utilisé en tant que flux entrant du dispositif d'absorption 2500, le flux liquide sortant du dispositif d'absorption 2500 étant conduit par l'intermédiaire des dispositifs 1030 et 1100 dans l'espace de réaction (1200) et de là, le cas échéant, est conduit dans le dispositif de séparation (1210).

4. Procédé selon l'une quelconque des revendications 1 à 3, en particulier selon la revendication 2 ou 3, dans lequel lors d'une mise hors service d'une partie d'installation ou de plusieurs parties d'installation des étapes (I) à (IX), pour autant qu'elles soient réalisées, en particulier lors de la mise hors service du dispositif de génération de phosgène (4000) de l'étape (IX), le flux liquide sortant de l'espace de réaction (1200) ou, le cas échéant, du dispositif de séparation (1210), est utilisé en tant que flux entrant du dispositif de distillation 2100, le flux gazeux sortant du dispositif de distillation 2100 étant utilisé en tant que flux entrant du dispositif d'absorption 2500, le flux liquide sortant du dispositif d'absorption 2500 étant conduit par l'intermédiaire des dispositifs 1030 et 1100 dans l'espace de réaction (1200) et de là, le cas échéant, est conduit dans le dispositif de séparation (1210).

5. Procédé selon l'une quelconque des revendications 1 à 4, en particulier selon l'une quelconque des revendications 2 à 4, dans lequel lors d'une mise hors service d'une partie d'installation ou de plusieurs parties d'installation des étapes (I) à (IX), pour autant que celles-ci sont réalisées, en particulier lors de la mise hors service du dispositif de génération de phosgène (4000) de l'étape (IX), le flux liquide sortant de l'espace de réaction (1200) ou, le cas échéant, du dispositif de séparation (1210), est utilisé en tant que flux entrant du dispositif de distillation 2100, le flux liquide sortant du dispositif de distillation 2100 étant utilisé en tant que flux entrant du dispositif de distillation 2200, le flux gazeux sortant du dispositif de distillation 2200, éventuellement après condensation dans le condensateur 2310, étant utilisé en tant que flux entrant du dispositif de distillation 2300, le flux liquide sortant du dispositif de distillation 2300 étant conduit par l'intermédiaire des dispositifs 1040, 1020 et 1100 dans l'espace de réaction (1200) et de là, le cas échéant, est conduit dans le dispositif de séparation (1210).

6. Procédé selon l'une quelconque des revendications 1 à 5, en particulier selon l'une quelconque des revendications 2 à 5, dans lequel le dispositif 2410 pour la séparation de polymère est présent et dans lequel lors d'une mise hors service d'une partie d'installation ou de plusieurs parties d'installation des étapes (I) à (IX), pour autant que celles-ci sont réalisées, en particulier lors de la mise hors service du dispositif de génération de phosgène (4000) de l'étape (IX), le flux sortant de la cuve du dispositif pour la séparation de polymère (2410), est utilisé en tant que flux entrant du dispositif de distillation 2200, éventuellement par l'intermédiaire d'une cuve de stockage (2420), le flux liquide sortant du dispositif de distillation 2200 étant recyclé dans le dispositif pour la séparation de polymère 2410.

7. Procédé selon l'une quelconque des revendications 1 à 6, en particulier selon l'une quelconque des revendications 2 à 6, dans lequel l'étape (VII) est réalisée et dans lequel lors d'une mise hors service d'une partie d'installation ou de plusieurs parties d'installation des étapes (I) à (IX), pour autant que celles-ci sont réalisées, en particulier lors de la mise hors service du dispositif de génération de phosgène (4000) de l'étape (IX), le flux liquide sortant du dispositif d'absorption 2600 est recyclé à nouveau dans le dispositif d'absorption 2600, éventuellement par l'intermédiaire d'une cuve de stockage (2620).

8. Procédé selon l'une quelconque des revendications 1 à 7, dans lequel l'amine (2) est choisie dans le groupe constitué par la méthylène-diphényldiamine, la polyméthylène-polyphénylène-polyamine, un mélange de méthylène-diphényldiamine et de polyméthylène-polyphénylène-polyamine, la toluylènediamine, la xylylènendiamine, l'hexaméthylènediamine, l'isophoronediamine et la naphtyldiamine.

9. Installation (10.000) pour la préparation d'isocyanates (1) en phase liquide, comprenant les parties d'installation
I) une section de réaction (1000) comprenant
I.1) un dispositif (1020) pour la mise à disposition d'une amine (2), préférablement sous forme d'une solution d'amine (20) dans un solvant (4),
I.2) un dispositif (1030) pour la mise à disposition de phosgène (3), préférablement sous forme d'une solution de phosgène (30) dans un solvant (4),
I.3) un dispositif (1040) pour la mise à disposition d'un solvant (4),
I.4) un dispositif de mélange (1100) pour le mélange de l'amine (2) ou de la solution d'amine (20) avec du phosgène (3) ou avec la solution de phosgène (30) et éventuellement du solvant (4) supplémentaire et
I.5) un espace de réaction (1200) disposé fluidiquement après le dispositif de mélange pour la réalisation de la phosgénation, en aval duquel est éventuellement disposé un dispositif de séparation (1210), l'espace de réaction ou le dispositif de séparation étant équipé de conduites d'évacuation pour un flux liquide (60) et pour un flux gazeux (70) ;
II) un dispositif de distillation (2100) pour la séparation du flux liquide (60) en un flux liquide (80) et un flux gazeux (90) ;
III) un dispositif de distillation (2200) pour la séparation du flux liquide (80) en un flux gazeux (110) et un flux liquide (100) ;
IV) un dispositif de distillation (2300) pour la séparation du flux gazeux (110), préférablement après sa liquéfaction dans un condensateur (2310), en un flux liquide (120) et en un flux gazeux (130) ;
V) un dispositif de distillation (2400) pour l'obtention d'un flux liquide d'isocyanate (140) à partir du flux liquide (100), un flux gazeux (150) contenant des sous-produits et éventuellement du solvant étant produit, comprenant éventuellement un dispositif disposé en amont pour la séparation de polymère (2410) pour la séparation de fractions d'isocyanate polymériques (141) ;
VI) un dispositif pour l'absorption (2500) des flux gazeux (70), (90) et (130) dans un solvant avec obtention d'un flux liquide (160) et d'un flux gazeux (170) ;
VII) éventuellement (et préférablement) un dispositif pour l'absorption (2600) du flux gazeux (170) dans de l'eau ;
VIII) éventuellement (et préférablement) un dispositif pour le traitement de flux de gaz d'échappement (3000), qui est conçu pour le traitement de flux de gaz d'échappement au moins de VII), préférablement pour le traitement de flux de gaz d'échappement de toutes les parties d'installation en présence ;
IX) éventuellement (et préférablement) un dispositif (4000) pour la préparation de phosgène, qui est relié au dispositif (1030);
l'installation (10.000) étant conçue de sorte que lors d'une mise hors service d'une ou de plusieurs des parties d'installation I) à IX), pour autant que celles-ci sont présentes,
le flux massique m₂ est réduit à zéro et, dans au moins une des parties d'installation qui n'ont pas été mises hors service, le flux sortant de cette au moins une partie d'installation qui n'a pas été mise hors service
(i) peut être recyclé dans la partie d'installation respective ou
(ii) est conduit dans une partie d'installation disposée fluidiquement avant ou après celle-ci et de là, éventuellement par l'intermédiaire d'autres parties d'installation qui n'ont pas été mises hors service, peut être recyclé dans la partie d'installation sortante.

10. Installation (10.000) selon la revendication 9, dans laquelle la réduction du flux massique m₂ à zéro et/ou le recyclage du flux sortant de l'au moins une partie d'installation qui n'a pas été mise hors service
(i) est réalisé (e) dans la partie d'installation respective ou
(ii) est conduit(e) dans une partie d'installation disposée fluidiquement avant ou après et de là, éventuellement par l'intermédiaire d'autres parties d'installation qui n'ont pas été mises hors service, est réalisé(e) dans la partie d'installation sortante
lors d'une mise hors service d'une ou de plusieurs des parties d'installation I) à IX), pour autant que celles-ci sont présentes, par des dispositifs de contrôle de procédés industriels.

11. Procédé pour l'exploitation d'une installation pour la préparation d'isocyanates en phase liquide, comprenant les parties d'installation suivantes :
I) une section de réaction (1000) comprenant
I.1) un dispositif (1020) pour la mise à disposition d'une amine (2), préférablement sous forme d'une solution d'amine (20) dans un solvant (4),
I.2) un dispositif (1030) pour la mise à disposition de phosgène (3), préférablement sous forme d'une solution de phosgène (30) dans un solvant (4),
I.3) un dispositif (1040) pour la mise à disposition d'un solvant (4),
I.4) un dispositif de mélange (1100) pour le mélange de l'amine (2) ou de la solution d'amine (20) avec du phosgène (3) ou avec la solution de phosgène (30) et éventuellement du solvant (4) supplémentaire et
I.5) un espace de réaction (1200) disposé fluidiquement après le dispositif de mélange pour la réalisation de la phosgénation, en aval duquel est éventuellement disposé un dispositif de séparation (1210), l'espace de réaction ou le dispositif de séparation étant équipé de conduites d'évacuation pour un flux liquide (60) et pour un flux gazeux (70) ;
II) un dispositif de distillation (2100) pour la séparation du flux liquide (60) en un flux liquide (80) et un flux gazeux (90) ;
III) un dispositif de distillation (2200) pour la séparation du flux liquide (80) en un flux gazeux (110) et un flux liquide (100) ;
IV) un dispositif de distillation (2300) pour la séparation du flux gazeux (110), préférablement après sa liquéfaction dans un condensateur (2310), en un flux liquide (120) et en un flux gazeux (130) ;
V) un dispositif de distillation (2400) pour l'obtention d'un flux liquide d'isocyanate (140) à partir du flux liquide (100), un flux gazeux (150) contenant des sous-produits et éventuellement du solvant étant produit, comprenant éventuellement un dispositif (2410) disposé en amont pour la séparation de polymère pour la séparation de fractions d'isocyanate polymériques (141) ;
VI) un dispositif pour l'absorption (2500) des flux gazeux (70), (90) et (130) dans un solvant avec obtention d'un flux liquide (160) et d'un flux gazeux (170) ;
VII) éventuellement (et préférablement) un dispositif pour l'absorption (2600) du flux gazeux (170) dans de l'eau ;
VIII) éventuellement (et préférablement) un dispositif pour le traitement de flux de gaz d'échappement (3000), qui est conçu pour le traitement de flux de gaz d'échappement au moins de VII), préférablement pour le traitement de flux de gaz d'échappement de toutes les parties d'installation en présence ;
IX) éventuellement (et préférablement) un dispositif (4000) pour la préparation de phosgène, qui est relié au dispositif (1030) ;
dans lequel
lors d'une interruption de la production, les étapes suivantes sont accomplies :
(i) interruption de l'alimentation de l'amine (2) dans le dispositif de mélange 1100 ;
(ii) le cas échéant, arrêt du dispositif (4000) pour la préparation de phosgène ;
(iii) diminution de l'alimentation de phosgène dans le dispositif de mélange (1100) jusqu'à préférablement une valeur dans la plage de 10% à 50% par rapport à l'alimentation de phosgène lors d'une exploitation normale ;
(iv) diminution de l'alimentation en solvant dans le dispositif de mélange (1100) jusqu'à préférablement une valeur dans la plage de 10% à 50% par rapport à l'alimentation en solvant lors d'une exploitation normale, préférablement par réduction de l'alimentation en solvant dans les dispositifs 1020 et 1030 ;
(v) conduite d'au moins une partie d'installation de sorte que le flux sortant de la partie d'installation respective
(v) (i) est recyclé dans la partie d'installation respective ou
(v) (ii) est conduit dans une partie d'installation disposée fluidiquement avant ou après celle-ciet de là, éventuellement par l'intermédiaire d'autres parties d'installation qui n'ont pas été mises hors service, est recyclé dans la partie d'installation sortante.

12. Procédé selon la revendication 11, dans lequel à la suite de l'étape (v), les étapes suivantes sont accomplies :
(vi) mise hors service d'au moins une partie d'installation ;
(vii) au besoin, ouverture de l'au moins une partie d'installation mise hors service dans l'étape (vi) ;
(viii) réalisation d'une mesure de maintenance, de nettoyage et/ou de réparation dans l'au moins une partie d'installation mise hors service dans l'étape (vi) ;
(ix) au besoin, fermeture et éventuellement inertisation de l'au moins une partie d'installation mise hors service dans l'étape (vi).

13. Procédé selon la revendication 12, dans lequel à la suite de l'étape (ix) les étapes suivantes sont accomplies :
(x) mise en route de l'au moins une partie d'installation mise hors service dans l'étape (vi),
(xi) le cas échéant, mise en route du dispositif 4000 pour la génération de phosgène,
(xii) début, de préférence dans cet ordre, de l'alimentation en solvant, en phosgène et en amine dans la section de réaction 1000 (I).

14. Procédé selon la revendication 13, dans lequel à la suite de l'étape (xii) les étapes suivantes sont accomplies :
(xiii) attente des réactants ou des adjuvants, et, dès que ceux-ci sont arrivés,
(xiv) début, de préférence dans cet ordre, de l'alimentation en solvant, en phosgène et en amine dans la section de réaction 1000 (I).

15. Procédé selon l'une quelconque des revendications 11 à 14, dans lequel l'amine (2) est choisie dans le groupe constitué par la méthylène-diphényldiamine, la polyméthylène-polyphénylène-polyamine, un mélange de méthylène-diphényldiamine et de polyméthylène-polyphénylène-polyamine, la toluylènediamine, la xylylènendiamine, l'hexaméthylènediamine, l'isophoronediamine et la naphtyldiamine.
